(19) 

Europäisches
Patentamt
European
Patent Office
Office européen
des brevets

(11)  **EP 4 249 917 A2**

(12)  **EUROPEAN PATENT APPLICATION**

(43) Date of publication:
**27.09.2023  Bulletin 2023/39**

(21) Application number: **23170037.8**

(22) Date of filing: **19.09.2019**

(51) International Patent Classification (IPC):
**G01N 33/574** (2006.01)

(52) Cooperative Patent Classification (CPC):
**G01N 33/57415; G01N 33/57492**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.09.2018  US 201862734677 P
23.04.2019  US 201962837507 P
15.05.2019  US 201962848163 P**

(62) Document number(s) of the earlier application(s) in
accordance with Art. 76 EPC:
**19780486.7 / 3 857 230**

(71) Applicant: **F. Hoffmann-La Roche AG
4070 Basel (CH)**

(72) Inventors:
• **MOLINERO, Luciana Lorena
South San Francisco, California, 94080-4990 (US)**
• **CHUI, Stephen
South San Francisco, California, 94080-4990 (US)**
• **FUNKE, Roel
South San Francisco, California, 94080-4990 (US)**

(74) Representative: **Mewburn Ellis LLP
Aurora Building
Counterslip
Bristol BS1 6BX (GB)**

Remarks:
•This application was filed on 26-04-2023 as a
divisional application to the application mentioned
under INID code 62.
•Claims filed after the date of filing of the application
(Rule 68(4) EPC).

(54)  **DIAGNOSTIC METHODS FOR TRIPLE-NEGATIVE BREAST CANCER**

(57)  The invention provides methods and compositions for diagnosing and treating locally advanced or metastatic breast cancer (e.g., TNBC). In some embodiments, the methods include determining the presence and/or expression level of one or more of PD-L1, CD8, and/or stromal tumor-infiltrating lymphocytes (sTILs). Provided herein are methods for identifying a patient suffering from a locally advanced or metastatic TNBC who is likely to respond to treatment with an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel). Also provided are methods of selecting a therapy for a patient suffering from a locally advanced or metastatic TNBC. Further provided are methods of treating a patient suffering from locally advanced or metastatic TNBC. In some embodiments, the methods include administering a PD-1 axis binding antagonist (e.g., atezolizumab) and a taxane (e.g., nab-paclitaxel or paclitaxel) to the patient, for example, a patient identified as one who is likely to respond to the treatment. Also provided are pharmaceutical compositions for use in treating a patient suffering from locally advanced or metastatic TNBC.

EP 4 249 917 A2

**Description**

**FIELD OF THE INVENTION**

This invention relates to methods of diagnosing and treating breast cancers (e.g., locally advanced or metastatic triple-negative breast cancer (TNBC)), for example, by administering a PD-1 axis binding antagonist (e.g., atezolizumab) and a taxane (e.g., nab-paclitaxel or paclitaxel) to patients who have been identified as likely to respond based on the presence and/or expression of a biomarker of the invention.

**BACKGROUND OF THE INVENTION**

Cancer remains one of the most deadly threats to human health. Cancers, or malignant tumors, metastasize and grow rapidly in an uncontrolled manner, making timely detection and treatment extremely difficult. In the U.S., cancer affects nearly 1.3 million new patients each year, and is the second leading cause of death after heart disease, accounting for approximately 1 in 4 deaths. Solid tumors are responsible for most of those deaths. Breast cancer is the most common cancer among women. Approximately 10-15% of breast cancers are triple-negative for expression of estrogen, progesterone, and HER2 receptors, also referred to as triple-negative breast cancer (TNBC). TNBC is usually more aggressive than estrogen receptor-positive breast cancer and HER2-positive breast cancer, and can be difficult to treat.

Programmed death-ligand 1 (PD-L1) is a protein that has been implicated in the suppression of immune system responses during cancer, chronic infections, pregnancy, tissue allografts, and autoimmune diseases. PD-L1 regulates the immune response by binding to an inhibitory receptor, known as programmed death 1 (PD-1), which is expressed on the surface of T-cells, B-cells, and monocytes. PD-L1 negatively regulates T-cell function also through interaction with another receptor, B7-1. Formation of the PD-L1/PD-1 and PD-L1/B7-1 complexes negatively regulates T-cell receptor signaling, resulting in the subsequent downregulation of T-cell activation and suppression of anti-tumor immune activity.

Despite the significant advancement in the treatment of cancer (e.g., breast cancer (e.g., locally advanced or metastatic TNBC)), improved therapies and diagnostic methods are still being sought.

**SUMMARY OF THE INVENTION**

This invention relates to, *inter alia,* methods for identifying a patient suffering from a locally advanced or metastatic TNBC who is likely to respond to treatment with an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel); methods of selecting a therapy for a patient suffering from a locally advanced or metastatic TNBC; methods of treating a patient suffering from locally advanced or metastatic TNBC; and pharmaceutical compositions for use in treating a patient suffering from locally advanced or metastatic TNBC.

In one aspect, the invention features a method for identifying a patient suffering from a locally advanced or metastatic TNBC who is likely to respond to treatment with an anti-cancer therapy comprising (i) a human PD-1 axis binding antagonist selected from an anti-PD-L1 antibody and an anti-PD-1 antibody and (ii) a taxane, the method comprising determining the expression level of PD-L1 in a tumor sample obtained from the patient, wherein the patient has not been previously treated for the TNBC, and wherein a detectable expression level of PD-L1 in the tumor sample identifies the patient as likely to respond to treatment with the anti-cancer therapy.

In another aspect, the invention features a method for identifying a patient suffering from a locally advanced or metastatic TNBC who is likely to respond to treatment with an anti-cancer therapy comprising (i) a human PD-1 axis binding antagonist selected from an anti-PD-L1 antibody and an anti-PD-1 antibody and (ii) a taxane, the method comprising determining the expression level of PD-L1 in tumor-infiltrating immune cells in a tumor sample obtained from the patient, wherein the patient has not been previously treated for the TNBC, and wherein a detectable expression level of PD-L1 in tumor-infiltrating immune cells that comprise about 1 % or more of the tumor sample identifies the patient as likely to respond to treatment with the anti-cancer therapy.

In another aspect, the invention features a method for selecting an anti-cancer therapy for a patient suffering from a locally advanced or metastatic TNBC, the method comprising: (a) determining the expression level of PD-L1 in a tumor sample obtained from the patient, wherein the patient has not been previously treated for the TNBC; and (b) selecting an anti-cancer therapy comprising (i) a human PD-1 axis binding antagonist selected from an anti-PD-L1 antibody and an anti-PD-1 antibody and (ii) a taxane for the patient based on a detectable expression level of PD-L1 in the tumor sample.

In another aspect, the invention features a method for selecting an anti-cancer therapy for a patient suffering from a locally advanced or metastatic TNBC, the method comprising: (a) determining the expression level of PD-L1 in tumor-infiltrating immune cells in a tumor sample obtained from the patient, wherein the patient has not been previously treated for the TNBC; and (b) selecting an anti-cancer therapy comprising (i) a human PD-1 axis binding antagonist selected from an anti-PD-L1 antibody and an anti-PD-1 antibody and (ii) a taxane for the patient based on a detectable expression level of PD-L1 in tumor-infiltrating immune cells that comprise about 1 % or more of the tumor sample.

In another aspect, the invention features a method of treating a patient suffering from a locally advanced or metastatic TNBC, the method comprising administering to the patient an effective amount of an anti-cancer therapy comprising (i) a human PD-1 axis binding antagonist selected from an anti-PD-L1 antibody and an anti-PD-1 antibody and (ii) a taxane, wherein the patient has not been previously treated for the TNBC, and wherein the patient has been identified as likely to respond to the anti-cancer therapy based on a detectable expression level of PD-L1 in a tumor sample obtained from the patient.

In another aspect, the invention features a method of treating a patient suffering from a locally advanced or metastatic TNBC, the method comprising administering to the patient an effective amount of an anti-cancer therapy comprising (i) a human PD-1 axis binding antagonist selected from an anti-PD-L1 antibody and an anti-PD-1 antibody and (ii) a taxane, wherein the patient has not been previously treated for the TNBC, and wherein the patient has been identified as likely to respond to the anti-cancer therapy based on a detectable expression level of PD-L1 in tumor-infiltrating immune cells that comprise about 1% or more of a tumor sample obtained from the patient. In some embodiments, the human PD-1 axis binding antagonist is atezolizumab.

In another aspect, the invention features a method for treating a patient suffering from a locally advanced or metastatic TNBC, the method comprising: (a) determining the expression level of PD-L1 in a tumor sample obtained from the patient, wherein the patient has not been previously treated for the TNBC; and (b) administering to the patient an effective amount of an anti-cancer therapy comprising (i) a human PD-1 axis binding antagonist selected from an anti-PD-L1 antibody and an anti-PD-1 antibody and (ii) a taxane based on a detectable expression level of PD-L1 in the tumor sample.

In another aspect, the invention features a method for treating a patient suffering from a locally advanced or metastatic TNBC, the method comprising: (a) determining the expression level of PD-L1 in tumor-infiltrating immune cells in a tumor sample obtained from the patient, wherein the patient has not been previously treated for the TNBC; and (b) administering to the patient an effective amount of an anti-cancer therapy comprising (i) a human PD-1 axis binding antagonist selected from an anti-PD-L1 antibody and an anti-PD-1 antibody and (ii) a taxane based on a detectable expression level of PD-L1 in tumor-infiltrating immune cells that comprise about 1 % or more of the tumor sample.

In another aspect, the invention features a pharmaceutical composition comprising a human PD-1 axis binding antagonist selected from an anti-PD-L1 antibody and an anti-PD-1 antibody for use in treatment of a patient diagnosed with locally advanced or metastatic TNBC, wherein the treatment comprises administration of the human PD-1 axis binding antagonist in combination with a taxane, and wherein the patent is identified as likely to respond to an anti-cancer therapy comprising the human PD-1 axis binding antagonist and a taxane based on a detectable expression level of PD-L1 in a tumor sample obtained from the patient.

In another aspect, the invention features a pharmaceutical composition comprising a human PD-1 axis binding antagonist selected from an anti-PD-L1 antibody and an anti-PD-1 antibody for use in treatment of a patient diagnosed with locally advanced or metastatic TNBC, wherein the treatment comprises administration of the human PD-1 axis binding antagonist in combination with a taxane, and wherein the patent is identified as likely to respond to an anti-cancer therapy comprising the human PD-1 axis binding antagonist and a taxane based on a detectable expression level of PD-L1 in tumor-infiltrating immune cells that comprise about 1% or more of a tumor sample obtained from the patient. In some embodiments, the human PD-1 axis binding antagonist is atezolizumab.

In some embodiments of any of the preceding aspects, the tumor sample obtained from the patient has a detectable expression level of PD-L1 in tumor-infiltrating immune cells that comprise (i) about 5% or more of the tumor sample or (ii) or about 10% or more of the tumor sample.

In some embodiments of any of the preceding aspects, the patient has received no prior chemotherapy or targeted systemic therapy for inoperable locally advanced or metastatic TNBC.

In some embodiments of any of the preceding aspects, the locally advanced TNBC is unresectable.

In some embodiments of any of the preceding aspects, the tumor sample is a formalin-fixed and paraffin-embedded (FFPE) tumor sample, an archival tumor sample, a fresh tumor sample, or a frozen tumor sample.

In some embodiments of any of the preceding aspects, the expression level of PD-L1 is a protein expression level. In some embodiments, the protein expression level of PD-L1 is determined using immunohistochemistry (IHC), immunofluorescence, flow cytometry, or Western blot. In some embodiments, the protein expression level of PD-L1 is determined using IHC. In some embodiments, the protein expression level of PD-L1 is detected using an anti-PD-L1 antibody. In some embodiments, the anti-PD-L1 antibody is SP142.

In some embodiments of any of the preceding aspects, the method further includes administering an effective amount of the anti-cancer therapy to the patient.

In some embodiments of any of the preceding aspects, the human PD-1 axis binding antagonist is atezolizumab.

In some embodiments of any of the preceding aspects, the taxane is nab-paclitaxel.

In other embodiments of any of the preceding aspects, the taxane is paclitaxel.

In some embodiments of any of the preceding aspects, whether the patient is likely to respond to treatment with the anti-cancer therapy is determined in terms of progression-free survival.

In some embodiments of any of the preceding aspects, whether the patient is likely to respond to treatment with the anti-cancer therapy is determined in terms of overall survival.

It is to be understood that one, some, or all of the properties of the various embodiments described herein may be combined to form other embodiments of the present invention. These and other aspects of the invention will become apparent to one of skill in the art. These and other embodiments of the invention are further described by the detailed description that follows.

## DETAILED DESCRIPTION OF EMBODIMENTS OF THE INVENTION

### I. Introduction

The present invention provides diagnostic and therapeutic methods and compositions for cancer, for example, breast cancer (e.g., locally advanced or metastatic triple-negative breast cancer (TNBC)), including in patients who have not been previously treated for their cancer. The invention is based, at least in part, on the discovery that determination of the presence and/or expression level(s) of biomarkers of the invention, for example, PD-L1, CD8, and/or stromal tumor-infiltrating lymphocytes (sTILs), in samples obtained from a patient (e.g., a tumor sample) is useful in diagnosing a patient suffering from cancer, for determining whether a patient having a cancer is likely to respond to treatment with an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel), for optimizing therapeutic efficacy of an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel), for patient selection for an anti-cancer therapy comprising a PD-1 axis binding antagonist (e.g., an anti-PD-L1 antibody (e.g., atezolizumab) or an anti-PD-1 antibody) and a taxane (e.g., nab-paclitaxel or paclitaxel), and/or treatment of a patient suffering from cancer with an anti-cancer therapy comprising a PD-1 axis binding antagonist (e.g., an anti-PD-L1 antibody (e.g., atezolizumab) or an anti-PD-1 antibody) and a taxane (e.g., nab-paclitaxel or paclitaxel). For example, in some embodiments, the patient is PD-L1-positive. In some embodiments, whether the patient is likely to respond to treatment with the anti-cancer therapy is determined in terms of progression-free survival. In other embodiments, whether the patient is likely to respond to treatment with the anti-cancer therapy is determined in terms of overall survival.

### II. Definitions

Before describing the invention in detail, it is to be understood that this invention is not limited to particular compositions or biological systems, which can, of course, vary. It is also to be understood that the terminology used herein is for the purpose of describing particular embodiments only, and is not intended to be limiting.

As used in this specification and the appended claims, the singular forms "a," "an," and "the" include plural referents unless the content clearly dictates otherwise. Thus, for example, reference to "a molecule" optionally includes a combination of two or more such molecules, and the like.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of" aspects and embodiments.

**The terms** "programmed death **ligand 1" and "PD-L1" refer herein to a native sequence** PD-L1 polypeptide, polypeptide variants, and fragments of a native sequence polypeptide and polypeptide variants (which are further defined herein). The PD-L1 polypeptide described herein may be that which is isolated from a variety of sources, such as from human tissue types or from another source, or prepared by recombinant or synthetic methods.

**A "native sequence PD-L1 polypeptide" comprises a polypeptide having the same amino acid** sequence as the corresponding PD-L1 polypeptide derived from nature.

**A "PD-L1** polypeptide variant," **or variations thereof, means a** PD-L1 polypeptide, generally an active PD-L1 polypeptide, as defined herein having at least about 80% amino acid sequence identity with any of the native sequence PD-L1 polypeptide sequences as disclosed herein. Such PD-L1 polypeptide variants include, for instance, PD-L1 polypeptides wherein one or more amino acid residues are added, or deleted, at the N- or C-terminus of a native amino acid sequence. Ordinarily, a PD-L1 polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence PD-L1 polypeptide sequence as disclosed herein. Ordinarily, PD-L1 variant polypeptides are at least about 10 amino acids in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 281, 282, 283, 284, 285, 286, 287, 288, or 289 amino acids in length, or more. Optionally, PD-L1 variant polypeptides will have no more than one conservative amino acid substitution as compared to a native PD-L1 polypeptide sequence, alternatively no more than 2, 3, 4, 5, 6, 7, 8, 9, or 10 conservative amino acid substitutions as compared to a native PD-L1 polypeptide sequence.

The term "CD8" or "cluster of differentiation 8" refers to a transmembrane glycoprotein that serves as a co-receptor for the T cell receptor (TCR), and refers to any native CD8 from any vertebrate source, including mammals such as primates (e.g., humans) and rodents (e.g., mice and rats), unless otherwise indicated. The term encompasses "full-length," unprocessed CD8 as well as any form of CD8 that results from processing in the cell. There are two isoforms of CD8, CD8A and CD8B, which are each encoded by a different gene. As used herein, CD8 encompasses CD8A and CD8B. In some embodiments, the CD8 is human CD8.

The term "CD8A" as used herein, refers to any native CD8A from any vertebrate source, including mammals such as primates (e.g., humans) and rodents (e.g., mice and rats), unless otherwise indicated. CD8A is also known as CD8 alpha. The term encompasses "full-length," unprocessed CD8A as well as any form of CD8A that results from processing in the cell. The term also encompasses naturally occurring variants of CD8A e.g., splice variants or allelic variants. The amino acid sequence of an exemplary human CD8A can be found under UniProtKB Accession No. P01732.

The term "CD8B" as used herein, refers to any native CD8B from any vertebrate source, including mammals such as primates (e.g., humans) and rodents (e.g., mice and rats), unless otherwise indicated. CD8B is also known as CD8 beta. The term encompasses "full-length," unprocessed CD8B as well as any form of CD8B that results from processing in the cell. The term also encompasses naturally occurring variants of CD8B, e.g., splice variants or allelic variants. The amino acid sequence of an exemplary human CD8B can be found under UniProtKB Accession No. P10966.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs.

"Oligonucleotide," as used herein, generally refers to short, single stranded, polynucleotides that are, but not necessarily, less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "primer" refers to a single-stranded polynucleotide that is capable of hybridizing to a nucleic acid and allowing polymerization of a complementary nucleic acid, generally by providing a free 3'-OH group.

The term "detection" includes any means of detecting, including direct and indirect detection.

The term "biomarker" as used herein refers to an indicator, e.g., predictive, diagnostic, and/or prognostic, which can be detected in a sample, for example, PD-L1, CD8, and/or sTILs. The biomarker may serve as an indicator of a particular subtype of a disease or disorder (e.g., cancer) characterized by certain, molecular, pathological, histological, and/or clinical features. In some embodiments, a biomarker is a gene. Biomarkers include, but are not limited to, polynucleotides (e.g., DNA and/or RNA), polynucleotide copy number alterations (e.g., DNA copy numbers), polypeptides, polypeptide and polynucleotide modifications (e.g., post-translational modifications), carbohydrates, and/or glycolipid-based molecular markers.

The "amount" or "level" of a biomarker associated with an increased clinical benefit to an individual is a detectable level in a biological sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a biological sample. "Expression" generally refers to the process by which information (e.g., gene-encoded and/or epigenetic information) is converted into the structures present and operating in the cell. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g., posttranslational modification of a polypeptide) shall also be regarded as expressed whether they originate from a transcript generated by alternative splicing or a degraded transcript, or from a post-translational processing of the polypeptide, e.g., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"increased expression," "increased expression level," "increased levels," "elevated expression," "elevated expression levels," or "elevated levels" refers to an increased expression or increased levels of a biomarker in an individual relative to a control, such as an individual or individuals who are not suffering from the disease or disorder (e.g., cancer) or an internal control (e.g., a housekeeping biomarker).

"Decreased expression," "decreased expression level," "decreased levels," "reduced expression," "reduced expression levels," or "reduced levels" refers to a decrease expression or decreased levels of a biomarker in an individual relative to a control, such as an individual or individuals who are not suffering from the disease or disorder (e.g., cancer) or an internal control (e.g., a housekeeping biomarker). In some embodiments, reduced expression is little or no expression.

The term "housekeeping biomarker" refers to a biomarker or group of biomarkers (e.g., polynucleotides and/or polypeptides) which are typically similarly present in all cell types. In some embodiments, the housekeeping biomarker is a "housekeeping gene." A "housekeeping gene" refers herein to a gene or group of genes which encode proteins whose activities are essential for the maintenance of cell function and which are typically similarly present in all cell types.

The term "PD-L1-positive" as used herein, refers to a sample (e.g., a tumor sample) that expresses a detectable level of PD-L1, or a subject from whom a PD-L1-positive sample has been obtained. PD-L1 may be detected directly or indirectly using any suitable approach, e.g., immunohistochemistry (IHC).

**"Amplification," as used herein generally refers to the process of producing multiple copies of a desired sequence. "Multiple copies" mean at least two copies. A "copy" does not necessarily mean** perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

**The term "multiplex-PCR" refers to a single PCR reaction carried out on nucleic acid obtained** from a single source (e.g., an individual) using more than one primer set for the purpose of amplifying two or more DNA sequences in a single reaction.

**The technique of "polymerase chain reaction" or "PCR" as used herein generally refers to a** procedure wherein minute amounts of a specific piece of nucleic acid, RNA and/or DNA, are amplified as described, for example, in U.S. Pat. No. 4,683,195. Generally, sequence information from the ends of the region of interest or beyond needs to be available, such that oligonucleotide primers can be designed; these primers will be identical or similar in sequence to opposite strands of the template to be amplified. **The 5' terminal nucleotides of the two primers may coincide with the ends of the amplified material. PCR** can be used to amplify specific RNA sequences, specific DNA sequences from total genomic DNA, and cDNA transcribed from total cellular RNA, bacteriophage, or plasmid sequences, etc. See generally Mullis et al., Cold Spring Harbor Symp. Quant. Biol. 51:263 (1987) and Erlich, ed., PCR Technology, (Stockton Press, NY, 1989). As used herein, PCR is considered to be one, but not the only, example of a nucleic acid polymerase reaction method for amplifying a nucleic acid test sample, comprising the use of a known nucleic acid (DNA or RNA) as a primer and utilizes a nucleic acid polymerase to amplify or generate a specific piece of nucleic acid or to amplify or generate a specific piece of nucleic acid which is complementary to a particular nucleic acid.

**"Quantitative real-time polymerase chain reaction" or "qRT-PCR" refers to a form of PCR wherein** the amount of PCR product is measured at each step in a PCR reaction. This technique has been described in various publications including, for example, Cronin et al., Am. J. Pathol. 164(1):35-42 (2004) and Ma et al., Cancer Cell 5:607-616 (2004).

**The term "microarray" refers to an ordered arrangement of hybridizable array elements,** preferably polynucleotide probes, on a substrate.

**The term "diagnosis" is used herein to refer to the identification or classifica**tion of a molecular or pathological state, disease or condition (e.g., cancer (e.g., breast cancer (e.g., locally advanced or metastatic TNBC))). **For example, "diagnosis" may refer to identification of a particular type of cancer. "Diagnosis" may also refe**r to the classification of a particular subtype of cancer, for instance, by histopathological criteria, or by molecular features (e.g., a subtype characterized by expression of one or a combination of biomarkers (e.g., particular genes or proteins encoded by said genes)).

**The term "sample," as used herein, refers to a composition that is obtained or derived from a** subject and/or individual of interest that contains a cellular and/or other molecular entity that is to be characterized and/or identified, for example, based on physical, biochemical, chemical, and/or **physiological characteristics. For example, the phrase "disease sample" and variations thereof refers to** any sample obtained from a subject of interest that would be expected or is known to contain the cellular and/or molecular entity that is to be characterized. Samples include, but are not limited to, tissue samples, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

By "tissue sample" or "cell sample" is meant a collection of similar cells obtained from a tissue of a subject or individual. The source of the tissue or cell sample may be solid tissue as from a fresh, frozen and/or preserved organ, tissue sample, biopsy, and/or aspirate; blood or any blood constituents such as plasma; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. For instance, a "tumor sample" is a tissue sample obtained from a tumor or other cancerous tissue. The tissue sample may contain a mixed population of cell types (e.g., tumor cells and non-tumor cells, cancerous cells and non-cancerous cells). The tissue sample may contain compounds which are not naturally intermixed with the tissue in nature such as preservatives, anticoagulants, buffers, fixatives, nutrients, antibiotics, or the like.

A "tumor-infiltrating immune cell," as used herein, refers to any immune cell present in a tumor or a sample thereof. Tumor-infiltrating immune cells include, but are not limited to, intratumoral immune cells, peritumoral immune cells, other tumor stroma cells (e.g., fibroblasts), or any combination thereof. Such tumor-infiltrating immune cells can be, for example, T lymphocytes (such as CD8+ T lymphocytes and/or CD4+ T lymphocytes), B lymphocytes, or other bone marrow-lineage cells, including granulocytes (e.g., neutrophils, eosinophils, and basophils), monocytes, macrophages, dendritic cells (e.g., interdigitating dendritic cells), histiocytes, and natural killer cells.

A "tumor cell" as used herein, refers to any tumor cell present in a tumor or a sample thereof. Tumor cells may be distinguished from other cells that may be present in a tumor sample, for example, stromal cells and tumor-infiltrating immune cells, using methods known in the art and/or described herein.

A "reference sample," "reference cell," "reference tissue," "control sample," "control cell," or "control tissue," as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (e.g., tissue or cells) of the same subject or individual. For example, the reference sample, reference cell, reference tissue, control sample, control cell, or control tissue may be healthy and/or non-diseased cells or tissue adjacent to the diseased cells or tissue (e.g., cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (e.g., tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual.

For the purposes herein a "section" of a tissue sample is meant a single part or piece of a tissue sample, for example, a thin slice of tissue or cells cut from a tissue sample (e.g., a tumor sample). It is to be understood that multiple sections of tissue samples may be taken and subjected to analysis, provided that it is understood that the same section of tissue sample may be analyzed at both morphological and molecular levels, or analyzed with respect to polypeptides (e.g., by immunohistochemistry) and/or polynucleotides (e.g., by in situ hybridization).

By "correlate" or "correlating" is meant comparing, in any way, the performance and/or results of a first analysis or protocol with the performance and/or results of a second analysis or protocol. For example, one may use the results of a first analysis or protocol in carrying out a second protocol and/or one may use the results of a first analysis or protocol to determine whether a second analysis or protocol should be performed. With respect to the embodiment of polypeptide analysis or protocol, one may use the results of the polypeptide expression analysis or protocol to determine whether a specific therapeutic regimen should be performed. With respect to the embodiment of polynucleotide analysis or protocol, one may use the results of the polynucleotide expression analysis or protocol to determine whether a specific therapeutic regimen should be performed.

The phrase "based on" when used herein means that the information about one or more biomarkers is used to inform a treatment decision, information provided on a package insert, or marketing/promotional guidance, etc.

The word "label" when used herein refers to a compound or composition that is conjugated or fused directly or indirectly to a reagent such as a polynucleotide probe or an antibody and facilitates detection of the reagent to which it is conjugated or fused. The label may itself be detectable (e.g., radioisotope labels or fluorescent labels) or, in the case of an enzymatic label, may catalyze chemical alteration of a substrate compound or composition which is detectable. The term is intended to encompass direct labeling of a probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin.

The term "PD-1 axis binding antagonist" refers to a molecule that inhibits the interaction of a PD-1 axis binding partner with either one or more of its binding partner, so as to remove T-cell dysfunction resulting from signaling on the PD-1 signaling axis, with a result being to restore or enhance T-cell function (e.g., proliferation, cytokine production, and/or target cell killing). As used herein, a PD-1 axis binding antagonist includes a PD-L1 binding antagonist, a PD-1 binding antagonist, and a PD-L2 binding antagonist.

The term "PD-L1 binding antagonist" refers to a molecule that decreases, blocks, inhibits, abrogates, or interferes with signal transduction resulting from the interaction of PD-L1 with either one or more of its binding partners, such as PD-1 and/or B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen-binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1 and/or B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody. In a specific aspect, an anti-PD-L1 antibody is atezolizumab, marketed as TECENTRIQ™ with a WHO Drug Information (International Nonproprietary Names for Pharmaceutical Substances), Recommended INN: List 74, Vol. 29, No. 3, 2015 (see page 387) described herein. In another specific aspect, an anti-PD-L1 antibody is MDX-1105 described herein. In still another specific aspect, an anti-PD-L1 antibody is YW243.55.S70 described herein. In still another specific aspect, an anti-PD-L1 antibody is MEDI4736 (durvalumab) described herein. In still another specific aspect, an anti-PD-L1 antibody is MSB0010718C (avelumab) described herein.

The term "PD-1 binding antagonist" refers to a molecule that decreases, blocks, inhibits, abrogates or interferes with signal transduction resulting from the interaction of PD-1 with one or more of its binding partners, such as PD-L1 and/or PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen-binding fragments thereof, immunoadhesins, fusion proteins, oligopeptides, and other molecules that decrease, block, inhibit, abrogate or interfere with signal transduction resulting from the interaction of PD-1 with PD-L1 and/or PD-L2. In one embodiment, a PD-1 binding antagonist reduces the negative co-stimulatory signal mediated by or through cell surface proteins expressed on T lymphocytes mediated signaling through PD-1 so as render a dysfunctional T-cell less dysfunctional (e.g., enhancing effector responses to antigen recognition). In some embodiments, the PD-1 binding antagonist is an anti-PD-1 antibody. In a specific aspect, a PD-1 binding antagonist is MDX-1106 (nivolumab) described herein. In another specific aspect, a PD-1 binding antagonist is MK-3475 (pembrolizumab) described herein. In another specific aspect, a PD-1 binding antagonist is MEDI-0680 (AMP-514) described herein. In another specific aspect, a PD-1 binding antagonist is PDR001 described herein. In another specific aspect, a PD-1 binding antagonist is REGN2810 described herein. In another specific aspect, a PD-1 binding antagonist is BGB-108 described herein.

The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one embodiment, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

A "taxane" as used herein is a diterpene which may bind to tubulin, promoting microtubule assembly and stabilization and/or prevent microtubule depolymerization. Taxanes included herein include taxoid 10-deacetylbaccatin III and/or derivatives thereof. Exemplary taxanes include, but are not limited to, paclitaxel (i.e., TAXOL®, CAS # 33069-62-4), docetaxel (i.e., TAXOTERE®, CAS # 114977-28-5), larotaxel, cabazitaxel, milataxel, tesetaxel, and/or orataxel. In some embodiments, the taxane is an albumin-coated nanoparticle (e.g., nano-albumin bound (nab)-paclitaxel, i.e., ABRAXANE® and/or nab-docetaxel, ABI-008). In some embodiments, the taxane is nab-paclitaxel (ABRAXANE®). In some embodiments, the taxane is formulated in CREMAPHOR® (e.g., TAXOL®) and/or in TWEEN® such as polysorbate 80 (e.g., TAXOTERE®). In some embodiments, the taxane is liposome-encapsulated taxane. In some embodiments, the taxane is a prodrug form and/or conjugated form of taxane (e.g., DHA covalently conjugated to paclitaxel, paclitaxel poliglumex, and/or linoleyl carbonate-paclitaxel). In some embodiments, the paclitaxel is formulated with substantially no surfactant (e.g., in the absence of CREMAPHOR® and/or TWEEN®, such as TOCOSOL® paclitaxel).

The term "dysfunction" in the context of immune dysfunction, refers to a state of reduced immune responsiveness to antigenic stimulation. The term includes the common elements of both "exhaustion" and/or "anergy" in which antigen recognition may occur, but the ensuing immune response is ineffective to control infection or tumor growth.

The term "dysfunctional," as used herein, also includes refractory or unresponsive to antigen recognition, specifically, impaired capacity to translate antigen recognition into down-stream T-cell effector functions, such as proliferation, cytokine production (e.g., IL-2) and/or target cell killing.

The term "anergy" refers to the state of unresponsiveness to antigen stimulation resulting from incomplete or insufficient signals delivered through the T-cell receptor (e.g., increase in intracellular $Ca^{+2}$ in the absence of ras-activation). T cell anergy can also result upon stimulation with antigen in the absence of co-stimulation, resulting in the cell becoming refractory to subsequent activation by the antigen even in the context of co-stimulation. The unresponsive state can often be overriden by the presence of Interleukin-2. Anergic T-cells do not undergo clonal expansion and/or acquire effector functions.

The term "exhaustion" refers to T cell exhaustion as a state of T cell dysfunction that arises from sustained TCR signaling that occurs during many chronic infections and cancer. It is distinguished from anergy in that it arises not through incomplete or deficient signaling, but from sustained signaling. It is defined by poor effector function, sustained expression of inhibitory receptors and a transcriptional state distinct from that of functional effector or memory T cells. Exhaustion prevents optimal control of infection and tumors. Exhaustion can result from both extrinsic negative regulatory pathways (e.g., immunoregulatory cytokines) as well as cell intrinsic negative regulatory (costimulatory) pathways (PD-1, B7-H3, B7-H4, etc.).

"Tumor immunity" refers to the process in which tumors evade immune recognition and clearance. Thus, as a therapeutic concept, tumor immunity is "treated" when such evasion is attenuated, and the tumors are recognized and attacked by the immune system. Examples of tumor recognition include tumor binding, tumor shrinkage and tumor clearance.

"Immunogenicity" refers to the ability of a particular substance to provoke an immune response. Tumors are immunogenic and enhancing tumor immunogenicity aids in the clearance of the tumor cells by the immune response. Examples of enhancing tumor immunogenicity include treatment with a PD-1 axis binding antagonist and a taxane.

**The terms "respond to" or "responsive to" in the context of the present invention indicates that a** patient suffering, suspected to suffer or prone to suffer from cancer (e.g., breast cancer (e.g., locally advanced or metastatic TNBC)), shows a response to a therapy, e.g., an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel). A skilled person will readily be in a position to determine whether a person treated with an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel) according to the methods of the invention shows a response. For example, a response may be reflected by decreased suffering from cancer, such as a diminished and/or halted tumor growth, reduction of the size of a tumor, and/or amelioration of one or more symptoms of cancer. Preferably, the response may be reflected by decreased or diminished indices of the metastatic conversion of the cancer or indices of the cancer, e.g., the prevention of the formation of metastases or a reduction of number or size of metastases. A response may be, e.g., a complete response, a partial response, an improvement in progression-free survival, an improvement in overall survival, or a sustained response. In some embodiments, response is an improvement in progression-free survival. In other embodiments, response is an improvement in overall survival.

"Sustained response" refers to the sustained effect on reducing tumor growth after cessation of a treatment. For example, the tumor size may remain to be the same or smaller as compared to the size at the beginning of the administration phase. In some embodiments, the sustained response has a duration at least the same as the treatment duration, at least 1.5X, 2.0X, 2.5X, or 3.0X length of the treatment duration.

As used herein, "reducing or inhibiting cancer relapse" means to reduce or inhibit tumor or cancer relapse or tumor or cancer progression. As disclosed herein, cancer relapse and/or cancer progression include, without limitation, cancer metastasis.

As used herein, "complete response" or "CR" refers to disappearance of all target lesions.

As used herein, "partial response" or "PR" refers to at least a 30% decrease in the sum of the longest diameters (SLD) of target lesions, taking as reference the baseline SLD.

As used herein, "stable disease" or "SD" refers to neither sufficient shrinkage of target lesions to qualify for PR, nor sufficient increase to qualify for PD, taking as reference the smallest SLD since the treatment started.

As used herein, "progressive disease" or "PD" refers to at least a 20% increase in the SLD of target lesions, taking as reference the smallest SLD recorded since the treatment started or the presence of one or more new lesions.

As used herein, "progression-free survival" (PFS) refers to the length of time during and after treatment during which the disease being treated (e.g., cancer) does not get worse. Progression-free survival may include the amount of time patients have experienced a complete response or a partial response, as well as the amount of time patients have experienced stable disease.

As used herein, "overall response rate" or "objective response rate" (ORR) refers to the sum of complete response (CR) rate and partial response (PR) rate.

As used herein, "overall survival" (OS) refers to the percentage of individuals in a group who are likely to be alive after a particular duration of time.

The term "pharmaceutical formulation" refers to a preparation which is in such form as to permit the biological activity of the active ingredient to be effective, and which contains no additional components which are unacceptably toxic to a subject to which the formulation would be administered. Such formulations are sterile. "Pharmaceutically acceptable" excipients (vehicles, additives) are those which can reasonably be administered to a subject mammal to provide an effective dose of the active ingredient employed.

As used herein, the term "treatment" refers to clinical intervention designed to alter the natural course of the individual or cell being treated during the course of clinical pathology. Desirable effects of treatment include decreasing the rate of disease progression, ameliorating or palliating the disease state, and remission or improved prognosis. For example, an individual is successfully "treated" if one or more symptoms associated with cancer are mitigated or eliminated, including, but are not limited to, reducing the proliferation of (or destroying) cancerous cells, decreasing symptoms resulting from the disease, increasing the quality of life of those suffering from the disease, decreasing the dose of other medications required to treat the disease, and/or prolonging survival of individuals.

As used herein, "delaying progression" of a disease means to defer, hinder, slow, retard, stabilize, and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

An "effective amount" or "therapeutically effective amount," as used interchangeably herein, is at least the minimum amount required to effect a measurable improvement or prevention of a particular disorder. An effective amount herein may vary according to factors such as the disease state, age, sex, and weight of the patient, and the ability of the agent to elicit a desired response in the individual. An effective amount is also one in which any toxic or detrimental effects of the treatment are outweighed by the therapeutically beneficial effects. For prophylactic use, beneficial or desired results include results such as eliminating or reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as decreasing one or more symptoms resulting from the disease, increasing the quality of life of those suffering from the disease, decreasing the dose of other medications required to treat the disease, and enhancing effect of another medication such as via targeting, delaying the progression of the disease, and/or prolonging survival. In the case of a cancer or a tumor, an effective amount of the drug may have the effect in reducing the number of cancer cells; reducing the tumor size; inhibiting (i.e., slow to some extent or desirably stop) cancer cell infiltration into peripheral organs; inhibit (i.e., slow to some extent and desirably stop) tumor metastasis; inhibiting to some extent tumor growth; and/or relieving to some extent one or more of the symptoms associated with the disorder. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

As used herein, "in combination with" or "in conjunction with" refer to administration of one treatment modality in addition to another treatment modality. As such, "in conjunction with" refers to administration of one treatment modality before, during, or after administration of the other treatment modality to the individual.

A "disorder" is any condition that would benefit from treatment including, but not limited to, chronic and acute disorders or diseases including those pathological conditions which predispose the mammal to the disorder in question.

The terms "cell proliferative disorder" and "proliferative disorder" refer to disorders that are associated with some degree of abnormal cell proliferation. In one embodiment, the cell proliferative disorder is cancer. In one embodiment, the cell proliferative disorder is a tumor.

The term "tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder," and "tumor" are not mutually exclusive as referred to herein.

The terms "cancer" and "cancerous" refer to or describe the physiological condition in mammals that is typically characterized by unregulated cell growth. The term "breast cancer" includes, but is not limited to, HER2+ breast cancer and triple-negative breast cancer (TNBC), which is a form of breast cancer in which the cancer cells are negative for estrogen receptors (ER-), progesterone receptors (PR-), and HER2 (HER2-), and which may be locally advanced, unresectable, and/or metastatic (e.g., metastatic triple-negative breast cancer (mTNBC)). The methods described herein are suitable for treatment of various stages of cancer, including cancers that are locally advanced and/or metastatic. In cancer staging, locally advanced is generally defined as cancer that has spread from a localized area to nearby tissues and/or lymph nodes. In the Roman numeral staging system, locally advanced usually is classified in Stage II or III. Cancer which is metastatic is a stage where the cancer spreads throughout the body to distant tissues and organs (stage IV).

The term "cytotoxic agent" as used herein refers to any agent that is detrimental to cells (e.g., causes cell death, inhibits proliferation, or otherwise hinders a cellular function). Cytotoxic agents include, but are not limited to, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents; growth inhibitory agents; enzymes and fragments thereof such as nucleolytic enzymes; and toxins such as small molecule toxins or enzymatically active toxins of bacterial, fungal, plant or animal origin, including fragments and/or variants thereof. Exemplary cytotoxic agents can be selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine kinase angiogenesis inhibitors, immunotherapeutic agents, proapoptotic agents, inhibitors of LDH-A, inhibitors of fatty acid biosynthesis, cell cycle signalling inhibitors, HDAC inhibitors, proteasome inhibitors, and inhibitors of cancer metabolism. In one embodiment the cytotoxic agent is a platinum-based chemotherapeutic agent. In one embodiment the cytotoxic agent is an antagonist of EGFR. In one embodiment the cytotoxic agent is N-(3-ethynylphenyl)-6,7-bis(2-methoxyethoxy)quinazolin-4-amine (e.g., erlotinib, TARCEVA™). In one embodiment the cytotoxic agent is a RAF inhibitor. In one embodiment, the RAF inhibitor is a BRAF and/or CRAF inhibitor. In one embodiment the RAF inhibitor is vemurafenib. In one embodiment the cytotoxic agent is a PI3K inhibitor.

As used herein, the term "chemotherapeutic agent" includes compounds useful in the treatment of cancer, such as mTNBC. Examples of chemotherapeutic agents include erlotinib (TARCEVA®, Genentech/OSI Pharm.), bortezomib (VELCADE®, Millennium Pharm.), disulfiram, epigallocatechin gallate, salinosporamide A, carfilzomib, 17-AAG (geldanamycin), radicicol, lactate dehydrogenase A (LDH-A), fulvestrant (FASLODEX®, AstraZeneca), sunitib (SUTENT®, Pfizer/Sugen), letrozole (FEMARA®, Novartis), imatinib mesylate (GLEEVEC®, Novartis), finasunate (VATALANIB®, Novartis), oxaliplatin (ELOXATIN®, Sanofi), 5-FU (5-fluorouracil), leucovorin, rapamycin (Sirolimus, RAPAMUNE®, Wyeth), Lapatinib (TYKERB®, GSK572016, Glaxo Smith Kline), lonafamib (SCH 66336), sorafenib (NEXAVAR®, Bayer Labs), gefitinib (IRESSA®, AstraZeneca), AG1478, alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; callystatin; CC-1065 (including its adozelesin, carzelesin and bizelesin synthetic analogs); cryptophycins (particularly cryptophycin 1 and cryptophycin 8); adrenocorticosteroids (including prednisone and prednisolone); cyproterone acetate; $5\alpha$-reductases including finasteride and dutasteride); vorinostat, romidepsin, panobinostat, valproic acid, mocetinostat dolastatin; aldesleukin, talc duocarmycin (including the synthetic analogs, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin $\gamma1I$ and calicheamicin $\omega1I$ (Angew Chem. Inti. Ed. Engl. 33:183-186 (1994)); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxanes; chloranmbucil; GEMZAR® (gemcitabine); 6-thioguanine; mercaptopurine; methotrexate; vinblastine; etoposide (VP-16); ifosfamide; mitoxantrone; vincristine; NAVELBINE® (vinorelbine); novantrone; teniposide; edatrexate; daunomycin; aminopterin; capecitabine (XELODA®); ibandronate; CPT-11; topoisomerase inhibitor RFS 2000; difluoromethylornithine (DMFO); retinoids such as retinoic acid; and pharmaceutically acceptable salts, acids, and derivatives of any of the above.

Chemotherapeutic agents also include "platinum-based" chemotherapeutic agents, which comprise an organic compound which contains platinum as an integral part of the molecule. Typically platinum-based chemotherapeutic agents are coordination complexes of platinum. Platinum-based chemotherapeutic agents are sometimes called "platins" in the art. Examples of platinum-based chemotherapeutic agents include, but are not limited to, carboplatin, cisplatin, and oxaliplatin.

Chemotherapeutic agents also include (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, iodoxyfene, 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN®, rIL-2; a topoisomerase 1 inhibitor such as LURTOTECAN®; ABARELIX® rmRH; and (ix) pharmaceutically acceptable salts, acids, and derivatives of any of the above.

Chemotherapeutic agents also include antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, full-length $IgG_1$ λ antibody genetically modified to recognize interleukin-12 p40 protein.

Chemotherapeutic agents also include "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533) and variants thereof, such as chimerized 225 (C225 or Cetuximab; ERBUTIX®) and reshaped human 225 (H225) (see, e.g., WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al., Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6. 3 and E7.6. 3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, e.g., EP659439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA® Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA®) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER-targeted tyrosine kinase inhibitors such as imatinib mesylate (GLEEVEC®, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (e.g., those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC®); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone), rapamycin (sirolimus, RAPAMUNE®); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca).

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate and fluprednidene acetate; immune selective anti-inflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, **tumor necrosis factor alpha (TNFα) blockers such as etanercept (Enbrel), infliximab (Remicade),** adalimumab (Humira), certolizumab pegol (Cimzia), golimumab (Simponi), Interleukin 1 (IL-1) blockers such as anakinra (Kineret), T cell costimulation blockers such as abatacept (Orencia), Interleukin 6 (IL-6) blockers such as tocilizumab (ACTEMERA®); Interleukin 13 (IL-13) blockers such as lebrikizumab; Interferon alpha (IFN) blockers such as rontalizumab; Beta 7 integrin blockers such as rhuMAb Beta7; IgE pathway blockers such as Anti-M1 prime; Secreted homotrimeric LTa3 and membrane bound heterotrimer LTa1/β2 blockers such as Anti-lymphotoxin alpha (LTa); radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); miscellaneous investigational agents such as thioplatin, PS-341, phenylbutyrate, ET-18- $OCH_3$, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol, MARINOL®); beta-lapachone; lapachol; colchicines; betulinic acid; acetylcamptothecin, scopolectin, and 9-aminocamptothecin); podophyllotoxin; tegafur (UFTORAL®); bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); and epidermal growth factor receptor (EGF-R); vaccines such as THERATOPE® vaccine; perifosine, COX-2 inhibitor (e.g., celecoxib or etoricoxib), proteosome inhibitor (e.g., PS341); CCI-779; tipifarnib (R11577); orafenib, ABT510; Bcl-2 inhibitor such as oblimersen sodium (GENASENSE®); pixantrone; farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents also include non-steroidal anti-inflammatory drugs with analgesic, antipyretic and anti-inflammatory effects. NSAIDs include non-selective inhibitors of the enzyme cyclooxygenase. Specific examples of NSAIDs include aspirin, propionic acid derivatives such as ibuprofen, fenoprofen, ketoprofen, flurbiprofen, oxaprozin and naproxen, acetic acid derivatives such as indomethacin, sulindac, etodolac, diclofenac, enolic acid derivatives such as piroxicam, meloxicam, tenoxicam, droxicam, lornoxicam and isoxicam, fenamic acid derivatives such as mefenamic acid, meclofenamic acid, flufenamic acid, tolfenamic acid, and COX-2 inhibitors such as celecoxib, etoricoxib, lumiracoxib, parecoxib, rofecoxib, rofecoxib, and valdecoxib. NSAIDs can be indicated for the symptomatic relief of conditions such as rheumatoid arthritis, osteoarthritis, inflammatory arthropathies, ankylosing spondylitis, psoriatic arthritis, Reiter's syndrome, acute gout, dysmenorrhoea, metastatic bone pain, headache and migraine, postoperative pain, mild-to-moderate pain due to inflammation and tissue injury, pyrexia, ileus, and renal colic.

A "growth inhibitory agent" when used herein refers to a compound or composition which inhibits growth of a cell either *in vitro* or *in vivo.* In one embodiment, a growth inhibitory agent is growth inhibitory antibody that prevents or reduces proliferation of a cell expressing an antigen to which the antibody binds. In another embodiment, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in Mendelsohn and Israel, eds., The Molecular Basis of Cancer, Chapter 1, entitled "Cell cycle regulation, oncogenes, and antineoplastic drugs" by Murakami et al. (W.B. Saunders, Philadelphia, 1995), e.g., p. 13.

The term "prodrug" as used herein refers to a precursor or derivative form of a pharmaceutically active substance that is less cytotoxic to tumor cells compared to the parent drug and is capable of being enzymatically activated or converted into the more active parent form. See, for example, Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-tactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

An "anti-angiogenesis agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. It should be understood that the anti-angiogenesis agent includes those agents that bind and block the angiogenic activity of the angiogenic factor or its receptor. For example, an anti-angiogenesis agent is an antibody or other antagonist to an angiogenic agent as defined above, e.g., antibodies to VEGF-A or the VEGF-A receptor (e.g., KDR receptor or Flt-1 receptor), anti-PDGFR inhibitors such as GLEEVEC™ (Imatinib Mesylate). Anti-angiogenesis agents also include native angiogenesis inhibitors, e.g., angiostatin, endostatin, etc. See, for example, Klagsbrun and D'Amore, Annu. Rev. Physiol., 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5(12):1359-1364 (1999); Tonini et al., Oncogene, 22:6549-6556 (2003) and, Sato Int. J. Clin. Oncol., 8:200-206 (2003).

The terms "individual," a "subject," **or a "patient," as used interchangeably herein,** for purposes of treatment refer to any animal classified as a mammal, including humans, domestic and farm animals, and zoo, sports, or pet animals, such as dogs, horses, cats, cows, etc. Preferably, the mammal is human.

The term "antibody" herein is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In some embodiments, an antibody is purified (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. An isolated antibody includes the antibody *in situ* within recombinant cells since at least one component of the antibody's natural environment will not be present. Ordinarily, however, an isolated antibody will be prepared by at least one purification step.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

The term "constant domain" refers to the portion of an immunoglobulin molecule having a more conserved amino acid sequence relative to the other portion of the immunoglobulin, the variable domain, which contains the antigen binding site. The constant domain contains the $C_H1$, $C_H2$ and $C_H3$ domains (collectively, CH) of the heavy chain and the CHL (or CL) domain of the light chain.

The "variable region" or "variable domain" of an antibody refers to the amino-terminal domains of the heavy or light chain of the antibody. The variable domain of the heavy chain may be referred to as "$V_H$." The variable domain of the light chain may be referred to as "$V_L$." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat et al., Sequences of Proteins of Immunological Interest, Fifth Edition, National Institute of Health, Bethesda, Md. (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any mammalian species can be assigned to one of two clearly distinct types, called kappa ("κ") and lambda ("λ"), based on the amino acid sequences of their constant domains.

The term IgG "isotype" or "subclass" as used herein is meant any of the subclasses of immunoglobulins defined by the chemical and antigenic characteristics of their constant regions.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains **that correspond to the different classes of immunoglobulins are called** α, γ, ε, γ, **and** μ, **respectively.** The subunit structures and three-dimensional configurations of different classes of immunoglobulins are well known and described generally in, for example, Abbas et al. Cellular and Mol. Immunology, 4th ed. (W.B. Saunders, Co., 2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

A "naked antibody" for the purposes herein is an antibody that is not conjugated to a cytotoxic moiety or radiolabel.

"Antibody fragments" comprise a portion of an intact antibody, preferably comprising the antigen-binding region thereof. In some embodiments, the antibody fragment described herein is an antigen-binding fragment. Examples of antibody fragments include Fab, Fab', F(ab')$_2$, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')$_2$ fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')$_2$ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv, see, e.g., Pluckthün, in The Pharmacology of Monoclonal Antibodies, vol. 113, Rosenburg and Moore eds., (Springer-Verlag, New York, 1994), pp. 269-315.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

The term "monoclonal antibody" as used herein refers to an antibody obtained from a population of substantially homogeneous antibodies, e.g., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the invention may be made by a variety of techniques, including, for example, the hybridoma method (e.g., Kohler and Milstein, Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Pat. No. 4,816,567), phage-display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Pat. Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg et al., Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see, e.g., U.S. Pat. No. 4,816,567; and Morrison et al., Proc. Natl. Acad. Sci. USA 81:6851-6855 (1984)). Chimeric antibodies include PRIMATIZED® antibodies wherein the antigen-binding region of the antibody is derived from an antibody produced by, e.g., immunizing macaque monkeys with the antigen of interest.

"Humanized" forms of non-human (e.g., murine) antibodies are chimeric antibodies that contain minimal sequence derived from non-human immunoglobulin. In one embodiment, a humanized antibody is a human immunoglobulin (recipient antibody) in which residues from a HVR of the recipient are replaced by residues from a HVR of a non-human species (donor antibody) such as mouse, rat, rabbit, or nonhuman primate having the desired specificity, affinity, and/or capacity. In some embodiments, FR residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see, e.g., Jones et al., Nature 321:522-525 (1986); Riechmann et al., Nature 332:323-329 (1988); and Presta, Curr. Op. Struct. Biol. 2:593-596 (1992). See also, for example, Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

A "species-dependent antibody" is one which has a stronger binding affinity for an antigen from a first mammalian species than it has for a homologue of that antigen from a second mammalian species. Normally, the species-dependent antibody "binds specifically" to a human antigen (e.g., has a binding affinity (Kd) value of no more than about $1 \times 10^{-7}$ M, preferably no more than about $1 \times 10^{-8}$ M and preferably no more than about $1 \times 10^{-9}$ M) but has a binding affinity for a homologue of the antigen from a second nonhuman mammalian species which is at least about 50 fold, or at least about 500 fold, or at least about 1000 fold, weaker than its binding affinity for the human antigen. The species-dependent antibody can be any of the various types of antibodies as defined above, but preferably is a humanized or human antibody.

The term "hypervariable region," "HVR," or "HV," when used herein refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, e.g., Xu et al., Immunity 13:37-45 (2000); Johnson and Wu, in Methods in Molecular Biology 248:1-25 (Lo, ed.,

Human Press, Totowa, N.J., 2003). Indeed, naturally occurring camelid antibodies consisting of a heavy chain only are functional and stable in the absence of light chain. See, e.g., Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat Numbering) |
| H1 | H31-H35 | H26-H35 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat et al., *supra,* for each of these definitions.

"Framework" or "FR" residues are those variable domain residues other than the HVR residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g., residues 82a, 82b, and 82c, etc., according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (e.g., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., *supra*). The "EU index as in Kabat" refers to the residue numbering of the human IgG1 EU antibody.

The expression "linear antibodies" refers to the antibodies described in Zapata et al. (1995 Protein Eng, 8(10):1057-1062). Briefly, these antibodies comprise a pair of tandem Fd segments (VH-CH1-VH-CH1) which, together with complementary light chain polypeptides, form a pair of antigen binding regions. Linear antibodies can be bispecific or monospecific.

As used herein, the term "binds," "specifically binds to," or is "specific for" refers to measurable and reproducible interactions such as binding between a target and an antibody, which is determinative of the presence of the target in the presence of a heterogeneous population of molecules including biological molecules. For example, an antibody that binds to or specifically binds to a target (which can be an epitope) is an antibody that binds this target with greater affinity, avidity, more readily, and/or with greater duration than it binds to other targets. In one embodiment, the extent of binding of an antibody to an unrelated target is less than about 10% of the binding of the antibody to the target as measured, e.g., by a radioimmunoassay (RIA). In certain embodiments, an antibody that specifically binds to a target has a dissociation constant (Kd) of $\leq$ 1$\mu$M, $\leq$ 100 nM, $\leq$ 10 nM, $\leq$ 1 nM, or $\leq$ 0.1 nM. In certain embodiments, an antibody specifically binds to an epitope on a protein that is conserved among the protein from different species. In another embodiment, specific binding can include, but does not require exclusive binding.

"Percent (%) amino acid sequence identity" with respect to the polypeptide sequences identified herein is defined as the percentage of amino acid residues in a candidate sequence that are identical with the amino acid residues in the polypeptide being compared, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program ALIGN-2. The ALIGN-2 sequence comparison computer program was authored by Genentech, Inc. and the source code has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-**2 in that program's alignment of A and B, and where Y is the total number** of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The amino acid sequences described herein are contiguous amino acid sequences unless otherwise specified.

**The term "package insert" is used to refer to instructions customarily included in commercial** packages of therapeutic products, that contain information about the indications, usage, dosage, administration, combination therapy, contraindications and/or warnings concerning the use of such therapeutic products.

**The terms "pharmaceutical formulation" and "pharmaceutical composition" are used** interchangeably herein, and refer to a preparation which is in such form as to permit the biological activity of an active ingredient contained therein to be effective, and which contains no additional components which are unacceptably toxic to a subject to which the formulation would be administered. Such formulations are sterile. In a preferred embodiment, the pharmaceutical composition or pharmaceutical formulation is administered to a human subject.

**A "sterile" pharmaceutical formulation is aseptic or free or essentially free from all living** microorganisms and their spores.

**A "pharmaceutically acceptable carrier" refers to an ingredient in a pharmaceutical formulation,** other than an active ingredient, which is nontoxic to a subject. A pharmaceutically acceptable carrier includes, but is not limited to, a buffer, excipient, stabilizer, or preservative.

**As used herein, "administering" is meant a method of giving a dosage of a compound (e.g.,** a PD-1 axis binding antagonist (e.g., an anti-PD-L1 antibody (e.g., atezolizumab) or an anti-PD-1 antibody) and/or a taxane (e.g., nab-paclitaxel or paclitaxel)) or a composition (e.g., a pharmaceutical composition, e.g., a pharmaceutical composition including a PD-1 axis binding antagonist and/or a taxane, optionally also including an additional therapeutic agent) to a subject. The compositions utilized in the methods described herein can be administered, for example, intravitreally, intramuscularly, intravenously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subcutaneously, subconjunctivally, intravesicularly, mucosally, intrapericardially, intraumbilically, intraocularly, intraorbitally, orally, topically, transdermally, periocularly, conjunctivally, subtenonly, intracamerally, subretinally, retrobulbarly, intracanalicularly, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the compound or composition being administered and the severity of the condition, disease, or disorder being treated).

## III. Diagnostic Methods

Provided herein are diagnostic methods for identifying a patient suffering from a cancer (e.g., a breast cancer) who is likely to respond to treatment with an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel). Also provided herein are methods of predicting whether a patient suffering from a cancer (e.g., a breast cancer) is likely to respond to treatment with an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel). Further provided herein are methods of selecting an anti-cancer therapy for a patient suffering from a cancer (e.g., a breast cancer). In some embodiments, the method includes determining the presence or level of at least one biomarker of the invention, including PD-L1 (e.g., PD-L1 expression on tumor-infiltrating immune cells (IC) in a tumor sample obtained from the patient and/or PD-L1 expression on tumor cells (TC) in a tumor sample obtained from the patient), CD8 (e.g., CD8 expression in a tumor sample obtained from the patient), and/or the presence of stromal TILs (sTILs), e.g., in a tumor sample obtained from the patient. For example, in some embodiments, the patient is PD-L1-positive. In some embodiments, the patient is suffering from a locally advanced or metastatic breast cancer (e.g., locally advanced or metastatic TNBC). In some embodiments, the patient has not been previously treated for the locally advanced or metastatic breast cancer (e.g., locally advanced or metastatic TNBC). In other words, the methods provided herein may be used for treatment-naive patients, for example, to select a first-line therapy for the patient. In some embodiments, whether the patient is likely to respond to treatment with the anti-cancer therapy is determined in terms of progression-free survival. In other embodiments, whether the patient is likely to respond to treatment with the anti-cancer therapy is determined in terms of overall survival.

For example, provided herein is a method for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in a sample (e.g., a tumor sample) obtained from the patient, wherein the patient has not been previously treated for the breast cancer (e.g., the locally advanced or metastatic TNBC), and wherein a detectable expression level of PD-L1 in the sample identifies the patient as likely to respond to treatment with the anti-cancer therapy.

In another example, provided herein is a method for predicting whether a patient suffering from a locally advanced or metastatic breast cancer (e.g., locally advanced or metastatic TNBC) is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in a sample (e.g., a tumor sample) obtained from the patient, wherein the patient has not been previously treated for the breast cancer (e.g., the locally advanced or metastatic TNBC), and wherein a detectable expression level of PD-L1 in the sample indicates that the patient is likely to respond to treatment with the anti-cancer therapy.

In a further example, provided herein is a method for selecting an anti-cancer therapy for a patient suffering from a locally advanced or metastatic breast cancer (e.g., locally advanced or metastatic TNBC), the method comprising: (a) determining the expression level of PD-L1 in a sample (e.g., a tumor sample) obtained from the patient, wherein the patient has not been previously treated for the breast cancer (e.g., the locally advanced or metastatic TNBC); and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in the sample.

Any suitable sample may be used. For example, the sample may be a tumor sample.

In another example, provided herein is a method for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the breast cancer (e.g., the locally advanced or metastatic TNBC), and wherein a detectable expression level of PD-L1 in tumor-infiltrating immune cells that comprise about 1% or more of the tumor sample identifies the patient as likely to respond to treatment with the anti-cancer therapy.

In yet another example, provided herein is a method for predicting whether a patient suffering from a locally advanced or metastatic breast cancer (e.g., locally advanced or metastatic TNBC) is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the breast cancer (e.g., the locally advanced or metastatic TNBC), and wherein a detectable expression level of PD-L1 in tumor-infiltrating immune cells that comprise about 1% or more of the tumor sample indicates that the patient is likely to respond to treatment with the anti-cancer therapy.

In a still further example, provided herein is a method for selecting an anti-cancer therapy for a patient suffering from a locally advanced or metastatic breast cancer (e.g., locally advanced or metastatic TNBC), the method comprising: (a) determining the expression level of PD-L1 in tumor-infiltrating immune cells in a tumor sample obtained from the patient, wherein the patient has not been previously treated for the breast cancer (e.g., the locally advanced or metastatic TNBC); and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in tumor-infiltrating immune cells that comprise about 1% or more of the tumor sample.

In some embodiments of any of the preceding aspects, the locally advanced or metastatic breast cancer is a locally advanced or metastatic TNBC.

In one example, provided herein is a method for identifying a patient suffering from a locally advanced TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1% or more of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1 % or more of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of PD-L1 in tumor-infiltrating immune cells in a tumor sample obtained from the patient, wherein the patient has not been previously treated for the locally advanced TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in tumor-infiltrating immune cells that comprise about 1% or more of the tumor sample.

In a still further example, provided herein is a method for selecting an anti-cancer therapy for a patient suffering from a metastatic TNBC, the method comprising: (a) determining the expression level of PD-L1 in tumor-infiltrating immune cells in a tumor sample obtained from the patient, wherein the patient has not been previously treated for the metastatic TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in tumor-infiltrating immune cells that comprise about 1% or more of the tumor sample.

In one example, provided herein is a method for identifying a patient suffering from a locally advanced TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1% or more of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1 % or more of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of PD-L1 in tumor-infiltrating immune cells in a tumor sample obtained from the patient, wherein the patient has not been previously treated for the locally advanced TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in tumor-infiltrating immune cells that comprise about 1% or more of the tumor sample.

In a still further example, provided herein is a method for selecting an anti-cancer therapy for a patient suffering from a metastatic TNBC, the method comprising: (a) determining the expression level of PD-L1 in tumor-infiltrating immune cells in a tumor sample obtained from the patient, wherein the patient has not been previously treated for the metastatic TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in tumor-infiltrating immune cells that comprise about 1% or more of the tumor sample.

In one example, provided herein is a method for identifying a patient suffering from a locally advanced TNBC who is likely to respond to treatment with an anti-cancer therapy comprising atezolizumab and nab-paclitaxel, the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising anti-cancer therapy comprising atezolizumab and nab-paclitaxel, the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1% or more of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and nab-paclitaxel, the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and nab-paclitaxel, the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1% or more of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of PD-L1 in tumor-infiltrating immune cells in a tumor sample obtained from the patient, wherein the patient has not been previously treated for the locally advanced TNBC; and (b) selecting an anti-cancer therapy comprising atezolizumab and nab-paclitaxel for the patient based on a detectable expression level of PD-L1 in tumor-infiltrating immune cells that comprise about 1% or more of the tumor sample.

In a still further example, provided herein is a method for selecting an anti-cancer therapy for a patient suffering from a metastatic TNBC, the method comprising: (a) determining the expression level of PD-L1 in tumor-infiltrating immune cells in a tumor sample obtained from the patient, wherein the patient has not been previously treated for the metastatic TNBC; and (b) selecting an anti-cancer therapy comprising atezolizumab and nab-paclitaxel for the patient based on a detectable expression level of PD-L1 in tumor-infiltrating immune cells that comprise about 1% or more of the tumor sample.

In one example, provided herein is a method for identifying a patient suffering from a locally advanced TNBC who is likely to respond to treatment with an anti-cancer therapy comprising atezolizumab and paclitaxel, the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising anti-cancer therapy comprising atezolizumab and paclitaxel, the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1% or more of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and paclitaxel, the method comprising determining the expression level of PD-L1 in tumor-infiltrating immune cells in a tumor sample obtained from the patient, wherein the patient has not been previously treated for the locally advanced TNBC, and wherein a detectable expression level of PD-L1 in tumor-infiltrating immune cells that comprise about 1% or more of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and paclitaxel, the method comprising determining the expression level of PD-L1 in tumor-infiltrating immune cells in a tumor sample obtained from the patient, wherein the patient has not been previously treated for the metastatic TNBC, and wherein a detectable expression level of PD-L1 in tumor-infiltrating immune cells that comprise about 1% or more of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of PD-L1 in tumor-infiltrating immune cells in a tumor sample obtained from the patient, wherein the patient has not been previously treated for the locally advanced TNBC; and (b) selecting an anti-cancer therapy comprising atezolizumab and paclitaxel for the patient based on a detectable expression level of PD-L1 in tumor-infiltrating immune cells that comprise about 1% or more of the tumor sample.

In a still further example, provided herein is a method for selecting an anti-cancer therapy for a patient suffering from a metastatic TNBC, the method comprising: (a) determining the expression level of PD-L1 in tumor-infiltrating immune cells in a tumor sample obtained from the patient, wherein the patient has not been previously treated for the metastatic TNBC; and (b) selecting an anti-cancer therapy comprising atezolizumab and paclitaxel for the patient based on a detectable expression level of PD-L1 in tumor-infiltrating immune cells that comprise about 1% or more of the tumor sample.

In some embodiments of any of the preceding methods, the tumor sample obtained from the patient has been determined to have a detectable expression level of PD-L1 in tumor-infiltrating immune cells that comprise about 1% or more (e.g., about 1% or more, 2% or more, 3% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, about 50% or more, about 60% or more, about 70% or more, about 80% or more, about 90% or more, about 95% or more, about 96% or more, about 97% or more, about 98% or more, about 99% or more, or 100%) of the tumor sample. For example, in some embodiments, the tumor sample obtained from the patient has been determined to have a detectable expression level of PD-L1 in tumor-infiltrating immune cells that comprise from about 1% to less than about 5% (e.g., from 1% to 4.9%, from 1% to 4.5%, from 1% to 4%, from 1% to 3.5%, from 1% to 3%, from 1% to 2.5%, or from 1% to 2%) of the tumor sample.

In some embodiments of any of the preceding methods, the tumor sample obtained from the patient has been determined to have a detectable expression level of PD-L1 in about 1% or more (e.g., about 1% or more, 2% or more, 3% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, about 50% or more, about 60% or more, about 70% or more, about 80% or more, about 90% or more, about 95% or more, about 96% or more, about 97% or more, about 98% or more, about 99% or more, or 100%) of the tumor-infiltrating immune cells in the tumor sample. For example, in some embodiments, the tumor sample obtained from the patient has been determined to have a detectable expression level of PD-L1 in from about 1% to less than about 5% (e.g., from 1% to 4.9%, from 1% to 4.5%, from 1% to 4%, from 1% to 3.5%, from 1% to 3%, from 1% to 2.5%, or from 1% to 2%) of the tumor-infiltrating immune cells in the tumor sample.

In other embodiments, the tumor sample obtained from the patient has been determined to have a detectable expression level of PD-L1 in tumor-infiltrating immune cells that comprise about 5% or more of the tumor sample. For example, in some embodiments, the tumor sample obtained from the patient has been determined to have a detectable expression level of PD-L1 in tumor-infiltrating immune cells that comprise from about 5% to less than about 10% (e.g., from 5% to 9.5%, from 5% to 9%, from 5% to 8.5%, from 5% to 8%, from 5% to 7.5%, from 5% to 7%, from 5% to 6.5%, from 5% to 6%, from 5% to 5.5%, from 6% to 9.5%, from 6% to 9%, from 6% to 8.5%, from 6% to 8%, from 6% to 7.5%, from 6% to 7%, from 6% to 6.5%, from 7% to 9.5%, from 7% to 9%, from 7% to 7.5%, from 8% to 9.5%, from 8% to 9%, or from 8% to 8.5%) of the tumor sample.

In yet other embodiments, the tumor sample obtained from the patient has been determined to have a detectable expression level of PD-L1 in about 5% or more of the tumor-infiltrating immune cells in the tumor sample. For example, in some embodiments, the tumor sample obtained from the patient has been determined to have a detectable expression level of PD-L1 in from about 5% to less than about 10% (e.g., from 5% to 9.5%, from 5% to 9%, from 5% to 8.5%, from 5% to 8%, from 5% to 7.5%, from 5% to 7%, from 5% to 6.5%, from 5% to 6%, from 5% to 5.5%, from 6% to 9.5%, from 6% to 9%, from 6% to 8.5%, from 6% to 8%, from 6% to 7.5%, from 6% to 7%, from 6% to 6.5%, from 7% to 9.5%, from 7% to 9%, from 7% to 7.5%, from 8% to 9.5%, from 8% to 9%, or from 8% to 8.5%) of the tumor-infiltrating immune cells in the tumor sample.

In still further embodiments, the tumor sample obtained from the patient has been determined to have a detectable expression level of PD-L1 in tumor-infiltrating immune cells that comprise about 10% or more (e.g., 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 60% or more, 70% or more, 80% or more, 90% or more, 95% or more, 96% or more, 97% or more, 98% or more, 99% or more, or 100%) of the tumor sample.

In still further embodiments, the tumor sample obtained from the patient has been determined to have a detectable expression level of PD-L1 in about 10% or more (e.g., 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 60% or more, 70% or more, 80% or more, 90% or more, 95% or more, 96% or more, 97% or more, 98% or more, 99% or more, or 100%) of the tumor-infiltrating immune cells in the tumor sample.

In yet other embodiments, the tumor sample obtained from the patient has been determined to have a detectable expression level of PD-L1 in about 50% or more (e.g., about 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more) of the tumor cells in the tumor sample and/or a detectable expression level of PD-L1 in tumor-infiltrating immune cells that comprise about 10% or more (e.g., 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 60% or more, 70% or more, 80% or more, 90% or more, 95% or more, 96% or more, 97% or more, 98% or more, 99% or more, or 100%) of the tumor sample.

It is to be understood that in any of the preceding methods, the percentage of the tumor sample comprised by tumor-infiltrating immune cells may be in terms of the percentage of tumor area covered by tumor-infiltrating immune cells in a section of the tumor sample obtained from the patient, for example, as assessed by IHC using an anti-PD-L1 antibody (e.g., the SP142 antibody). See, for example, Example 3 (e.g., Table 5).

In another example, provided herein is a method for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor cells in a tumor sample obtained from the patient, wherein the patient has not been previously treated for the breast cancer (e.g., the locally advanced or metastatic TNBC), and wherein a detectable expression level of PD-L1 in about 1% or more of the tumor cells in the tumor sample identifies the patient as likely to respond to treatment with the anti-cancer therapy.

In yet another example, provided herein is a method for predicting whether a patient suffering from a locally advanced or metastatic breast cancer (e.g., locally advanced or metastatic TNBC) is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor cells in a tumor sample obtained from the patient, wherein the patient has not been previously treated for the breast cancer (e.g., the locally advanced or metastatic TNBC), and wherein a detectable expression level of PD-L1 in about 1% or more of the tumor cells in the tumor sample indicates that the patient is likely to respond to treatment with the anti-cancer therapy.

In a further example, provided herein is a method for selecting an anti-cancer therapy for a patient suffering from a locally advanced or metastatic breast cancer (e.g., locally advanced or metastatic TNBC), the method comprising: (a) determining the expression level of PD-L1 in tumor cells in a tumor sample obtained from the patient, wherein the patient has not been previously treated for the breast cancer (e.g., the locally advanced or metastatic TNBC); and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in about 1% or more of the tumor cells in the tumor sample.

In some embodiments of any of the preceding aspects, the locally advanced or metastatic breast cancer is a locally advanced or metastatic TNBC.

In one example, provided herein is a method for identifying a patient suffering from a locally advanced TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor cells in a tumor sample obtained from the patient, wherein the patient has not been previously treated for the locally advanced TNBC, and wherein a detectable expression level of PD-L1 about 1% or more of the tumor cells in the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor cells in a tumor sample obtained from the patient, wherein the patient has not been previously treated for the metastatic TNBC, and wherein a detectable expression level of PD-L1 in about 1% or more of the tumor cells in the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor cells in a tumor sample obtained from the patient, wherein the patient has not been previously treated for the locally advanced TNBC, and wherein a detectable expression level of PD-L1 in about 1% or more of the tumor cells in the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of PD-L1 in tumor cells in a tumor sample obtained from the patient, wherein the patient has not been previously treated for the metastatic TNBC, and wherein a detectable expression level of PD-L1 in about 1% or more of the tumor cells in the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of PD-L1 in tumor cells in a tumor sample obtained from the patient, wherein the patient has not been previously treated for the locally advanced TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in about 1% or more of the tumor cells in the tumor sample.

In a still further example, provided herein is a method for selecting an anti-cancer therapy for a patient suffering from a metastatic TNBC, the method comprising: (a) determining the expression level of PD-L1 in tumor cells in a tumor sample obtained from the patient, wherein the patient has not been previously treated for the metastatic TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of PD-L1 in about 1% or more of the tumor cells in the tumor sample.

In one example, provided herein is a method for identifying a patient suffering from a locally advanced TNBC who is likely to respond to treatment with an anti-cancer therapy comprising atezolizumab and nab-paclitaxel, the method comprising determining the expression level of PD-L1 in tumor cells in a tumor sample obtained from the patient, wherein the patient has not been previously treated for the locally advanced TNBC, and wherein a detectable expression level of PD-L1 about 1% or more of the tumor cells in the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising atezolizumab and nab-paclitaxel, the method comprising determining the expression level of PD-L1 in tumor cells in a tumor sample obtained from the patient, wherein the patient has not been previously treated for the metastatic TNBC, and wherein a detectable expression level of PD-L1 in about 1% or more of the tumor cells in the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and nab-paclitaxel, the method comprising determining the expression level of PD-L1 in tumor cells in a tumor sample obtained from the patient, wherein the patient has not been previously treated for the locally advanced TNBC, and wherein a detectable expression level of PD-L1 in about 1% or more of the tumor cells in the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and nab-paclitaxel, the method comprising determining the expression level of PD-L1 in tumor cells in a tumor sample obtained from the patient, wherein the patient has not been previously treated for the metastatic TNBC, and wherein a detectable expression level of PD-L1 in about 1% or more of the tumor cells in the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of PD-L1 in tumor cells in a tumor sample obtained from the patient, wherein the patient has not been previously treated for the locally advanced TNBC; and (b) selecting an anti-cancer therapy comprising atezolizumab and nab-paclitaxel for the patient based on a detectable expression level of PD-L1 in about 1% or more of the tumor cells in the tumor sample.

In a still further example, provided herein is a method for selecting an anti-cancer therapy for a patient suffering from a metastatic TNBC, the method comprising: (a) determining the expression level of PD-L1 in tumor cells in a tumor sample obtained from the patient, wherein the patient has not been previously treated for the metastatic TNBC; and (b) selecting an anti-cancer therapy comprising atezolizumab and nab-paclitaxel for the patient based on a detectable expression level of PD-L1 in about 1 % or more of the tumor cells in the tumor sample.

In one example, provided herein is a method for identifying a patient suffering from a locally advanced TNBC who is likely to respond to treatment with an anti-cancer therapy comprising atezolizumab and paclitaxel, the method comprising determining the expression level of PD-L1 in tumor cells in a tumor sample obtained from the patient, wherein the patient has not been previously treated for the locally advanced TNBC, and wherein a detectable expression level of PD-L1 about 1% or more of the tumor cells in the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising atezolizumab and paclitaxel, the method comprising determining the expression level of PD-L1 in tumor cells in a tumor sample obtained from the patient, wherein the patient has not been previously treated for the metastatic TNBC, and wherein a detectable expression level of PD-L1 in about 1% or more of the tumor cells in the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and paclitaxel, the method comprising determining the expression level of PD-L1 in tumor cells in a tumor sample obtained from the patient, wherein the patient has not been previously treated for the locally advanced TNBC, and wherein a detectable expression level of PD-L1 in about 1% or more of the tumor cells in the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and paclitaxel, the method comprising determining the expression level of PD-L1 in tumor cells in a tumor sample obtained from the patient, wherein the patient has not been previously treated for the metastatic TNBC, and wherein a detectable expression level of PD-L1 in about 1% or more of the tumor cells in the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of PD-L1 in tumor cells in a tumor sample obtained from the patient, wherein the patient has not been previously treated for the locally advanced TNBC; and (b) selecting an anti-cancer therapy comprising atezolizumab and paclitaxel for the patient based on a detectable expression level of PD-L1 in about 1% or more of the tumor cells in the tumor sample.

In a still further example, provided herein is a method for selecting an anti-cancer therapy for a patient suffering from a metastatic TNBC, the method comprising: (a) determining the expression level of PD-L1 in tumor cells in a tumor sample obtained from the patient, wherein the patient has not been previously treated for the metastatic TNBC; and (b) selecting an anti-cancer therapy comprising atezolizumab and paclitaxel for the patient based on a detectable expression level of PD-L1 in about 1% or more of the tumor cells in the tumor sample.

In some embodiments of any of the preceding methods, a tumor sample obtained from the patient has been determined to have a detectable expression level of PD-L1 in about 1% or more (e.g., about 1% or more, 2% or more, 3% or more, 5% or more, 6% or more, 7% or more, 8% or more, 9% or more, 10% or more, 11% or more, 12% or more, 13% or more, 14% or more, 15% or more, 16% or more, 17% or more,18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more) of the tumor cells in the tumor sample. For example, in some embodiments, the tumor sample obtained from the patient has been determined to have a detectable expression level of PD-L1 in from about 1% to less than about 5% (e.g., from 1% to 4.9%, from 1% to 4.5%, from 1% to 4%, from 1% to 3.5%, from 1% to 3%, from 1% to 2.5%, or from 1% to 2%) of the tumor cells in the tumor sample. In other embodiments, a tumor sample obtained from the patient has been determined to have a detectable expression level of PD-L1 in less than about 1% of the tumor cells in the tumor sample.

In other embodiments, the tumor sample obtained from the patient has been determined to have a detectable expression level of PD-L1 in about 5% or more of the tumor cells in the tumor sample. For example, in some embodiments, the tumor sample obtained from the patient has been determined to have a detectable expression level of PD-L1 in from about 5% to less than 50% (e.g., from 5% to 49.5%, from 5% to 45%, from 5% to 40%, from 5% to 35%, from 5% to 30%, from 5% to 25%, from 5% to 20%, from 5% to 15%, from 5% to 10%, from 5% to 9%, from 5% to 8%, from 5% to 7%, from 5% to 6%, from 10% to 49.5%, from 10% to 40%, from 10% to 35%, from 10% to 30%, from 10% to 25%, from 10% to 20%, from 10% to 15%, from 15% to 49.5%, from 15% to 45%, from 15% to 40%, from 15% to 35%, from 15% to 30%, from 15% to 30%, from 15% to 25%, from 15% to 20%, from 20% to 49.5%, from 20% to 45%, from 20% to 40%, from 20% to 35%, from 20% to 30%, from 20% to 25%, from 25% to 49.5%, from 25% to 45%, from 25% to 40%, from 25% to 35%, from 25% to 30%, from 30% to 49.5%, from 30% to 45%, from 30% to 40%, from 30% to 35%, from 35% to 49.5%, from 35% to 45%, from 35% to 40%, from 40% to 49.5%, from 40% to 45%, or from 45% to 49.5%) of the tumor cells in the tumor sample.

In yet other embodiments, the tumor sample obtained from the patient has been determined to have a detectable expression level of PD-L1 in about 50% or more (e.g., about 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more) of the tumor cells in the tumor sample. In some embodiments, the tumor sample obtained from the patient has been determined to have a detectable expression level of PD-L1 in from about 50% to about 99% (e.g., from 50% to 99%, from 50% to 95%, from 50% to 90%, from 50% to 85%, from 50% to 80%, from 50% to 75%, from 50% to 70%, from 50% to 65%, from 50% to 60%, from 50% to 55%, from 55% to 99%, from 55% to 95%, from 55% to 90%, from 55% to 85%, from 55% to 80%, from 55% to 75%, from 55% to 70%, from 55% to 65%, from 55% to 60%, from 60% to 99%, from 60% to 95%, from 60% to 90%, from 60% to 85%, from 60% to 80%, from 60% to 75%, from 60% to 70%, from 60% to 65%, from 65% to 99%, from 65% to 95%, from 65% to 90%, from 65% to 85%, from 65% to 80%, from 65% to 75%, from 65% to 70%, from 70% to 99%, from 70% to 95%, from 70% to 90%, from 70% to 85%, from 70% to 80%, from 70% to 75%, from 75% to 99%, from 75% to 95%, from 75% to 90%, from 75% to 85%, from 75% to 80%, from 80% to 99%, from 80% to 95%, from 80% to 90%, from 80% to 85%, from 85% to 99%, from 85% to 95%, from 85% to 90%, from 90% to 99%, or from 90% to 95%) of the tumor cells in the tumor sample.

In another example, provided herein is a method for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of CD8 in tumor-infiltrating immune cells in a tumor sample obtained from the patient, wherein the patient has not been previously treated for the breast cancer (e.g., the locally advanced or metastatic TNBC), and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample identifies the patient as likely to respond to treatment with the anti-cancer therapy.

In another example, provided herein is a method for predicting whether a patient suffering from a locally advanced or metastatic breast cancer (e.g., locally advanced or metastatic TNBC) is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of CD8 in tumor-infiltrating immune cells in a tumor sample obtained from the patient, wherein the patient has not been previously treated for the breast cancer (e.g., the locally advanced or metastatic TNBC), and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample indicates that the patient is likely to respond to treatment with the anti-cancer therapy.

In a further example, provided herein is a method for selecting an anti-cancer therapy for a patient suffering from a locally advanced or metastatic breast cancer (e.g., locally advanced or metastatic TNBC), the method comprising: (a) determining the expression level of CD8 in tumor-infiltrating immune cells in a tumor sample obtained from the patient, wherein the patient has not been previously treated for the breast cancer (e.g., the locally advanced or metastatic TNBC); and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

Any of the methods described herein may include determining the presence and/or expression level of two or more of PD-L1, CD8, and sTILs. For example, in some embodiments, the method includes determining the presence and/or expression level of PD-L1 and CD8. In another embodiment, the method includes determining the presence and/or expression level of PD-L1 and sTILs. In another embodiment, the method includes determining the presence and/or expression level of CD8 and sTILs. In another embodiment, the method includes determining the presence and/or expression level of PD-L1, CD8, and sTILs.

In some embodiments of any of the preceding aspects, the locally advanced or metastatic breast cancer is a locally advanced or metastatic TNBC.

In one example, provided herein is a method for identifying a patient suffering from a locally advanced TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of CD8 in tumor-infiltrating immune cells in a tumor sample obtained from the patient, wherein the patient has not been previously treated for the locally advanced TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of CD8 in tumor-infiltrating immune cells in a tumor sample obtained from the patient, wherein the patient has not been previously treated for the metastatic TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of CD8 in tumor-infiltrating immune cells in a tumor sample obtained from the patient, wherein the patient has not been previously treated for the locally advanced TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the expression level of CD8 in tumor-infiltrating immune cells in a tumor sample obtained from the patient, wherein the patient has not been previously treated for the metastatic TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of CD8 in tumor-infiltrating immune cells in a tumor sample obtained from the patient, wherein the patient has not been previously treated for the locally advanced TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In a still further example, provided herein is a method for selecting an anti-cancer therapy for a patient suffering from a metastatic TNBC, the method comprising: (a) determining the expression level of CD8 in tumor-infiltrating immune cells in a tumor sample obtained from the patient, wherein the patient has not been previously treated for the metastatic TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In one example, provided herein is a method for identifying a patient suffering from a locally advanced TNBC who is likely to respond to treatment with an anti-cancer therapy comprising atezolizumab and nab-paclitaxel, the method comprising determining the expression level of CD8 in tumor-infiltrating immune cells in a tumor sample obtained from the patient, wherein the patient has not been previously treated for the locally advanced TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising atezolizumab and nab-paclitaxel, the method comprising determining the expression level of CD8 in tumor-infiltrating immune cells in a tumor sample obtained from the patient, wherein the patient has not been previously treated for the metastatic TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and nab-paclitaxel, the method comprising determining the expression level of CD8 in tumor-infiltrating immune cells in a tumor sample obtained from the patient, wherein the patient has not been previously treated for the locally advanced TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and nab-paclitaxel, the method comprising determining the expression level of CD8 in tumor-infiltrating immune cells in a tumor sample obtained from the patient, wherein the patient has not been previously treated for the metastatic TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of CD8 in tumor-infiltrating immune cells in a tumor sample obtained from the patient, wherein the patient has not been previously treated for the locally advanced TNBC; and (b) selecting an anti-cancer therapy comprising atezolizumab and nab-paclitaxel for the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In a still further example, provided herein is a method for selecting an anti-cancer therapy for a patient suffering from a metastatic TNBC, the method comprising: (a) determining the expression level of CD8 in tumor-infiltrating immune cells in a tumor sample obtained from the patient, wherein the patient has not been previously treated for the metastatic TNBC; and (b) selecting an anti-cancer therapy comprising atezolizumab and nab-paclitaxel for the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In one example, provided herein is a method for identifying a patient suffering from a locally advanced TNBC who is likely to respond to treatment with an anti-cancer therapy comprising atezolizumab and paclitaxel, the method comprising determining the expression level of CD8 in tumor-infiltrating immune cells in a tumor sample obtained from the patient, wherein the patient has not been previously treated for the locally advanced TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising atezolizumab and paclitaxel, the method comprising determining the expression level of CD8 in tumor-infiltrating immune cells in a tumor sample obtained from the patient, wherein the patient has not been previously treated for the metastatic TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and paclitaxel, the method comprising determining the expression level of CD8 in tumor-infiltrating immune cells in a tumor sample obtained from the patient, wherein the patient has not been previously treated for the locally advanced TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and paclitaxel, the method comprising determining the expression level of CD8 in tumor-infiltrating immune cells in a tumor sample obtained from the patient, wherein the patient has not been previously treated for the metastatic TNBC, and wherein a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the expression level of CD8 in tumor-infiltrating immune cells in a tumor sample obtained from the patient, wherein the patient has not been previously treated for the locally advanced TNBC; and (b) selecting an anti-cancer therapy comprising atezolizumab and paclitaxel for the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In a still further example, provided herein is a method for selecting an anti-cancer therapy for a patient suffering from a metastatic TNBC, the method comprising: (a) determining the expression level of CD8 in tumor-infiltrating immune cells in a tumor sample obtained from the patient, wherein the patient has not been previously treated for the metastatic TNBC; and (b) selecting an anti-cancer therapy comprising atezolizumab and paclitaxel for the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

For example, in some embodiments, the tumor sample obtained from the patient has been determined to have a detectable expression level of CD8 in tumor-infiltrating immune cells that comprise about 0.5% or more, about 0.55% or more, about 0.6% or more, about 0.65% or more, about 0.7% or more, about 0.75% or more, about 0.8% or more, about 0.85% or more, about 0.9% or more, about 0.95% or more, about 1% or more, about 1.05% or more, about 1.1% or more, about 1.2% or more, about 1.25% or more, about 1.3% or more, about 1.35% or more, about 1.4% or more, about 1.45% or more, about 1.5% or more, about 1.55% or more, about 1.6% or more, about 1.65% or more, about 1.7% or more, about 1.75% or more, about 1.8% or more, about 1.85% or more, about 1.9% or more, about 1.95% or more, about 2% or more, about 2.1% or more, about 2.2% or more, about 2.3% or more, about 2.4% or more, about 2.5% or more, about 2.6% or more, about 2.7% or more, about 2.8% or more, about 2.9% or more, about 3% or more, about 3.1% or more, about 3.2% or more, about 3.3% or more, about 3.4% or more, about 3.5% or more, about 3.6% or more, about 3.7% or more, about 3.8% or more, about 3.9% or more, about 4% or more, about 4.1% or more, about 4.2% or more, about 4.3% or more, about 4.4% or more, about 4.5% or more, about 4.6% or more, about 4.7% or more, about 4.8% or more, about 4.9% or more, about 5% or more, about 5.5% or more, about 6% or more, about 6.5% or more, about 7% or more, about 7.5% or more, about 8% or more, about 8.5% or more, about 9% or more, about 9.5% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 21%, about 22% or more, about 23% or more, about 24% or more, about 25% or more, about 26% or more, about 27% or more, about 28% or more, about 29% or more, about 30% or more, about 31% or more, about 32% or more, about 33% or more, about 34% or more, about 35% or more, about 36% or more, about 37% or more, about 38% or more, about 39% or more, about 40% or more, about 41% or more, about 42% or more, about 43% or more, about 44% or more, about 45% or more, about 46% or more, about 47% or more, about 48% or more, about 49% or more, about 50% or more, about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more) of the tumor sample.

For example, in some embodiments, the tumor sample obtained from the patient has been determined to have a detectable expression level of CD8 in tumor-infiltrating immune cells that comprise about 0.5% to 50%, from 0.5% to 45%, from 0.5% to 40%, from 0.5% to 35%, from 0.5% to 30%, from 0.5% to 25%, from 0.5% to 20%, from 0.5% to 15%, from 0.5% to 10%, from 0.5% to 9%, from 0.5% to 8%, from 0.5% to 7%, from 0.5% to 6%, from 0.5% to 5%, from 0.5% to 4%, from 0.5% to 3%, from 0.5% to 2%, from 0.5% to 1%, from 1% to 50%, from 1% to 40%, from 1% to 35%, from 1% to 30%, from 1% to 25%, from 1% to 20%, from 1% to 15%, from 1% to 10%, from 1% to 9%, from 1% to 8%, from 1% to 7%, from 1% to 6%, from 1% to 5%, from 1% to 4%, from 1% to 3%, from 1% to 2%, from 1.35% to 50%, from 1.35% to 45%, from 1.35% to 40%, from 1.35% to 35%, from 1.35% to 30%, from 1.35% to 30%, from 1.35% to 25%, from 1.35% to 20%, from 1.35% to 15%, from 1.35% to 10%, from 1.35% to 9%, from 1.35% to 8%, from 1.35% to 7%, from 1.35% to 6%, from 1.35% to 5%, from 1.35% to 4%, from 1.35% to 3%, from 1.35% to 2%, from 1.5% to 50%, from 1.5% to 45%, from 1.5% to 40%, from 1.5% to 35%, from 1.5% to 30%, from 1.5% to 30%, from 1.5% to 25%, from 1.5% to 20%, from 1.5% to 15%, from 1.5% to 10%, from 1.5% to 9%, from 1.5% to 8%, from 1.5% to 7%, from 1.5% to 6%, from 1.5% to 5%, from 1.5% to 4%, from 1.5% to 3%, from 1.5% to 2%, from 2% to 50%, from 2% to 45%, from 2% to 40%, from 2% to 35%, from 2% to 30%, from 2% to 25%, from 2% to 20%, from 2% to 15%, from 2% to 10%, from 2% to 9%, from 2% to 8%, from 2% to 7%, from 2% to 6%, from 2% to 5%, from 2% to 4%, from 2% to 3%, from 3% to 50%, from 3% to 45%, from 3% to 40%, from 3% to 35%, from 3% to 30%, from 3% to 25%, from 3% to 20%, from 3% to 15%, from 3% to 10%, from 3% to 9%, from 3% to 8%, from 3% to 7%, from 3% to 6%, from 3% to 5%, from 3% to 4%, from 4% to 50%, from 4% to 45%, from 4% to 40%, from 4% to 35%, from 4% to 30%, from 4% to 25%, from 4% to 20%, from 4% to 15%, from 4% to 10%, from 4% to 9%, from 4% to 8%, from 4% to 7%, from 4% to 6%, from 4% to 5%, from 5% to 50%, from 5% to 45%, from 5% to 40%, from 5% to 35%, from 5% to 30%, from 5% to 25%, from 5% to 20%, from 5% to 15%, from 5% to 10%, from 5% to 9%, from 5% to 8%, from 5% to 7%, from 5% to 6%, from 6% to 50%, from 6% to 45%, from 6% to 40%, from 6% to 35%, from 6% to 30%, from 6% to 25%, from 6% to 20%, from 6% to 15%, from 6% to 10%, from 6% to 9%, from 6% to 8%, from 6% to 7%, from 7% to 50%, from 7% to 45%, from 7% to 40%, from 7% to 35%, from 7% to 30%, from 7% to 25%, from 7% to 20%, from 7% to 15%, from 7% to 10%, from 7% to 9%, from 7% to 8%, from 8% to 50%, from 8% to 45%, from 8% to 40%, from 8% to 35%, from 8% to 30%, from 8% to 25%, from 8% to 20%, from 8% to 15%, from 8% to 10%, from 8% to 9%, from 9% to 50%, from 9% to 45%, from 9% to 40%, from 9% to 35%, from 9% to 30%, from 9% to 25%, from 9% to 20%, from 9% to 15%, from 9% to 10%, from 10% to 50%, from 10% to 45%, from 10% to 40%, from 10% to 35%, from 10% to 30%, from 10% to 25%, from 10% to 20%, or from 10% to 15% of the tumor sample.

It is to be understood that in any of the preceding methods, the percentage of the tumor sample comprised by tumor-infiltrating immune cells may be in terms of the percentage of tumor area covered by tumor-infiltrating immune cells that express CD8 (e.g., T cells) in a section of the tumor sample. In other embodiments, the percentage of tumor-infiltrating immune cells that express CD8 (e.g., T cells) relative to the total number of tumor-infiltrating immune cells can be used as a biomarker.

For example, provided herein is a method for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the percentage of sTILs in a tumor sample obtained from the patient, wherein the patient has not been previously treated for the breast cancer (e.g., the locally advanced or metastatic TNBC), and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) identifies the patient as likely to respond to treatment with the anti-cancer therapy.

In another example, provided herein is a method for predicting whether a patient suffering from a locally advanced or metastatic breast cancer (e.g., locally advanced or metastatic TNBC) is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the percentage of sTILs in a tumor sample obtained from the patient, wherein the patient has not been previously treated for the breast cancer (e.g., the locally advanced or metastatic TNBC), and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample indicates that the patient is likely to respond to treatment with the anti-cancer therapy.

In a further example, provided herein is a method for selecting an anti-cancer therapy for a patient suffering from a locally advanced or metastatic breast cancer (e.g., locally advanced or metastatic TNBC), the method comprising: (a) determining the percentage of sTILs in a tumor sample obtained from the patient, wherein the patient has not been previously treated for the breast cancer (e.g., the locally advanced or metastatic TNBC); and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In some embodiments of any of the preceding aspects, the locally advanced or metastatic breast cancer is a locally advanced or metastatic TNBC.

In one example, provided herein is a method for identifying a patient suffering from a locally advanced TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the percentage of sTILs in a tumor sample obtained from the patient, wherein the patient has not been previously treated for the locally advanced TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the percentage of sTILs in a tumor sample obtained from the patient, wherein the patient has not been previously treated for the metastatic TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the percentage of sTILs in a tumor sample obtained from the patient, wherein the patient has not been previously treated for the locally advanced TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), the method comprising determining the percentage of sTILs in a tumor sample obtained from the patient, wherein the patient has not been previously treated for the metastatic TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the percentage of sTILs in a tumor sample obtained from the patient, wherein the patient has not been previously treated for the locally advanced TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In a still further example, provided herein is a method for selecting an anti-cancer therapy for a patient suffering from a metastatic TNBC, the method comprising: (a) determining the percentage of sTILs in a tumor sample obtained from the patient, wherein the patient has not been previously treated for the metastatic TNBC; and (b) selecting an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) for the patient based on percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In one example, provided herein is a method for identifying a patient suffering from a locally advanced TNBC who is likely to respond to treatment with an anti-cancer therapy comprising atezolizumab and nab-paclitaxel, the method comprising determining the percentage of sTILs in a tumor sample obtained from the patient, wherein the patient has not been previously treated for the locally advanced TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising atezolizumab and nab-paclitaxel, the method comprising determining the percentage of sTILs in a tumor sample obtained from the patient, wherein the patient has not been previously treated for the metastatic TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and nab-paclitaxel, the method comprising determining the percentage of sTILs in a tumor sample obtained from the patient, wherein the patient has not been previously treated for the locally advanced TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and nab-paclitaxel, the method comprising determining the percentage of sTILs in a tumor sample obtained from the patient, wherein the patient has not been previously treated for the metastatic TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the percentage of sTILs in a tumor sample obtained from the patient, wherein the patient has not been previously treated for the locally advanced TNBC; and (b) selecting an anti-cancer therapy comprising atezolizumab and nab-paclitaxel for the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In a still further example, provided herein is a method for selecting an anti-cancer therapy for a patient suffering from a metastatic TNBC, the method comprising: (a) determining the percentage of sTILs in a tumor sample obtained from the patient, wherein the patient has not been previously treated for the metastatic TNBC; and (b) selecting an anti-cancer therapy comprising atezolizumab and nab-paclitaxel for the patient based on percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In one example, provided herein is a method for identifying a patient suffering from a locally advanced TNBC who is likely to respond to treatment with an anti-cancer therapy comprising atezolizumab and paclitaxel, the method comprising determining the percentage of sTILs in a tumor sample obtained from the patient, wherein the patient has not been previously treated for the locally advanced TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample identifies the patient as likely to respond to treatment with the anti-cancer therapy.

In another example, provided herein is a method for identifying a patient suffering from a metastatic TNBC who is likely to respond to treatment with an anti-cancer therapy comprising atezolizumab and paclitaxel, the method comprising determining the percentage of sTILs in a tumor sample obtained from the patient, wherein the patient has not been previously treated for the metastatic TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample identifies the patient as likely to respond to treatment with the anti-cancer therapy.

In yet another example, provided herein is a method for predicting whether a patient suffering from a locally advanced TNBC is likely to respond to treatment with an anti-cancer therapy comprising atezolizumab and paclitaxel, the method comprising determining the percentage of sTILs in a tumor sample obtained from the patient, wherein the patient has not been previously treated for the locally advanced TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample indicates that the patient is likely to respond to treatment with the anti-cancer therapy.

In a further example, provided herein is a method for predicting whether a patient suffering from a metastatic TNBC is likely to respond to treatment with an anti-cancer therapy comprising atezolizumab and paclitaxel, the method comprising determining the percentage of sTILs in a tumor sample obtained from the patient, wherein the patient has not been previously treated for the metastatic TNBC, and wherein a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample indicates that the patient is likely to respond to treatment with the anti-cancer therapy.

In yet a further example, provided herein is a method for selecting an anti-cancer therapy for a patient suffering from a locally advanced TNBC, the method comprising: (a) determining the percentage of sTILs in a tumor sample obtained from the patient, wherein the patient has not been previously treated for the locally advanced TNBC; and (b) selecting an anti-cancer therapy comprising atezolizumab and paclitaxel for the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In a still further example, provided herein is a method for selecting an anti-cancer therapy for a patient suffering from a metastatic TNBC, the method comprising: (a) determining the percentage of sTILs in a tumor sample obtained from the patient, wherein the patient has not been previously treated for the metastatic TNBC; and (b) selecting an anti-cancer therapy comprising atezolizumab and paclitaxel for the patient based on percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

For example, in some embodiments, the tumor sample obtained from the patient has been determined to have a percentage of sTILs of about 5% or more, about 5.5% or more, about 6% or more, about 6.5% or more, about 7% or more, about 7.5% or more, about 8% or more, about 8.5% or more, about 9% or more, about 9.5% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 21%, about 22% or more, about 23% or more, about 24% or more, about 25% or more, about 26% or more, about 27% or more, about 28% or more, about 29% or more, about 30% or more, about 31% or more, about 32% or more, about 33% or more, about 34% or more, about 35% or more, about 36% or more, about 37% or more, about 38% or more, about 39% or more, about 40% or more, about 41% or more, about 42% or more, about 43% or more, about 44% or more, about 45% or more, about 46% or more, about 47% or more, about 48% or more, about 49% or more, about 50% or more, about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more) of the tumor sample.

For example, in some embodiments, the percentage of sTILs is from about 5% to 50%, from 5% to 45%, from 5% to 40%, from 5% to 35%, from 5% to 30%, from 5% to 25%, from 5% to 20%, from 5% to 15%, from 5% to 10%, from 5% to 9%, from 5% to 8%, from 5% to 7%, from 5% to 6%, from 6% to 50%, from 6% to 45%, from 6% to 40%, from 6% to 35%, from 6% to 30%, from 6% to 25%, from 6% to 20%, from 6% to 15%, from 6% to 10%, from 6% to 9%, from 6% to 8%, from 6% to 7%, from 7% to 50%, from 7% to 45%, from 7% to 40%, from 7% to 35%, from 7% to 30%, from 7% to 25%, from 7% to 20%, from 7% to 15%, from 7% to 10%, from 7% to 9%, from 7% to 8%, from 8% to 50%, from 8% to 45%, from 8% to 40%, from 8% to 35%, from 8% to 30%, from 8% to 25%, from 8% to 20%, from 8% to 15%, from 8% to 10%, from 8% to 9%, from 9% to 50%, from 9% to 45%, from 9% to 40%, from 9% to 35%, from 9% to 30%, from 9% to 25%, from 9% to 20%, from 9% to 15%, from 9% to 10%, from 10% to 50%, from 10% to 45%, from 10% to 40%, from 10% to 35%, from 10% to 30%, from 10% to 25%, from 10% to 20%, or from 10% to 15% of the tumor sample.

It is to be understood that in any of the preceding methods, the percentage of sTILs of the tumor sample may be the area occupied by mononuclear inflammatory cells over the total intratumoral stromal area. The percentage of sTILs may be assessed using any suitable approach known in the art, e.g., as described in Salgado et al. Annals of Oncology 26:259-271, 2015.

In some embodiments of any of the preceding aspects, the patient has received no prior chemotherapy or targeted systemic therapy for the locally advanced or metastatic breast cancer. For example, in some embodiments, the patient has received no prior chemotherapy or targeted systemic therapy for inoperable locally advanced or metastatic TNBC. In some embodiments, the patient has received no prior chemotherapy or targeted systemic therapy for inoperable locally advanced TNBC. In other embodiments, the patient has received no prior chemotherapy or targeted systemic therapy for inoperable metastatic TNBC.

In some embodiments of any of the preceding aspects, the locally advanced TNBC is unresectable.

In some embodiments of any of the preceding aspects, the tumor sample is a formalin-fixed and paraffin-embedded (FFPE) tumor sample, an archival tumor sample, a fresh tumor sample, or a frozen tumor sample.

In some embodiments of any of the preceding aspects, the PD-1 binding antagonist is a human PD-1 binding antagonist, such as an anti-PD-L1 antibody (e.g., atezolizumab) or an anti-PD-1 antibody. In some embodiments, the PD-1 binding antagonist is atezolizumab.

In some embodiments of any of the preceding aspects, the taxane is nab-paclitaxel.

In other embodiments of any of the preceding aspects, the taxane is paclitaxel.

In some embodiments of any of the preceding aspects, whether the patient is likely to respond to treatment with the anti-cancer therapy is determined in terms of progression-free survival. For example, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) **may increase the patient's progression**-free survival by about 1 month, about 2 months, about 2.5 months, about 3 months, about 3.5 months, about 4 months, about 4.5 months, about 5 months, about 5.5 months, about 6 months, about 6.5 months, about 7 months, or longer, as compared to treatment with an anti-cancer therapy comprising the taxane without the PD-1 axis binding antagonist. In one embodiment, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) **may increase the patient's progression**-free survival by about 2.5 months as compared to treatment with an anti-cancer therapy comprising the taxane without the PD-1 axis binding antagonist.

In some embodiments of any of the preceding aspects, whether the patient is likely to respond to treatment with the anti-cancer therapy is determined in terms of overall survival. For example, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) may increase **the patient's** overall survival by about 1 month, about 2 months, about 3 months, about 4 months, about 5 months, about 6 months, about 7 months, about 8 months, about 9 months, about 10 months, about 11 months, about 12 months, about 13 months, about 14 months, or longer, as compared to treatment with an anti-cancer therapy comprising the taxane without the PD-1 axis binding antagonist. In one embodiment, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) may increase the patient's overall survival by about 7 months as compared to treatment with an anti-cancer therapy comprising the taxane without the PD-1 axis binding antagonist.

In some embodiments of any of the preceding aspects, whether the patient is likely to respond to treatment with the anti-cancer therapy is determined in terms of overall response rate.

In some embodiments of any of the preceding aspects, whether the patient is likely to respond to treatment with the anti-cancer therapy is determined in terms of complete response rate.

In some embodiments of any of the preceding aspects, whether the patient is likely to respond to treatment with the anti-cancer therapy is determined in terms of partial response rate.

The presence and/or expression level of any of the biomarkers described above (including PD-L1 (e.g., PD-L1 expression, e.g., on tumor-infiltrating immune cells (IC) in a tumor sample obtained from the patient and/or PD-L1 expression on tumor cells (TC) in a tumor sample obtained from the patient), CD8 (e.g., CD8 expression in a tumor sample obtained from the patient), and/or the presence of stromal TILs (sTILs), e.g., in a tumor sample obtained from the patient) may be assessed qualitatively and/or quantitatively based on any suitable criterion known in the art, including but not limited to DNA, mRNA, cDNA, proteins, protein fragments, and/or gene copy number. Methodologies for measuring such biomarkers are known in the art and understood by the skilled artisan, including, but not limited to, IHC, Western blot analysis, immunoprecipitation, molecular binding assays, ELISA, ELIFA, fluorescence **activated cell sorting**

("FACS"), MassARRAY, proteomics, quantitative blood based assays (e.g., Serum ELISA), biochemical enzymatic activity assays, in situ hybridization (ISH), fluorescence in situ hybridization (FISH), Southern analysis, Northern analysis, whole genome sequencing, polymerase chain reaction (PCR) including quantitative real time PCR (qRT-PCR) and other amplification type detection methods, such as, for example, branched DNA, SISBA, TMA and the like, RNASeq, microarray analysis, gene expression profiling, whole-genome sequencing (WGS), and/or serial analysis of gene expression ("SAGE"), as well as any one of the wide variety of assays that can be performed by protein, gene, and/or tissue array analysis. Typical protocols for evaluating the status of genes and gene products are found, for example, in Ausubel et al. eds. (Current Protocols In Molecular Biology, 1995), Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In some embodiments of any of the preceding methods, the expression level of a biomarker may be a protein expression level. In certain embodiments, the method comprises contacting the sample with antibodies that specifically bind to a biomarker described herein under conditions permissive for binding of the biomarker, and detecting whether a complex is formed between the antibodies and biomarker. Such method may be an *in vitro* or *in vivo* method. In some embodiments, an antibody is used to select patients eligible for treatment with an anti-cancer therapy that includes a PD-1 axis binding antagonist, e.g., an anti-PD-L1 antibody (e.g., atezolizumab) or an anti-PD-1 antibody, e.g., a biomarker for selection of individuals.

Any method of measuring protein expression levels known in the art or provided herein may be used. For example, in some embodiments, a protein expression level of a biomarker is determined using a method selected from the group consisting of immunohistochemistry (IHC), flow cytometry (e.g., fluorescence-activated cell sorting (FACS™)), Western blot, enzyme-linked immunosorbent assay (ELISA), immunoprecipitation, immunofluorescence, radioimmunoassay, dot blotting, immunodetection methods, HPLC, surface plasmon resonance, optical spectroscopy, mass spectrometry, and HPLC.

In some embodiments, the protein expression level of the biomarker is determined in tumor-infiltrating immune cells. In some embodiments, the protein expression level of the biomarker is determined in tumor cells. In some embodiments, the protein expression level of the biomarker is determined in tumor-infiltrating immune cells and/or in tumor cells. In some embodiments, the protein expression level of the biomarker is determined in peripheral blood mononuclear cells (PBMCs).

In certain embodiments, the presence and/or expression level/amount of a biomarker protein in a sample is examined using IHC and staining protocols. IHC staining of tissue sections has been shown to be a reliable method of determining or detecting the presence of proteins in a sample. In some embodiments of any of the methods, assays and/or kits, the biomarker is one or more of the protein expression products of PD-L1 or CD8. In one embodiment, an expression level of biomarker is determined using a method comprising: (a) performing IHC analysis of a sample (such as a tumor sample obtained from a patient) with an antibody; and (b) determining expression level of a biomarker in the sample. In some embodiments, IHC staining intensity is determined relative to a reference. In some embodiments, the reference is a reference value. In some embodiments, the reference is a reference sample (e.g., a control cell line staining sample, a tissue sample from non-cancerous patient, or a tumor sample that is determined to be negative for the biomarker of interest).

For example, in some embodiments, the protein expression level of PD-L1 is determined using IHC. In some embodiments, the protein expression level of PD-L1 is detected using an anti-PD-L1 antibody. Any suitable anti-PD-L1 antibody may be used. In some embodiments, the anti-PD-L1 antibody is SP142.

In another example, in some embodiments, the protein expression level of CD8 is determined using IHC. In some embodiments, the protein expression level of CD8 is detected using an anti-CD8 antibody. Any suitable anti-CD8 antibody may be used.

IHC may be performed in combination with additional techniques such as morphological staining and/or *in situ* hybridization (e.g., ISH). Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for IHC typically will be labeled with a detectable moiety. Numerous labels are available which can be generally grouped into the following categories: (a) radioisotopes, such as $^{35}S$, $^{14}C$, $^{125}I$, $^{3}H$, and $^{131}I$; (b) colloidal gold particles; (c) fluorescent labels including, but are not limited to, rare earth chelates (europium chelates), Texas Red, rhodamine, fluorescein, dansyl, lissamine, umbelliferone, phycocrytherin, phycocyanin, or commercially-available fluorophores such as SPECTRUM ORANGE7 and SPECTRUM GREEN7 and/or derivatives of any one or more of the above; (d) various enzyme-substrate labels are available and U.S. Patent No. 4,275,149 provides a review of some of these. Examples of enzymatic labels include luciferases (e.g., firefly luciferase and bacterial luciferase; see, e.g., U.S. Patent No. 4,737,456), luciferin, 2,3-dihydrophthalazinediones, malate dehydrogenase, urease, peroxidase such as horseradish peroxidase **(HRPO), alkaline phosphatase,** β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example, horseradish peroxidase (HRPO) with hydrogen peroxidase as a substrate; alkaline phosphatase (AP) with para-Nitrophenyl **phosphate as chromogenic substrate; and** β-**D-galactosidase (**β-D-Gal) with a chromogenic substrate (e.g., p-nitrophenyl-(3-D-galactosidase) or fluorogenic substrate (e.g., 4-methylumbelliferyl-β-D-galactosidase). For a general review of these, see, for example, U.S. Patent Nos. 4,275,149 and 4,318,980.

Specimens may be prepared, for example, manually, or using an automated staining instrument (e.g., a Ventana BenchMark XT or Benchmark ULTRA instrument). Specimens thus prepared may be mounted and coverslipped. Slide evaluation is then determined, for example, using a microscope, and staining intensity criteria, routinely used in the art, may be employed. In one embodiment, it is to be understood that when cells and/or tissue from a tumor is examined using IHC, staining can be determined or assessed in tumor cell(s) and/or tissue (as opposed to stromal or surrounding tissue that may be present in the sample). In other embodiments, staining can be determined or assessed in stromal or surrounding tissue that may be present in the sample. In some embodiments, it is understood that when cells and/or tissue from a tumor is examined using IHC, staining includes determining or assessing in tumor-infiltrating immune cells, including intratumoral or peritumoral immune cells. In some embodiments, the presence of a biomarker is detected by IHC in >0% of the sample, in at least 1% of the sample, in at least 5% of the sample, in at least 10% of the sample, in at least 15% of the sample, in at least 15% of the sample, in at least 20% of the sample, in at least 25% of the sample, in at least 30% of the sample, in at least 35% of the sample, in at least 40% of the sample, in at least 45% of the sample, in at least 50% of the sample, in at least 55% of the sample, in at least 60% of the sample, in at least 65% of the sample, in at least 70% of the sample, in at least 75% of the sample, in at least 80% of the sample, in at least 85% of the sample, in at least 90% of the sample, in at least 95% of the sample, or more. Samples may be scored using any method known in the art, for example, by a pathologist or automated image analysis.

In some embodiments of any of the methods, the biomarker is detected by immunohistochemistry using a diagnostic antibody (i.e., primary antibody). In some embodiments, the diagnostic antibody specifically binds human antigen. In some embodiments, the diagnostic antibody is a non-human antibody. In some embodiments, the diagnostic antibody is a rat, mouse, or rabbit antibody. In some embodiments, the diagnostic antibody is a rabbit antibody. In some embodiments, the diagnostic antibody is a monoclonal antibody. In some embodiments, the diagnostic antibody is directly labeled. In other embodiments, the diagnostic antibody is indirectly labeled (e.g., by a secondary antibody).

In other embodiments of any of the preceding methods, the expression level of a biomarker may be a nucleic acid expression level (e.g., a DNA expression level or an RNA expression level (e.g., an mRNA expression level)). Any suitable method of determining a nucleic acid expression level may be used. In some embodiments, the nucleic acid expression level is determined using RNAseq, RT-qPCR, qPCR, multiplex qPCR or RT-qPCR, microarray analysis, SAGE, MassARRAY technique, ISH, or a combination thereof.

Methods for the evaluation of mRNAs in cells are well known and include, for example, serial analysis of gene expression (SAGE), whole genome sequencing (WGS), hybridization assays using complementary DNA probes (such as in situ hybridization using labeled riboprobes specific for the one or more genes, Northern blot and related techniques) and various nucleic acid amplification assays (such as RT-PCR (e.g., qRT-PCR) using complementary primers specific for one or more of the genes, and other amplification type detection methods, such as, for example, branched DNA, SISBA, TMA and the like). In addition, such methods can include one or more steps that allow one to determine the levels of target mRNA in a biological sample (e.g., by simultaneously examining the levels a comparative control mRNA sequence **of a "housekeeping" gene such as an actin family member). Optionally, the sequence of the** amplified target cDNA can be determined. Optional methods include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlates with increased or reduced clinical benefit of treatment comprising an immunotherapy and a suppressive stromal antagonist may be arrayed on a solid support. Hybridization of a labeled probe with a particular array member indicates that the sample from which the probe was derived expresses that gene.

In some embodiments of any of the preceding embodiments, the sample is obtained from the individual prior to (e.g., minutes, hours, days, weeks (e.g., 1, 2, 3, 4, 5, 6, or 7 weeks), months, or years prior to) administration of the anti-cancer therapy. In some embodiments of any of the preceding methods, the sample from the individual is obtained about 2 to about 10 weeks (e.g., 2, 3, 4, 5, 6, 7, 8, 9, or 10 weeks) following administration of the anti-cancer therapy. In some embodiments, the sample from the individual is obtained about 4 to about 6 weeks following administration of the anti-cancer therapy.

In some embodiments of any of the preceding methods, the expression level or number of a biomarker is detected in a tissue sample, a primary or cultured cells or cell line, a cell supernatant, a cell lysate, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, or any combination thereof. In some embodiments, the sample is a tissue sample (e.g., a tumor tissue sample), a cell sample, a whole blood sample, a plasma sample, a serum sample, or a combination thereof. In some embodiments, the tumor tissue sample wherein the tumor tissue sample includes tumor cells, tumor-infiltrating immune cells, stromal cells, or a combination thereof. In some embodiments, the tumor tissue sample is a formalin-fixed and paraffin-embedded (FFPE) sample, an archival sample, a fresh sample, or a frozen sample.

For example, in some embodiments of any of the preceding methods, the expression level of a biomarker is detected in tumor-infiltrating immune cells, tumor cells, PBMCs, or combinations thereof using known techniques (e.g., IHC, immunofluorescence microscopy, or flow cytometry). Tumor-infiltrating immune cells include, but are not limited to, intratumoral immune cells, peritumoral immune cells or any combinations thereof, and other tumor stroma cells (e.g., fibroblasts). Such tumor infiltrating immune cells may be T lymphocytes (such as CD8$^+$ T lymphocytes (e.g., CD8$^+$ T effector (Teff) cells) and/or CD4$^+$ T lymphocytes (e.g., CD4$^+$ Teff cells), B lymphocytes, or other bone marrow-lineage cells including granulocytes (neutrophils, eosinophils, basophils), monocytes, macrophages, dendritic cells (e.g., interdigitating dendritic cells), histiocytes, and natural killer (NK) cells. In some embodiments, the staining for a biomarker is detected as membrane staining, cytoplasmic staining, or combinations thereof. In other embodiments, the absence of a biomarker is detected as absent or no staining in the sample, relative to a reference sample.

In particular embodiments of any of the preceding methods, the expression level of a biomarker is assessed in a sample that contains or is suspected to contain cancer cells. The sample may be, for example, a tissue biopsy or a metastatic lesion obtained from a patient suffering from, suspected to suffer from, or diagnosed with cancer (e.g., a breast cancer (e.g., a locally advanced or metastatic breast cancer (e.g., a locally advanced or metastatic TNBC))). In some embodiments, the sample is a sample of breast tissue, a biopsy of a breast tumor, a known or suspected metastatic breast cancer lesion or section, or a blood sample, e.g., a peripheral blood sample, known or suspected to comprise circulating cancer cells, e.g., breast cancer cells. The sample may comprise both cancer cells, i.e., tumor cells, and non-cancerous cells (e.g., lymphocytes, such as T cells or NK cells), and, in certain embodiments, comprises both cancerous and non-cancerous cells. Methods of obtaining biological samples including tissue resections, biopsies, and body fluids, e.g., blood samples comprising cancer/tumor cells, are well known in the art.

The patient may have an advanced, refractory, recurrent, chemotherapy-resistant, and/or platinum-resistant form of the cancer.

In certain embodiments, the presence and/or expression levels/amount of a biomarker in a first sample is increased or elevated as compared to presence/absence and/or expression levels/amount in a second sample. In certain embodiments, the presence/absence and/or expression levels/amount of a biomarker in a first sample is decreased or reduced as compared to presence and/or expression levels/amount in a second sample. In certain embodiments, the second sample is a reference sample, reference cell, reference tissue, control sample, control cell, or control tissue.

In certain embodiments, a reference sample, reference cell, reference tissue, control sample, control cell, or control tissue is a single sample or combined multiple samples from the same patient or individual that are obtained at one or more different time points than when the test sample is obtained. For example, a reference sample, reference cell, reference tissue, control sample, control cell, or control tissue is obtained at an earlier time point from the same patient or individual than when the test sample is obtained. Such reference sample, reference cell, reference tissue, control sample, control cell, or control tissue may be useful if the reference sample is obtained during initial diagnosis of cancer and the test sample is later obtained when the cancer becomes metastatic.

In certain embodiments, a reference sample, reference cell, reference tissue, control sample, control cell, or control tissue is a combined multiple samples from one or more healthy individuals who are not the patient. In certain embodiments, a reference sample, reference cell, reference tissue, control sample, control cell, or control tissue is a combined multiple samples from one or more individuals with a disease or disorder (e.g., cancer) who are not the patient or individual. In certain embodiments, a reference sample, reference cell, reference tissue, control sample, control cell, or control tissue is pooled RNA samples from normal tissues or pooled plasma or serum samples from one or more individuals who are not the patient. In certain embodiments, a reference sample, reference cell, reference tissue, control sample, control cell, or control tissue is pooled RNA samples from tumor tissues or pooled plasma or serum samples from one or more individuals with a disease or disorder (e.g., cancer) who are not the patient.

In some embodiments of any of the preceding aspects, the method further includes administering an effective amount of the anti-cancer therapy to the patient. The method may further include any of the methods described below in Section IV.

## IV. Methods of Treatment

Provided herein are methods for treating or delaying progression of locally advanced or metastatic breast cancer (e.g., locally advanced or metastatic TNBC) in an individual comprising administering to the individual an effective amount of a PD-1 axis binding antagonist (e.g., an anti-PD-L1 antibody (e.g., atezolizumab) or an anti-PD-1 antibody) and a taxane (e.g., nab-paclitaxel or paclitaxel). In some embodiments, the treatment results in a response in the individual after treatment. In some embodiments, the response is a partial response. In some embodiments, the response is a complete response. In some embodiments, the treatment results in a sustained response (e.g., a sustained partial response or complete response) in the individual after cessation of the treatment. For example, in some embodiments, the patient is PD-L1-positive. The methods described herein may find use in treating conditions where enhanced immunogenicity is desired such as increasing tumor immunogenicity for the treatment of cancer. Also provided herein are methods of enhancing immune function in an individual having a locally advanced or metastatic breast cancer comprising administering to the individual an effective amount of a PD-1 axis binding antagonist (e.g., an anti-PD-L1 antibody (e.g., atezolizumab) or an anti-PD-1 antibody) and a taxane (e.g., nab-paclitaxel or paclitaxel). Any of the PD-1 axis binding antagonists and the taxanes known in the art or described herein may be used in the methods.

For example, provided herein is a method of treating a patient suffering from a locally advanced or metastatic breast cancer (e.g., locally advanced or metastatic TNBC), the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the breast cancer (e.g., the locally advanced or metastatic TNBC), and wherein the patient has been identified as likely to respond to the anti-cancer therapy based on a detectable expression level of PD-L1 a sample (e.g., a tumor sample) obtained from the patient.

In another aspect, the invention features a method of treating a patient suffering from a locally advanced or metastatic breast cancer (e.g., locally advanced or metastatic TNBC), the method comprising: (a) determining the expression level of PD-L1 in a sample (e.g., a tumor sample) obtained from the patient, wherein the patient is previously untreated for the breast cancer (e.g., the locally advanced or metastatic TNBC), and (b) administering a therapeutically effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of PD-L1 in the sample.

In a further aspect, the invention features a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with a locally advanced or metastatic breast cancer (e.g., locally advanced or metastatic TNBC), wherein the treatment comprises administration of the human PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of PD-L1 in a sample (e.g., a tumor sample) obtained from the patient. In some embodiments, the patient is previously untreated for the breast cancer (e.g., the locally advanced or metastatic TNBC).

Any suitable sample may be used. For example, the sample may be a tumor sample.

In another example, provided herein is a method of treating a patient suffering from a locally advanced or metastatic breast cancer (e.g., locally advanced or metastatic TNBC), the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the breast cancer (e.g., the locally advanced or metastatic TNBC), and wherein the patient has been identified as likely to respond to the anti-cancer therapy based on a detectable expression level of PD-L1 in tumor-infiltrating immune cells that comprise about 1 % or more of a tumor sample obtained from the patient.

In yet another aspect, the invention features a method of treating a patient suffering from a locally advanced or metastatic breast cancer (e.g., locally advanced or metastatic TNBC), the method comprising: (a) determining the expression level of PD-L1 in tumor-infiltrating immune cells in a tumor sample obtained from the patient, wherein the patient is previously untreated for the breast cancer (e.g., the locally advanced or metastatic TNBC), and (b) administering a therapeutically effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of PD-L1 in tumor-infiltrating immune cells that comprise about 1% or more of the tumor sample.

In another aspect, the invention features a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with a locally advanced or metastatic breast cancer (e.g., locally advanced or metastatic TNBC), wherein the treatment comprises administration of the human PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of PD-L1 in tumor-infiltrating immune cells that comprise about 1 % or more of a tumor sample obtained from the patient. In some embodiments, the patient is previously untreated for the breast cancer (e.g., the locally advanced or metastatic TNBC).

In some embodiments of any of the preceding aspects, the locally advanced or metastatic breast cancer is a locally advanced or metastatic TNBC.

For example, provided herein is a method of treating a patient suffering from a locally advanced TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the locally advanced TNBC, and wherein the patient has been identified as likely to respond to the anti-cancer therapy based on a detectable expression level of PD-L1 in tumor-infiltrating immune cells that comprise about 1% or more of a tumor sample obtained from the patient.

In another example, provided herein is a method of treating a patient suffering from a metastatic TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the metastatic TNBC, and wherein the patient has been identified as likely to respond to the anti-cancer therapy based on a detectable expression level of PD-L1 in tumor-infiltrating immune cells that comprise about 1% or more of a tumor sample obtained from the patient.

In yet another example, provided herein is a method of treating a patient suffering from a locally advanced TNBC, the method comprising: (a) determining the expression level of PD-L1 in tumor-infiltrating immune cells in a tumor sample obtained from the patient, wherein the patient is previously untreated for the locally advanced TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of PD-L1 in tumor-infiltrating immune cells that comprise about 1% or more of the tumor sample.

In a further example, provided herein is a method of treating a patient suffering from a metastatic TNBC, the method comprising: (a) determining the expression level of PD-L1 in tumor-infiltrating immune cells in a tumor sample obtained from the patient, wherein the patient is previously untreated for the metastatic TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of PD-L1 in tumor-infiltrating immune cells that comprise about 1% or more of the tumor sample.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with locally advanced TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of PD-L1 in tumor-infiltrating immune cells that comprise about 1% or more of a tumor sample obtained from the patient. In some embodiments, the patient is previously untreated for the locally advanced TNBC.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with metastatic TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of PD-L1 in tumor-infiltrating immune cells that comprise about 1% or more of a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and nab-paclitaxel, wherein the patient has not been previously treated for the locally advanced TNBC, and wherein the patient has been identified as likely to respond to the anti-cancer therapy based on a detectable expression level of PD-L1 in tumor-infiltrating immune cells that comprise about 1 % or more of a tumor sample obtained from the patient.

In yet another example, provided herein is a method of treating a patient suffering from a metastatic TNBC, the method comprising administering to the patient an effective amount of an anti-cancer therapy comprising atezolizumab and nab-paclitaxel, wherein the patient has not been previously treated for the metastatic TNBC, and wherein the patient has been identified as likely to respond to the anti-cancer therapy based on a detectable expression level of PD-L1 in tumor-infiltrating immune cells that comprise about 1 % or more of a tumor sample obtained from the patient.

In a further example, provided herein is a method of treating a patient suffering from a locally advanced TNBC, the method comprising: (a) determining the expression level of PD-L1 in tumor-infiltrating immune cells in a tumor sample obtained from the patient, wherein the patient is previously untreated for the locally advanced TNBC, and (b) administering a therapeutically effective amount of the anti-cancer therapy comprising atezolizumab and nab-paclitaxel to the patient based on a detectable expression level of PD-L1 in tumor-infiltrating immune cells that comprise about 1 % or more of the tumor sample.

In yet another example, provided herein is a method of treating a patient suffering from a metastatic TNBC, the method comprising: (a) determining the expression level of PD-L1 in tumor-infiltrating immune cells in a tumor sample obtained from the patient, wherein the patient is previously untreated for the metastatic TNBC, and (b) administering a therapeutically effective amount of the anti-cancer therapy comprising atezolizumab and nab-paclitaxel to the patient based on a detectable expression level of PD-L1 in tumor-infiltrating immune cells that comprise about 1 % or more of the tumor sample.

In another example, provided herein is a pharmaceutical composition comprising atezolizumab for use in treatment of a patient diagnosed with locally advanced TNBC, wherein the treatment comprises administration of the atezolizumab in combination with nab-paclitaxel, and wherein the patent is identified as likely to respond to an anti-cancer therapy comprising atezolizumab and nab-paclitaxel based on a detectable expression level of PD-L1 in tumor-infiltrating immune cells that comprise about 1% or more of a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with nab-paclitaxel, and wherein the patent is identified as likely to respond to an anti-cancer therapy comprising atezolizumab and nab-paclitaxel based on a detectable expression level of PD-L1 in tumor-infiltrating immune cells that comprise about 1% or more of a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and paclitaxel, wherein the patient has not been previously treated for the locally advanced TNBC, and wherein the patient has been identified as likely to respond to the anti-cancer therapy based on a detectable expression level of PD-L1 in tumor-infiltrating immune cells that comprise about 1 % or more of a tumor sample obtained from the patient.

In yet another example, provided herein is a method of treating a patient suffering from a metastatic TNBC, the method comprising administering to the patient an effective amount of an anti-cancer therapy comprising atezolizumab and paclitaxel, wherein the patient has not been previously treated for the metastatic TNBC, and wherein the patient has been identified as likely to respond to the anti-cancer therapy based on a detectable expression level of PD-L1 in tumor-infiltrating immune cells that comprise about 1 % or more of a tumor sample obtained from the patient.

In a further example, provided herein is a method of treating a patient suffering from a locally advanced TNBC, the method comprising: (a) determining the expression level of PD-L1 in tumor-infiltrating immune cells in a tumor sample obtained from the patient, wherein the patient is previously untreated for the locally advanced TNBC, and (b) administering a therapeutically effective amount of the anti-cancer therapy comprising atezolizumab and paclitaxel to the patient based on a detectable expression level of PD-L1 in tumor-infiltrating immune cells that comprise about 1% or more of the tumor sample.

In yet another example, provided herein is a method of treating a patient suffering from a metastatic TNBC, the method comprising: (a) determining the expression level of PD-L1 in tumor-infiltrating immune cells in a tumor sample obtained from the patient, wherein the patient is previously untreated for the metastatic TNBC, and (b) administering a therapeutically effective amount of the anti-cancer therapy comprising atezolizumab and paclitaxel to the patient based on a detectable expression level of PD-L1 in tumor-infiltrating immune cells that comprise about 1% or more of the tumor sample.

In another example, provided herein is a pharmaceutical composition comprising atezolizumab for use in treatment of a patient diagnosed with locally advanced TNBC, wherein the treatment comprises administration of the atezolizumab in combination with paclitaxel, and wherein the patent is identified as likely to respond to an anti-cancer therapy comprising atezolizumab and paclitaxel based on a detectable expression level of PD-L1 in tumor-infiltrating immune cells that comprise about 1% or more of a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with paclitaxel, and wherein the patent is identified as likely to respond to an anti-cancer therapy comprising atezolizumab and paclitaxel based on a detectable expression level of PD-L1 in tumor-infiltrating immune cells that comprise about 1% or more of a tumor sample obtained from the patient. In some embodiments, the patient is previously untreated for the metastatic TNBC.

In some embodiments of any of the preceding methods, the tumor sample obtained from the patient has been determined to have a detectable expression level of PD-L1 in tumor-infiltrating immune cells that comprise about 1% or more (e.g., about 1% or more, 2% or more, 3% or more, 5% or more, 6% or more, 7% or more, 8% or more, 9% or more, 10% or more, 11% or more, 12% or more, 13% or more, 14% or more, 15% or more, 16% or more, 17% or more,18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, about 50% or more, about 60% or more, about 70% or more, about 80% or more, about 90% or more, about 95% or more, about 96% or more, about 97% or more, about 98% or more, about 99% or more, or 100%) of the tumor sample. For example, in some embodiments, the tumor sample obtained from the patient has been determined to have a detectable expression level of PD-L1 in tumor-infiltrating immune cells that comprise from about 1% to less than about 5% (e.g., from 1% to 4.9%, from 1% to 4.5%, from 1% to 4%, from 1% to 3.5%, from 1% to 3%, from 1% to 2.5%, or from 1% to 2%) of the tumor sample.

In some embodiments of any of the preceding methods, the tumor sample obtained from the patient has been determined to have a detectable expression level of PD-L1 in about 1% or more (e.g., about 1% or more, 2% or more, 3% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, about 50% or more, about 60% or more, about 70% or more, about 80% or more, about 90% or more, about 95% or more, about 96% or more, about 97% or more, about 98% or more, about 99% or more, or 100%) of the tumor-infiltrating immune cells in the tumor sample. For example, in some embodiments, the tumor sample obtained from the patient has been determined to have a detectable expression level of PD-L1 in from about 1% to less than about 5% (e.g., from 1% to 4.9%, from 1% to 4.5%, from 1% to 4%, from 1% to 3.5%, from 1% to 3%, from 1% to 2.5%, or from 1% to 2%) of the tumor-infiltrating immune cells in the tumor sample.

In other embodiments, the tumor sample obtained from the patient has been determined to have a detectable expression level of PD-L1 in tumor-infiltrating immune cells that comprise about 5% or more of the tumor sample. For example, in some embodiments, the tumor sample obtained from the patient has been determined to have a detectable expression level of PD-L1 in tumor-infiltrating immune cells that comprise from about 5% to less than about 10% (e.g., from 5% to 9.5%, from 5% to 9%, from 5% to 8.5%, from 5% to 8%, from 5% to 7.5%, from 5% to 7%, from 5% to 6.5%, from 5% to 6%, from 5% to 5.5%, from 6% to 9.5%, from 6% to 9%, from 6% to 8.5%, from 6% to 8%, from 6% to 7.5%, from 6% to 7%, from 6% to 6.5%, from 7% to 9.5%, from 7% to 9%, from 7% to 7.5%, from 8% to 9.5%, from 8% to 9%, or from 8% to 8.5%) of the tumor sample.

In yet other embodiments, the tumor sample obtained from the patient has been determined to have a detectable expression level of PD-L1 in about 5% or more of the tumor-infiltrating immune cells in the tumor sample. For example, in some embodiments, the tumor sample obtained from the patient has been determined to have a detectable expression level of PD-L1 in from about 5% to less than about 10% (e.g., from 5% to 9.5%, from 5% to 9%, from 5% to 8.5%, from 5% to 8%, from 5% to 7.5%, from 5% to 7%, from 5% to 6.5%, from 5% to 6%, from 5% to 5.5%, from 6% to 9.5%, from 6% to 9%, from 6% to 8.5%, from 6% to 8%, from 6% to 7.5%, from 6% to 7%, from 6% to 6.5%, from 7% to 9.5%, from 7% to 9%, from 7% to 7.5%, from 8% to 9.5%, from 8% to 9%, or from 8% to 8.5%) of the tumor-infiltrating immune cells in the tumor sample.

In still further embodiments, the tumor sample obtained from the patient has been determined to have a detectable expression level of PD-L1 in tumor-infiltrating immune cells that comprise about 10% or more (e.g., 10% or more, 11% or more, 12% or more, 13% or more, 14% or more, 15% or more, 16% or more, 17% or more,18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 60% or more, 70% or more, 80% or more, 90% or more, 95% or more, 96% or more, 97% or more, 98% or more, 99% or more, or 100%) of the tumor sample.

In still further embodiments, the tumor sample obtained from the patient has been determined to have a detectable expression level of PD-L1 in about 10% or more (e.g., 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 60% or more, 70% or more, 80% or more, 90% or more, 95% or more, 96% or more, 97% or more, 98% or more, 99% or more, or 100%) of the tumor-infiltrating immune cells in the tumor sample.

In yet other embodiments, the tumor sample obtained from the patient has been determined to have a detectable expression level of PD-L1 in about 50% or more (e.g., about 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more) of the tumor cells in the tumor sample and/or a detectable expression level of PD-L1 in tumor-infiltrating immune cells that comprise about 10% or more (e.g., 10% or more, 11% or more, 12% or more, 13% or more, 14% or more, 15% or more, 16% or more, 17% or more,18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 60% or more, 70% or more, 80% or more, 90% or more, 95% or more, 96% or more, 97% or more, 98% or more, 99% or more, or 100%) of the tumor sample.

It is to be understood that in any of the preceding methods, the percentage of the tumor sample comprised by tumor-infiltrating immune cells may be in terms of the percentage of tumor area covered by tumor-infiltrating immune cells in a section of the tumor sample obtained from the patient, for example, as assessed by IHC using an anti-PD-L1 antibody (e.g., the SP142 antibody). See, for example, Example 3 (e.g., Table 5).

In another example, provided herein is a method of treating a patient suffering from a locally advanced or metastatic breast cancer (e.g., locally advanced or metastatic TNBC), the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the breast cancer (e.g., the locally advanced or metastatic TNBC), and wherein the patient has been identified as likely to respond to the anti-cancer therapy based on a detectable expression level of PD-L1 in about 1 % or more of the tumor cells in a tumor sample obtained from the patient.

In another aspect, the invention features a method of treating a patient suffering from a locally advanced or metastatic breast cancer (e.g., locally advanced or metastatic TNBC), the method comprising: (a) determining the expression level of PD-L1 in tumor cells in a tumor sample obtained from the patient, wherein the patient is previously untreated for the breast cancer (e.g., the locally advanced or metastatic TNBC), and (b) administering a therapeutically effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of PD-L1 in about 1% or more of the tumor cells in the tumor sample.

In another aspect, the invention features a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with a locally advanced or metastatic breast cancer (e.g., locally advanced or metastatic TNBC), wherein the treatment comprises administration of the human PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of PD-L1 in about 1 % or more of the tumor cells in a tumor sample obtained from the patient. In some embodiments, the patient is previously untreated for the breast cancer (e.g., the locally advanced or metastatic TNBC).

In some embodiments of any of the preceding aspects, the locally advanced or metastatic breast cancer is a locally advanced or metastatic TNBC.

For example, provided herein is a method of treating a patient suffering from a locally advanced TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the locally advanced TNBC, and wherein the patient has been identified as likely to respond to the anti-cancer therapy based on a detectable expression level of PD-L1 in about 1% or more of the tumor cells in a tumor sample obtained from the patient.

In another example, provided herein is a method of treating a patient suffering from a metastatic TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the metastatic TNBC, and wherein the patient has been identified as likely to respond to the anti-cancer therapy based on a detectable expression level of PD-L1 in about 1 % or more of the tumor cells in a tumor sample obtained from the patient.

In yet another example, provided herein is a method of treating a patient suffering from a locally advanced TNBC, the method comprising: (a) determining the expression level of PD-L1 in tumor cells in a tumor sample obtained from the patient, wherein the patient is previously untreated for the locally advanced TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of PD-L1 in about 1% or more of the tumor cells in the tumor sample.

In a further example, provided herein is a method of treating a patient suffering from a metastatic TNBC, the method comprising: (a) determining the expression level of PD-L1 in tumor cells in a tumor sample obtained from the patient, wherein the patient is previously untreated for the metastatic TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of PD-L1 in about 1 % or more of the tumor cells in the tumor sample.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with locally advanced TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of PD-L1 in about 1 % or more of the tumor cells in a tumor sample obtained from the patient. In some embodiments, the patient is previously untreated for the locally advanced TNBC.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with metastatic TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of PD-L1 in about 1% or more of the tumor cells in a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and nab-paclitaxel, wherein the patient has not been previously treated for the locally advanced TNBC, and wherein the patient has been identified as likely to respond to the anti-cancer therapy based on a detectable expression level of PD-L1 in about 1% or more of the tumor cells in a tumor sample obtained from the patient.

In yet another example, provided herein is a method of treating a patient suffering from a metastatic TNBC, the method comprising administering to the patient an effective amount of an anti-cancer therapy comprising atezolizumab and nab-paclitaxel, wherein the patient has not been previously treated for the metastatic TNBC, and wherein the patient has been identified as likely to respond to the anti-cancer therapy based on a detectable expression level of PD-L1 in about 1% or more of the tumor cells in a tumor sample obtained from the patient.

In a further example, provided herein is a method of treating a patient suffering from a locally advanced TNBC, the method comprising: (a) determining the expression level of PD-L1 in tumor cells in a tumor sample obtained from the patient, wherein the patient is previously untreated for the locally advanced TNBC, and (b) administering a therapeutically effective amount of the anti-cancer therapy comprising atezolizumab and nab-paclitaxel to the patient based on a detectable expression level of PD-L1 in about 1% or more of the tumor cells in the tumor sample.

In yet another example, provided herein is a method of treating a patient suffering from a metastatic TNBC, the method comprising: (a) determining the expression level of PD-L1 in tumor cells in a tumor sample obtained from the patient, wherein the patient is previously untreated for the metastatic TNBC, and (b) administering a therapeutically effective amount of the anti-cancer therapy comprising atezolizumab and nab-paclitaxel to the patient based on a detectable expression level of PD-L1 in about 1 % or more of the tumor cells in the tumor sample.

In another example, provided herein is a pharmaceutical composition comprising atezolizumab for use in treatment of a patient diagnosed with locally advanced TNBC, wherein the treatment comprises administration of the atezolizumab in combination with nab-paclitaxel, and wherein the patent is identified as likely to respond to an anti-cancer therapy comprising atezolizumab and nab-paclitaxel based on a detectable expression level of PD-L1 in about 1% or more of the tumor cells in a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with nab-paclitaxel, and wherein the patent is identified as likely to respond to an anti-cancer therapy comprising atezolizumab and nab-paclitaxel based on a detectable expression level of PD-L1 in about 1% or more of the tumor cells in a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and paclitaxel, wherein the patient has not been previously treated for the locally advanced TNBC, and wherein the patient has been identified as likely to respond to the anti-cancer therapy based on a detectable expression level of PD-L1 in about 1% or more of the tumor cells in a tumor sample obtained from the patient.

In yet another example, provided herein is a method of treating a patient suffering from a metastatic TNBC, the method comprising administering to the patient an effective amount of an anti-cancer therapy comprising atezolizumab and paclitaxel, wherein the patient has not been previously treated for the metastatic TNBC, and wherein the patient has been identified as likely to respond to the anti-cancer therapy based on a detectable expression level of PD-L1 in about 1 % or more of the tumor cells in a tumor sample obtained from the patient.

In a further example, provided herein is a method of treating a patient suffering from a locally advanced TNBC, the method comprising: (a) determining the expression level of PD-L1 in tumor cells in a tumor sample obtained from the patient, wherein the patient is previously untreated for the locally advanced TNBC, and (b) administering a therapeutically effective amount of the anti-cancer therapy comprising atezolizumab and paclitaxel to the patient based on a detectable expression level of PD-L1 in about 1% or more of the tumor cells in the tumor sample.

In yet another example, provided herein is a method of treating a patient suffering from a metastatic TNBC, the method comprising: (a) determining the expression level of PD-L1 in tumor cells in a tumor sample obtained from the patient, wherein the patient is previously untreated for the metastatic TNBC, and (b) administering a therapeutically effective amount of the anti-cancer therapy comprising atezolizumab and paclitaxel to the patient based on a detectable expression level of PD-L1 in about 1% or more of the tumor cells in the tumor sample.

In another example, provided herein is a pharmaceutical composition comprising atezolizumab for use in treatment of a patient diagnosed with locally advanced TNBC, wherein the treatment comprises administration of the atezolizumab in combination with paclitaxel, and wherein the patent is identified as likely to respond to an anti-cancer therapy comprising atezolizumab and paclitaxel based on a detectable expression level of PD-L1 in about 1% or more of the tumor cells in a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with paclitaxel, and wherein the patent is identified as likely to respond to an anti-cancer therapy comprising atezolizumab and paclitaxel based on a detectable expression level of PD-L1 in about 1% or more of the tumor cells in a tumor sample obtained from the patient. In some embodiments, the patient is previously untreated for the metastatic TNBC.

In some embodiments of any of the preceding methods, a tumor sample obtained from the patient has been determined to have a detectable expression level of PD-L1 in about 1% or more (e.g., about 1% or more, 2% or more, 3% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more) of the tumor cells in the tumor sample. For example, in some embodiments, the tumor sample obtained from the patient has been determined to have a detectable expression level of PD-L1 in from about 1% to less than about 5% (e.g., from 1% to 4.9%, from 1% to 4.5%, from 1% to 4%, from 1% to 3.5%, from 1% to 3%, from 1% to 2.5%, or from 1% to 2%) of the tumor cells in the tumor sample. In other embodiments, a tumor sample obtained from the patient has been determined to have a detectable expression level of PD-L1 in less than about 1% of the tumor cells in the tumor sample.

In other embodiments, the tumor sample obtained from the patient has been determined to have a detectable expression level of PD-L1 in about 5% or more of the tumor cells in the tumor sample. For example, in some embodiments, the tumor sample obtained from the patient has been determined to have a detectable expression level of PD-L1 in from about 5% to less than 50% (e.g., from 5% to 49.5%, from 5% to 45%, from 5% to 40%, from 5% to 35%, from 5% to 30%, from 5% to 25%, from 5% to 20%, from 5% to 15%, from 5% to 10%, from 5% to 9%, from 5% to 8%, from 5% to 7%, from 5% to 6%, from 10% to 49.5%, from 10% to 40%, from 10% to 35%, from 10% to 30%, from 10% to 25%, from 10% to 20%, from 10% to 15%, from 15% to 49.5%, from 15% to 45%, from 15% to 40%, from 15% to 35%, from 15% to 30%, from 15% to 30%, from 15% to 25%, from 15% to 20%, from 20% to 49.5%, from 20% to 45%, from 20% to 40%, from 20% to 35%, from 20% to 30%, from 20% to 25%, from 25% to 49.5%, from 25% to 45%, from 25% to 40%, from 25% to 35%, from 25% to 30%, from 30% to 49.5%, from 30% to 45%, from 30% to 40%, from 30% to 35%, from 35% to 49.5%, from 35% to 45%, from 35% to 40%, from 40% to 49.5%, from 40% to 45%, or from 45% to 49.5%) of the tumor cells in the tumor sample.

In yet other embodiments, the tumor sample obtained from the patient has been determined to have a detectable expression level of PD-L1 in about 50% or more (e.g., about 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more) of the tumor cells in the tumor sample. In some embodiments, the tumor sample obtained from the patient has been determined to have a detectable expression level of PD-L1 in from about 50% to about 99% (e.g., from 50% to 99%, from 50% to 95%, from 50% to 90%, from 50% to 85%, from 50% to 80%, from 50% to 75%, from 50% to 70%, from 50% to 65%, from 50% to 60%, from 50% to 55%, from 55% to 99%, from 55% to 95%, from 55% to 90%, from 55% to 85%, from 55% to 80%, from 55% to 75%, from 55% to 70%, from 55% to 65%, from 55% to 60%, from 60% to 99%, from 60% to 95%, from 60% to 90%, from 60% to 85%, from 60% to 80%, from 60% to 75%, from 60% to 70%, from 60% to 65%, from 65% to 99%, from 65% to 95%, from 65% to 90%, from 65% to 85%, from 65% to 80%, from 65% to 75%, from 65% to 70%, from 70% to 99%, from 70% to 95%, from 70% to 90%, from 70% to 85%, from 70% to 80%, from 70% to 75%, from 75% to 99%, from 75% to 95%, from 75% to 90%, from 75% to 85%, from 75% to 80%, from 80% to 99%, from 80% to 95%, from 80% to 90%, from 80% to 85%, from 85% to 99%, from 85% to 95%, from 85% to 90%, from 90% to 99%, or from 90% to 95%) of the tumor cells in the tumor sample.

In another example, provided herein is a method of treating a patient suffering from a locally advanced or metastatic breast cancer (e.g., locally advanced or metastatic TNBC), the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the breast cancer (e.g., the locally advanced or metastatic TNBC), and wherein the patient has been identified as likely to respond to the anti-cancer therapy based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient.

In another aspect, the invention features a method of treating a patient suffering from a locally advanced or metastatic breast cancer (e.g., locally advanced or metastatic TNBC), the method comprising: (a) determining the expression level of CD8 in tumor-infiltrating immune cells in a tumor sample obtained from the patient, wherein the patient is previously untreated for the breast cancer (e.g., the locally advanced or metastatic TNBC), and (b) administering a therapeutically effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In another aspect, the invention features a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with a locally advanced or metastatic breast cancer (e.g., locally advanced or metastatic TNBC), wherein the treatment comprises administration of the human PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient. In some embodiments, the patient is previously untreated for the breast cancer (e.g., the locally advanced or metastatic TNBC).

In some embodiments of any of the preceding aspects, the locally advanced or metastatic breast cancer is a locally advanced or metastatic TNBC.

For example, provided herein is a method of treating a patient suffering from a locally advanced TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the locally advanced TNBC, and wherein the patient has been identified as likely to respond to the anti-cancer therapy based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient.

In another example, provided herein is a method of treating a patient suffering from a metastatic TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the metastatic TNBC, and wherein the patient has been identified as likely to respond to the anti-cancer therapy based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient.

In yet another example, provided herein is a method of treating a patient suffering from a locally advanced TNBC, the method comprising: (a) determining the expression level of CD8 in tumor-infiltrating immune cells in a tumor sample obtained from the patient, wherein the patient is previously untreated for the locally advanced TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In a further example, provided herein is a method of treating a patient suffering from a metastatic TNBC, the method comprising: (a) determining the expression level of CD8 in tumor-infiltrating immune cells in a tumor sample obtained from the patient, wherein the patient is previously untreated for the metastatic TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with locally advanced TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient. In some embodiments, the patient is previously untreated for the locally advanced TNBC.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with metastatic TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and nab-paclitaxel, wherein the patient has not been previously treated for the locally advanced TNBC, and wherein the patient has been identified as likely to respond to the anti-cancer therapy based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient.

In yet another example, provided herein is a method of treating a patient suffering from a metastatic TNBC, the method comprising administering to the patient an effective amount of an anti-cancer therapy comprising atezolizumab and nab-paclitaxel, wherein the patient has not been previously treated for the metastatic TNBC, and wherein the patient has been identified as likely to respond to the anti-cancer therapy based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient.

In a further example, provided herein is a method of treating a patient suffering from a locally advanced TNBC, the method comprising: (a) determining the expression level of CD8 in tumor-infiltrating immune cells in a tumor sample obtained from the patient, wherein the patient is previously untreated for the locally advanced TNBC, and (b) administering a therapeutically effective amount of the anti-cancer therapy comprising atezolizumab and nab-paclitaxel to the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In yet another example, provided herein is a method of treating a patient suffering from a metastatic TNBC, the method comprising: (a) determining the expression level of CD8 in tumor-infiltrating immune cells in a tumor sample obtained from the patient, wherein the patient is previously untreated for the metastatic TNBC, and (b) administering a therapeutically effective amount of the anti-cancer therapy comprising atezolizumab and nab-paclitaxel to the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In another example, provided herein is a pharmaceutical composition comprising atezolizumab for use in treatment of a patient diagnosed with locally advanced TNBC, wherein the treatment comprises administration of the atezolizumab in combination with nab-paclitaxel, and wherein the patent is identified as likely to respond to an anti-cancer therapy comprising atezolizumab and nab-paclitaxel based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with nab-paclitaxel, and wherein the patent is identified as likely to respond to an anti-cancer therapy comprising atezolizumab and nab-paclitaxel based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and paclitaxel, wherein the patient has not been previously treated for the locally advanced TNBC, and wherein the patient has been identified as likely to respond to the anti-cancer therapy based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient.

In yet another example, provided herein is a method of treating a patient suffering from a metastatic TNBC, the method comprising administering to the patient an effective amount of an anti-cancer therapy comprising atezolizumab and paclitaxel, wherein the patient has not been previously treated for the metastatic TNBC, and wherein the patient has been identified as likely to respond to the anti-cancer therapy based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient.

In a further example, provided herein is a method of treating a patient suffering from a locally advanced TNBC, the method comprising: (a) determining the expression level of CD8 in tumor-infiltrating immune cells in a tumor sample obtained from the patient, wherein the patient is previously untreated for the locally advanced TNBC, and (b) administering a therapeutically effective amount of the anti-cancer therapy comprising atezolizumab and paclitaxel to the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In yet another example, provided herein is a method of treating a patient suffering from a metastatic TNBC, the method comprising: (a) determining the expression level of CD8 in tumor-infiltrating immune cells in a tumor sample obtained from the patient, wherein the patient is previously untreated for the metastatic TNBC, and (b) administering a therapeutically effective amount of the anti-cancer therapy comprising atezolizumab and paclitaxel to the patient based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of the tumor sample.

In another example, provided herein is a pharmaceutical composition comprising atezolizumab for use in treatment of a patient diagnosed with locally advanced TNBC, wherein the treatment comprises administration of the atezolizumab in combination with paclitaxel, and wherein the patent is identified as likely to respond to an anti-cancer therapy comprising atezolizumab and paclitaxel based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with paclitaxel, and wherein the patent is identified as likely to respond to an anti-cancer therapy comprising atezolizumab and paclitaxel based on a detectable expression level of CD8 in tumor-infiltrating immune cells that comprise about 0.5% or more (e.g., about 0.5% or more, about 0.75% or more, about 1% or more, about 1.25% or more, about 1.35% or more, about 1.5% or more, about 2% or more, about 2.25% or more, about 2.5% or more, about 2.75% or more, about 3% or more, about 3.25% or more, about 3.5% or more, about 3.75% or more, about 4% or more, about 4.25% or more, about 4.5% or more, about 4.75% or more, or about 5% or more) of a tumor sample obtained from the patient. In some embodiments, the patient is previously untreated for the metastatic TNBC.

For example, in some embodiments, the tumor sample obtained from the patient has been determined to have a detectable expression level of CD8 in tumor-infiltrating immune cells that comprise about 0.5% or more, about 0.55% or more, about 0.6% or more, about 0.65% or more, about 0.7% or more, about 0.75% or more, about 0.8% or more, about 0.85% or more, about 0.9% or more, about 0.95% or more, about 1% or more, about 1.05% or more, about 1.1% or more, about 1.2% or more, about 1.25% or more, about 1.3% or more, about 1.35% or more, about 1.4% or more, about 1.45% or more, about 1.5% or more, about 1.55% or more, about 1.6% or more, about 1.65% or more, about 1.7% or more, about 1.75% or more, about 1.8% or more, about 1.85% or more, about 1.9% or more, about 1.95% or more, about 2% or more, about 2.1% or more, about 2.2% or more, about 2.3% or more, about 2.4% or more, about 2.5% or more, about 2.6% or more, about 2.7% or more, about 2.8% or more, about 2.9% or more, about 3% or more, about 3.1% or more, about 3.2% or more, about 3.3% or more, about 3.4% or more, about 3.5% or more, about 3.6% or more, about 3.7% or more, about 3.8% or more, about 3.9% or more, about 4% or more, about 4.1% or more, about 4.2% or more, about 4.3% or more, about 4.4% or more, about 4.5% or more, about 4.6% or more, about 4.7% or more, about 4.8% or more, about 4.9% or more, about 5% or more, about 5.5% or more, about 6% or more, about 6.5% or more, about 7% or more, about 7.5% or more, about 8% or more, about 8.5% or more, about 9% or more, about 9.5% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 21%, about 22% or more, about 23% or more, about 24% or more, about 25% or more, about 26% or more, about 27% or more, about 28% or more, about 29% or more, about 30% or more, about 31% or more, about 32% or more, about 33% or more, about 34% or more, about 35% or more, about 36% or more, about 37% or more, about 38% or more, about 39% or more, about 40% or more, about 41% or more, about 42% or more, about 43% or more, about 44% or more, about 45% or more, about 46% or more, about 47% or more, about 48% or more, about 49% or more, about 50% or more, about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more) of the tumor sample.

For example, in some embodiments, the tumor sample obtained from the patient has been determined to have a detectable expression level of CD8 in tumor-infiltrating immune cells that comprise about 0.5% to 50%, from 0.5% to 45%, from 0.5% to 40%, from 0.5% to 35%, from 0.5% to 30%, from 0.5% to 25%, from 0.5% to 20%, from 0.5% to 15%, from 0.5% to 10%, from 0.5% to 9%, from 0.5% to 8%, from 0.5% to 7%, from 0.5% to 6%, from 0.5% to 5%, from 0.5% to 4%, from 0.5% to 3%, from 0.5% to 2%, from 0.5% to 1%, from 1% to 50%, from 1% to 40%, from 1% to 35%, from 1% to 30%, from 1% to 25%, from 1% to 20%, from 1% to 15%, from 1% to 10%, from 1% to 9%, from 1% to 8%, from 1% to 7%, from 1% to 6%, from 1% to 5%, from 1% to 4%, from 1% to 3%, from 1% to 2%, from 1.35% to 50%, from 1.35% to 45%, from 1.35% to 40%, from 1.35% to 35%, from 1.35% to 30%, from 1.35% to 30%, from 1.35% to 25%, from 1.35% to 20%, from 1.35% to 15%, from 1.35% to 10%, from 1.35% to 9%, from 1.35% to 8%, from 1.35% to 7%, from 1.35% to 6%, from 1.35% to 5%, from 1.35% to 4%, from 1.35% to 3%, from 1.35% to 2%, from 1.5% to 50%, from 1.5% to 45%, from 1.5% to 40%, from 1.5% to 35%, from 1.5% to 30%, from 1.5% to 30%, from 1.5% to 25%, from 1.5% to 20%, from 1.5% to 15%, from 1.5% to 10%, from 1.5% to 9%, from 1.5% to 8%, from 1.5% to 7%, from 1.5% to 6%, from 1.5% to 5%, from 1.5% to 4%, from 1.5% to 3%, from 1.5% to 2%, from 2% to 50%, from 2% to 45%, from 2% to 40%, from 2% to 35%, from 2% to 30%, from 2% to 25%, from 2% to 20%, from 2% to 15%, from 2% to 10%, from 2% to 9%, from 2% to 8%, from 2% to 7%, from 2% to 6%, from 2% to 5%, from 2% to 4%, from 2% to 3%, from 3% to 50%, from 3% to 45%, from 3% to 40%, from 3% to 35%, from 3% to 30%, from 3% to 25%, from 3% to 20%, from 3% to 15%, from 3% to 10%, from 3% to 9%, from 3% to 8%, from 3% to 7%, from 3% to 6%, from 3% to 5%, from 3% to 4%, from 4% to 50%, from 4% to 45%, from 4% to 40%, from 4% to 35%, from 4% to 30%, from 4% to 25%, from 4% to 20%, from 4% to 15%, from 4% to 10%, from 4% to 9%, from 4% to 8%, from 4% to 7%, from 4% to 6%, from 4% to 5%, from 5% to 50%, from 5% to 45%, from 5% to 40%, from 5% to 35%, from 5% to 30%, from 5% to 25%, from 5% to 20%, from 5% to 15%, from 5% to 10%, from 5% to 9%, from 5% to 8%, from 5% to 7%, from 5% to 6%, from 6% to 50%, from 6% to 45%, from 6% to 40%, from 6% to 35%, from 6% to 30%, from 6% to 25%, from 6% to 20%, from 6% to 15%, from 6% to 10%, from 6% to 9%, from 6% to 8%, from 6% to 7%, from 7% to 50%, from 7% to 45%, from 7% to 40%, from 7% to 35%, from 7% to 30%, from 7% to 25%, from 7% to 20%, from 7% to 15%, from 7% to 10%, from 7% to 9%, from 7% to 8%, from 8% to 50%, from 8% to 45%, from 8% to 40%, from 8% to 35%, from 8% to 30%, from 8% to 25%, from 8% to 20%, from 8% to 15%, from 8% to 10%, from 8% to 9%, from 9% to 50%, from 9% to 45%, from 9% to 40%, from 9% to 35%, from 9% to 30%, from 9% to 25%, from 9% to 20%, from 9% to 15%, from 9% to 10%, from 10% to 50%, from 10% to 45%, from 10% to 40%, from 10% to 35%, from 10% to 30%, from 10% to 25%, from 10% to 20%, or from 10% to 15% of the tumor sample.

It is to be understood that in any of the preceding methods, the percentage of the tumor sample comprised by tumor-infiltrating immune cells may be in terms of the percentage of tumor area covered by tumor-infiltrating immune cells that express CD8 (e.g., T cells) in a section of the tumor sample. In other embodiments, the percentage of tumor-infiltrating immune cells that express CD8 (e.g., T cells) relative to the total number of tumor-infiltrating immune cells can be used as a biomarker.

In another example, provided herein is a method of treating a patient suffering from a locally advanced or metastatic breast cancer (e.g., locally advanced or metastatic TNBC), the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the breast cancer (e.g., the locally advanced or metastatic TNBC), and wherein the patient has been identified as likely to respond to the anti-cancer therapy based on a percentage of sTILs of about 5% or more in a tumor sample obtained from the patient.

In another aspect, the invention features a method of treating a patient suffering from a locally advanced or metastatic breast cancer (e.g., locally advanced or metastatic TNBC), the method comprising: (a) determining the percentage of sTILs in a tumor sample obtained from the patient, wherein the patient is previously untreated for the breast cancer (e.g., the locally advanced or metastatic TNBC), and (b) administering a therapeutically effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a percentage of sTILs of about 5% or more of the tumor sample.

In another aspect, the invention features a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with a locally advanced or metastatic breast cancer (e.g., locally advanced or metastatic TNBC), wherein the treatment comprises administration of the human PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a percentage of sTILs of about 5% or more of a tumor sample obtained from the patient. In some embodiments, the patient is previously untreated for the breast cancer (e.g., the locally advanced or metastatic TNBC).

In some embodiments of any of the preceding aspects, the locally advanced or metastatic breast cancer is a locally advanced or metastatic TNBC.

For example, provided herein is a method of treating a patient suffering from a locally advanced TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the locally advanced TNBC, and wherein the patient has been identified as likely to respond to the anti-cancer therapy based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient.

In another example, provided herein is a method of treating a patient suffering from a metastatic TNBC, the method comprising administering to the patient an effective amount of an anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel), wherein the patient has not been previously treated for the metastatic TNBC, and wherein the patient has been identified as likely to respond to the anti-cancer therapy based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient.

In yet another example, provided herein is a method of treating a patient suffering from a locally advanced TNBC, the method comprising: (a) determining the percentage of sTILs in a tumor sample obtained from the patient, wherein the patient is previously untreated for the locally advanced TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In a further example, provided herein is a method of treating a patient suffering from a metastatic TNBC, the method comprising: (a) determining the percentage of sTILs in a tumor sample obtained from the patient, wherein the patient is previously untreated for the metastatic TNBC, and (b) administering a therapeutically effective amount of the anti-cancer therapy comprising (i) a PD-1 axis binding antagonist (e.g., a human PD-1 axis binding antagonist selected from an anti-PD-L1 antibody (e.g., atezolizumab) and an anti-PD-1 antibody) and (ii) a taxane (e.g., nab-paclitaxel or paclitaxel) to the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with locally advanced TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient. In some embodiments, the patient is previously untreated for the locally advanced TNBC.

In another example, provided herein is a pharmaceutical composition comprising a PD-1 axis binding antagonist (e.g., a human PD-1 axis binding antagonist selected from an anti-PD-L1 antibody (e.g., atezolizumab) and an anti-PD-1 antibody) for use in treatment of a patient diagnosed with metastatic TNBC, wherein the treatment comprises administration of the PD-1 axis binding antagonist in combination with a taxane (e.g., nab-paclitaxel or paclitaxel), and wherein the patent is identified as likely to respond to an anti-cancer therapy comprising the human PD-1 axis binding antagonist and the taxane based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and nab-paclitaxel, wherein the patient has not been previously treated for the locally advanced TNBC, and wherein the patient has been identified as likely to respond to the anti-cancer therapy based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient.

In yet another example, provided herein is a method of treating a patient suffering from a metastatic TNBC, the method comprising administering to the patient an effective amount of an anti-cancer therapy comprising atezolizumab and nab-paclitaxel, wherein the patient has not been previously treated for the metastatic TNBC, and wherein the patient has been identified as likely to respond to the anti-cancer therapy based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient.

In a further example, provided herein is a method of treating a patient suffering from a locally advanced TNBC, the method comprising: (a) determining the percentage of sTILs in a tumor sample obtained from the patient, wherein the patient is previously untreated for the locally advanced TNBC, and (b) administering a therapeutically effective amount of the anti-cancer therapy comprising atezolizumab and nab-paclitaxel to the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In yet another example, provided herein is a method of treating a patient suffering from a metastatic TNBC, the method comprising: (a) determining the percentage of sTILs in a tumor sample obtained from the patient, wherein the patient is previously untreated for the metastatic TNBC, and (b) administering a therapeutically effective amount of the anti-cancer therapy comprising atezolizumab and nab-paclitaxel to the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In another example, provided herein is a pharmaceutical composition comprising atezolizumab for use in treatment of a patient diagnosed with locally advanced TNBC, wherein the treatment comprises administration of the atezolizumab in combination with nab-paclitaxel, and wherein the patent is identified as likely to respond to an anti-cancer therapy comprising atezolizumab and nab-paclitaxel based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with nab-paclitaxel, and wherein the patent is identified as likely to respond to an anti-cancer therapy comprising atezolizumab and nab-paclitaxel based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient. In some embodiments, the patient is previously untreated for the metastatic TNBC.

In yet another example, provided herein is a method of treating a patient suffering from a locally advanced TNBC, the method comprising administering to the patient an effective amount of an anti-cancer therapy comprising atezolizumab and paclitaxel, wherein the patient has not been previously treated for the locally advanced TNBC, and wherein the patient has been identified as likely to respond to the anti-cancer therapy based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient.

In yet another example, provided herein is a method of treating a patient suffering from a metastatic TNBC, the method comprising administering to the patient an effective amount of an anti-cancer therapy comprising atezolizumab and paclitaxel, wherein the patient has not been previously treated for the metastatic TNBC, and wherein the patient has been identified as likely to respond to the anti-cancer therapy based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient.

In a further example, provided herein is a method of treating a patient suffering from a locally advanced TNBC, the method comprising: (a) determining the percentage of sTILs in a tumor sample obtained from the patient, wherein the patient is previously untreated for the locally advanced TNBC, and (b) administering a therapeutically effective amount of the anti-cancer therapy comprising atezolizumab and paclitaxel to the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In yet another example, provided herein is a method of treating a patient suffering from a metastatic TNBC, the method comprising: (a) determining the percentage of sTILs in a tumor sample obtained from the patient, wherein the patient is previously untreated for the metastatic TNBC, and (b) administering a therapeutically effective amount of the anti-cancer therapy comprising atezolizumab and paclitaxel to the patient based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of the tumor sample.

In another example, provided herein is a pharmaceutical composition comprising atezolizumab for use in treatment of a patient diagnosed with locally advanced TNBC, wherein the treatment comprises administration of the atezolizumab in combination with paclitaxel, and wherein the patent is identified as likely to respond to an anti-cancer therapy comprising atezolizumab and paclitaxel based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient. In some embodiments, the patient is previously untreated for the locally advanced TNBC.

In another example, provided herein is a pharmaceutical composition comprising atezolizumab for use in treatment of a patient diagnosed with metastatic TNBC, wherein the treatment comprises administration of the atezolizumab in combination with paclitaxel, and wherein the patent is identified as likely to respond to an anti-cancer therapy comprising atezolizumab and paclitaxel based on a percentage of sTILs of about 5% or more (e.g., about 5% or more, 6% or more, 7% or more, 8% or more, 9% or more, 10% or more, 15% or more, 20% or more, 25% or more, 50% or more, 60% or more 70% or more, 80% or more, 90% or more, 95% or more, 99% or more, or 100%) of a tumor sample obtained from the patient. In some embodiments, the patient is previously untreated for the metastatic TNBC.

For example, in some embodiments, the tumor sample obtained from the patient has been determined to have a percentage of sTILs of about 5% or more, about 5.5% or more, about 6% or more, about 6.5% or more, about 7% or more, about 7.5% or more, about 8% or more, about 8.5% or more, about 9% or more, about 9.5% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 21%, about 22% or more, about 23% or more, about 24% or more, about 25% or more, about 26% or more, about 27% or more, about 28% or more, about 29% or more, about 30% or more, about 31% or more, about 32% or more, about 33% or more, about 34% or more, about 35% or more, about 36% or more, about 37% or more, about 38% or more, about 39% or more, about 40% or more, about 41% or more, about 42% or more, about 43% or more, about 44% or more, about 45% or more, about 46% or more, about 47% or more, about 48% or more, about 49% or more, about 50% or more, about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more) of the tumor sample.

For example, in some embodiments, the percentage of sTILs is from about 5% to 50%, from 5% to 45%, from 5% to 40%, from 5% to 35%, from 5% to 30%, from 5% to 25%, from 5% to 20%, from 5% to 15%, from 5% to 10%, from 5% to 9%, from 5% to 8%, from 5% to 7%, from 5% to 6%, from 6% to 50%, from 6% to 45%, from 6% to 40%, from 6% to 35%, from 6% to 30%, from 6% to 25%, from 6% to 20%, from 6% to 15%, from 6% to 10%, from 6% to 9%, from 6% to 8%, from 6% to 7%, from 7% to 50%, from 7% to 45%, from 7% to 40%, from 7% to 35%, from 7% to 30%, from 7% to 25%, from 7% to 20%, from 7% to 15%, from 7% to 10%, from 7% to 9%, from 7% to 8%, from 8% to 50%, from 8% to 45%, from 8% to 40%, from 8% to 35%, from 8% to 30%, from 8% to 25%, from 8% to 20%, from 8% to 15%, from 8% to 10%, from 8% to 9%, from 9% to 50%, from 9% to 45%, from 9% to 40%, from 9% to 35%, from 9% to 30%, from 9% to 25%, from 9% to 20%, from 9% to 15%, from 9% to 10%, from 10% to 50%, from 10% to 45%, from 10% to 40%, from 10% to 35%, from 10% to 30%, from 10% to 25%, from 10% to 20%, or from 10% to 15% of the tumor sample.

It is to be understood that in any of the preceding methods, the percentage of sTILs of the tumor sample may be the area occupied by mononuclear inflammatory cells over the total intratumoral stromal area. The percentage of sTILs may be assessed using any suitable approach known in the art, e.g., as described in Salgado et al. Annals of Oncology 26:259-271, 2015.

Any of the methods described herein may include determining the presence and/or expression level of two or more of PD-L1, CD8, and sTILs. For example, in some embodiments, the method includes determining the presence and/or expression level of PD-L1 and CD8. In another embodiment, the method includes determining the presence and/or expression level of PD-L1 and sTILs. In another embodiment, the method includes determining the presence and/or expression level of CD8 and sTILs. In another embodiment, the method includes determining the presence and/or expression level of PD-L1, CD8, and sTILs.

In some embodiments of any of the preceding aspects, the locally advanced TNBC is unresectable.

In some embodiments, the patient is a human. In some embodiments, the patient is suffering from locally advanced or metastatic breast cancer (e.g., TNBC). In some embodiments, the patient is suffering from locally advanced TNBC (e.g., unresectable locally advanced TNBC). In some embodiments, the metastatic breast cancer is mTNBC. In some embodiments, the patient has had two or fewer prior cytotoxic treatment regimens for locally advanced or metastatic breast cancer. In some embodiments, the patient has never had prior targeted systemic treatment for locally advanced or metastatic breast cancer. Thus, in certain embodiments, the methods for treating or delaying progression of locally advanced or metastatic breast cancer (e.g., locally advanced or metastatic TNBC) in an individual or for enhancing immune function in an individual having a locally advanced or metastatic breast cancer can serve as a first-line therapy for the individual.

In some embodiments, the individual has been treated with a cancer therapy before the combination treatment with a PD-1 axis binding antagonist (e.g., an anti-PD-L1 antibody (e.g., atezolizumab) or an anti-PD-1 antibody) and a taxane (e.g., nab-paclitaxel or paclitaxel). In some embodiments, the individual has cancer that is resistant to one or more cancer therapies. In some embodiments, resistance to cancer therapy includes recurrence of cancer or refractory cancer. Recurrence may refer to the reappearance of cancer, in the original site or a new site, after treatment. In some embodiments, resistance to a cancer therapy includes progression of the cancer during treatment with the anti-cancer therapy. In some embodiments, resistance to a cancer therapy includes cancer that does not response to treatment. The cancer may be resistant at the beginning of treatment or it may become resistant during treatment. In some embodiments, the cancer is at early stage or at late stage.

In some embodiments of any of the preceding aspects, the tumor sample is a formalin-fixed and paraffin-embedded (FFPE) tumor sample, an archival tumor sample, a fresh tumor sample, or a frozen tumor sample.

In some embodiments of any of the preceding aspects, the PD-1 binding antagonist is a human PD-1 binding antagonist, such as an anti-PD-L1 antibody (e.g., atezolizumab) or an anti-PD-1 antibody. In some embodiments, the PD-1 binding antagonist is atezolizumab.

In some embodiments of any of the preceding aspects, the taxane is nab-paclitaxel.

In other embodiments of any of the preceding aspects, the taxane is paclitaxel.

In some embodiments of any of the preceding aspects, whether the patient is likely to respond to treatment with the anti-cancer therapy is determined in terms of progression-free survival. For example, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) may increase the patient's progression-free survival by about 1 month, about 2 months, about 2.5 months, about 3 months, about 3.5 months, about 4 months, about 4.5 months, about 5 months, about 5.5 months, about 6 months, about 6.5 months, about 7 months, or longer, as compared to treatment with an anti-cancer therapy comprising the taxane without the PD-1 axis binding antagonist. In one embodiment, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) may increase the **patient's progression**-free survival by about 2.5 months as compared to treatment with an anti-cancer therapy comprising the taxane without the PD-1 axis binding antagonist.

In some embodiments of any of the preceding aspects, whether the patient is likely to respond to treatment with the anti-cancer therapy is determined in terms of overall survival. For example, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) may increase **the patient's overall survival by about 1 month,** about 2 months, about 3 months, about 4 months, about 5 months, about 6 months, about 7 months, about 8 months, about 9 months, about 10 months, about 11 months, about 12 months, about 13 months, about 14 months, or longer, as compared to treatment with an anti-cancer therapy comprising the taxane without the PD-1 axis binding antagonist. In one embodiment, treatment with an anti-cancer therapy comprising (i) a PD-1 axis binding antagonist (e.g., a PD-1 axis binding antagonist selected from an anti-PD-L1 antibody (e.g., atezolizumab) and an anti-PD-1 antibody) and (ii) a taxane (e.g., nab-paclitaxel or paclitaxel) **may increase the patient's overall survival by about 7 months as compared to treatment with** an anti-cancer therapy comprising the taxane without the PD-1 axis binding antagonist.

In some embodiments of any of the preceding aspects, whether the patient is likely to respond to treatment with the anti-cancer therapy is determined in terms of overall response rate.

In some embodiments of any of the preceding aspects, whether the patient is likely to respond to treatment with the anti-cancer therapy is determined in terms of complete response rate.

In some embodiments of any of the preceding aspects, whether the patient is likely to respond to treatment with the anti-cancer therapy is determined in terms of partial response rate.

The presence and/or expression level of any of the biomarkers described above (e.g., PD-L1, CD8, and/or sTILs) can be determined using any method described herein (e.g., in Section III above), or using approaches that are known in the art.

In some embodiments of any of the preceding methods and uses, the methods provided herein include administration of an effective amount of a PD-1 axis binding antagonist selected from a PD-L1 binding antagonist (e.g., an anti-PD-L1 antibody) and a PD-1 binding antagonist (e.g., an anti-PD-1 antibody). In some embodiments, the PD-L1 binding antagonist is an antibody, such as an antibody that is capable of inhibiting PD-L1 binding to PD-1 and B7.1, but does not disrupt binding of PD-1 to PD-L2. In some embodiments, the PD-L1 binding antagonist antibody is atezolizumab, which may be administered at a dose of about 700 mg to about 900 mg every two weeks (e.g., about 750 mg to about 900 mg every two weeks, e.g., about 800 mg to about 850 mg every two weeks). In some embodiments, atezolizumab is administered at a dose of about 840 mg every two weeks.

As a general proposition, the therapeutically effective amount of a PD-1 axis binding antagonist (e.g., an anti-PD-L1 antibody (e.g., atezolizumab) or an anti-PD-1 antibody) may be administered to a human will be in the range of about 0.01 to about 50 mg/kg of patient body weight whether by one or more administrations. In some embodiments, for example, the antagonist (e.g., an anti-PD-L1 antibody (e.g., atezolizumab) or an anti-PD-1 antibody) is administered in a dose of about 0.01 to about 45 mg/kg, about 0.01 to about 40 mg/kg, about 0.01 to about 35 mg/kg, about 0.01 to about 30 mg/kg, about 0.01 to about 25 mg/kg, about 0.01 to about 20 mg/kg, about 0.01 to about 15 mg/kg, about 0.01 to about 10 mg/kg, about 0.01 to about 5 mg/kg, or about 0.01 to about 1 mg/kg administered daily, for example. In some embodiments, the antagonist (e.g., an anti-PD-L1 antibody (e.g., atezolizumab) or an anti-PD-1 antibody) is administered at 15 mg/kg. However, other dosage regimens may be useful. In one embodiment, a PD-1 axis binding antagonist (e.g., an anti-PD-L1 antibody (e.g., atezolizumab) or an anti-PD-1 antibody) is administered to a human at a dose of about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, or about 1500 mg. In some embodiments, a PD-1 axis binding antagonist (e.g., an anti-PD-L1 antibody (e.g., atezolizumab) or an anti-PD-1 antibody) is administered at a dose of about 800 mg to about 850 mg every two weeks. In some embodiments, a PD-1 axis binding antagonist (e.g., an anti-PD-L1 antibody (e.g., atezolizumab) or an anti-PD-1 antibody) is administered at a dose of about 840 mg every two weeks. The dose may be administered as a single dose or as multiple doses (e.g., 2 or 3 doses), such as infusions. The dose of the antibody administered in a combination treatment may be reduced as compared to a single treatment. In some embodiments, for example, the method for treating or delaying progression of locally advanced or metastatic breast cancer in an individual comprises a dosing regimen comprising treatment cycles, wherein the individual is administered, on days 1 and 15 of each cycle, a human PD-1 axis binding antagonist (e.g., an anti-PD-L1 antibody (e.g., atezolizumab) or an anti-PD-1 antibody) at a dose of about 840 mg, wherein each cycle is 28 days (i.e., each cycle is repeated every 28 days). The progress of this therapy is easily monitored by conventional techniques.

In some embodiments, the methods provided herein include administration of an effective amount of a taxane (e.g., nab-paclitaxel (ABRAXANE®), paclitaxel, or docetaxel). As a general proposition, the therapeutically effective amount of a taxane (e.g., nab-paclitaxel or paclitaxel) administered to a human will be in the range of about 25 to about 300 mg/m$^2$ (e.g., about 25 mg/m$^2$, about 50 mg/m$^2$, about 75 mg/m$^2$, about 100 mg/m$^2$, about 125 mg/m$^2$, about 150 mg/m$^2$, about 175 mg/m$^2$, about 200 mg/m$^2$, about 225 mg/m$^2$, about 250 mg/m$^2$, about 275 mg/m$^2$, or about 300 mg/m$^2$), whether by one or more administrations. In some embodiments, the taxane is nab-paclitaxel (ABRAXANE®). In some embodiments, the nab-paclitaxel (ABRAXANE®) is administered to the individual at a dose of about 100 mg/m$^2$ to about 125 mg/m$^2$ every week. In some embodiments, the nab-paclitaxel (ABRAXANE®) is administered to the individual at a dose of about 100 mg/m$^2$ every week. For example, in some embodiments, about 100 mg/m$^2$ of nab-paclitaxel (ABRAXANE®) is administered. In some embodiments, nab-paclitaxel (ABRAXANE®) is administered at 100 mg/m$^2$ once a week. In some embodiments, about 125 mg/m$^2$ of paclitaxel is administered. In other embodiments, the taxane is paclitaxel. In some embodiments, the paclitaxel is administered to the individual at a dose of about 75 mg/m$^2$ to about 125 mg/m$^2$ every week. In some embodiments, the paclitaxel is administered to the individual at a dose of about 90 mg/m$^2$ t every week. In some embodiments, paclitaxel is administered at 200 mg/m$^2$ every three weeks. In some embodiments, the taxane (e.g., nab-paclitaxel or paclitaxel) may be administered weekly, every 2 weeks, every 3 weeks, every 4 weeks, on days 1, 8 and 15 of each 21-day cycle, or on days 1, 8, and 15 of each 28-day cycle. For example, in some embodiments, the nan- paclitaxel is administered to the individual on days 1, 8, and 15 of each 28-day cycle. In another example, in some embodiments, the paclitaxel is administered to the individual on days 1, 8, and 15 of each 28-day cycle.

In some embodiments, the PD-1 axis binding antagonist (e.g., an anti-PD-L1 antibody (e.g., atezolizumab) or an anti-PD-1 antibody) and the taxane (e.g., nab-paclitaxel or paclitaxel) are administered in a single dosing regimen. The administration of these agents may be concurrent or separate within the context of the dosing regimen. For example, in some embodiments, the methods provided herein include a dosing regimen comprising treatment cycles, wherein the individual is administered, on days 1 and 15 of each cycle, a human PD-1 axis binding antagonist (e.g., an anti-PD-L1 antibody (e.g., atezolizumab) or an anti-PD-1 antibody) at a dose of about 840 mg, and on days 1, 8, and 15 of each cycle, a taxane (e.g., nab-paclitaxel or paclitaxel) at a dose of about 80 mg/m$^2$ to about 100 mg/m$^2$, each cycle being repeated every 28 days. For example, in some embodiments, the methods provided herein include a dosing regimen comprising treatment cycles, wherein the individual is administered, on days 1 and 15 of each cycle, atezolizumab at a dose of about 840 mg, and on days 1, 8, and 15 of each cycle, nab-paclitaxel at a dose of about 100 mg/m$^2$, each cycle being repeated every 28 days. In another example, in some embodiments, the methods provided herein include a dosing regimen comprising treatment cycles, wherein the individual is administered, on days 1 and 15 of each cycle, atezolizumab at a dose of about 840 mg, and on days 1, 8, and 15 of each cycle, paclitaxel at a dose of about 90 mg/m$^2$, each cycle being repeated every 28 days.

In some embodiments, the methods further comprise administering an effective amount of a chemotherapeutic agent. In some embodiments, the chemotherapeutic agent is a platinum-based chemotherapeutic agent, such as carboplatin.

In some embodiments, the combination therapy of the invention comprises administration of a PD-1 axis binding antagonist (e.g., an anti-PD-L1 antibody (e.g., atezolizumab) or an anti-PD-1 antibody) and a taxane (e.g., nab-paclitaxel or paclitaxel). The PD-1 axis binding antagonist (e.g., an anti-PD-L1 antibody (e.g., atezolizumab) or an anti-PD-1 antibody) and the taxane (e.g., nab-paclitaxel or paclitaxel) may be administered in any suitable manner known in the art. For example, the PD-1 axis binding antagonist (e.g., an anti-PD-L1 antibody (e.g., atezolizumab) or an anti-PD-1 antibody) and the taxane (e.g., nab-paclitaxel or paclitaxel) may be administered sequentially (at different times) or concurrently (at the same time). In some embodiments, the PD-1 axis binding antagonist (e.g., an anti-PD-L1 antibody (e.g., atezolizumab) or an anti-PD-1 antibody) is in a separate composition as the taxane (e.g., nab-paclitaxel or paclitaxel). In some embodiments, the PD-1 axis binding antagonist (e.g., an anti-PD-L1 antibody (e.g., atezolizumab) or an anti-PD-1 antibody) is in the same composition as the taxane (e.g., nab-paclitaxel or paclitaxel).

The PD-1 axis binding antagonist (e.g., an anti-PD-L1 antibody (e.g., atezolizumab) or an anti-PD-1 antibody) and the taxane (e.g., nab-paclitaxel or paclitaxel) may be administered by the same route of administration or by different routes of administration. In some embodiments, the PD-1 axis binding antagonist (e.g., an anti-PD-L1 antibody (e.g., atezolizumab) or an anti-PD-1 antibody) and/or the taxane (e.g., nab-paclitaxel or paclitaxel) is administered intravenously, intramuscularly, subcutaneously, topically, orally, transdermally, intraperitoneally, intraorbitally, by implantation, by inhalation, intrathecally, intraventricularly, or intranasally. In some embodiments, the taxane (e.g., nab-paclitaxel or paclitaxel) is administered intravenously, intramuscularly, subcutaneously, topically, orally, transdermally, intraperitoneally, intraorbitally, by implantation, by inhalation, intrathecally, intraventricularly, or intranasally. An effective amount of the PD-1 axis binding antagonist (e.g., an anti-PD-L1 antibody (e.g., atezolizumab) or an anti-PD-1 antibody) and the taxane (e.g., nab-paclitaxel or paclitaxel) may be administered for prevention or treatment of disease. The appropriate dosage of the PD-1 axis binding antagonist (e.g., an anti-PD-L1 antibody (e.g., atezolizumab) or an anti-PD-1 antibody) and/or the taxane (e.g., nab-paclitaxel or paclitaxel) may be determined based on the type of disease to be treated, the type of the PD-1 axis binding antagonist and the taxane, the severity and course of the disease, the clinical condition of the individual, the individual's clinical history and response to the treatment, and the discretion of the attending physician. In some embodiments, the PD-1 axis binding antagonist (e.g., an anti-PD-L1 antibody (e.g., atezolizumab) or an anti-PD-1 antibody) and/or the taxane (e.g., nab-paclitaxel or paclitaxel) is administered intravenously by infusion.

In some embodiments, the methods may further comprise an additional therapy. The additional therapy may be radiation therapy, surgery (e.g., lumpectomy and a mastectomy), chemotherapy, gene therapy, DNA therapy, viral therapy, RNA therapy, immunotherapy, bone marrow transplantation, nanotherapy, monoclonal antibody therapy, or a combination of the foregoing. The additional therapy may be in the form of adjuvant or neoadjuvant therapy. In some embodiments, the additional therapy is the administration of small molecule enzymatic inhibitor or anti-metastatic agent. In some embodiments, the additional therapy is the administration of side-effect limiting agents (e.g., agents intended to lessen the occurrence and/or severity of side effects of treatment, such as anti-nausea agents, etc.). In some embodiments, the additional therapy is radiation therapy. In some embodiments, the additional therapy is surgery. In some embodiments, the additional therapy is a combination of radiation therapy and surgery. In some embodiments, the additional therapy is gamma irradiation. In some embodiments, the additional therapy is therapy targeting PI3K/AKT/mTOR pathway, HSP90 inhibitor, tubulin inhibitor, apoptosis inhibitor, and/or chemopreventative agent. The additional therapy may be one or more of the chemotherapeutic agents described herein.

In some embodiments, the methods further comprise administering a platinum-based chemotherapeutic agent with the PD-1 axis binding antagonist (e.g., an anti-PD-L1 antibody (e.g., atezolizumab) or an anti-PD-1 antibody) and taxane (e.g., nab-paclitaxel or paclitaxel). In some embodiments, the platinum-based chemotherapeutic agent is carboplatin. Dosages and administration of carboplatin are well-known in the art. An exemplary dosage of carboplatin is administered with a target area under the curve (AUC) of 6 mg/ml. In some embodiments, the carboplatin is administered intravenously every 3 weeks.

In some embodiments, the methods include treating an individual suffering from mTNBC by administering anti-PD-L1 antibody atezolizumab at 800 mg IV administered every two weeks (q2w), along with nab-paclitaxel (ABRAXANE®) at 125 mg/m$^2$ IV every week (q1w), for three weeks within the context of a 4-week (28-day) treatment cycle, which may be repeated until there is loss of clinical benefit, complete response, remission, or otherwise, at the discretion of the attending physician.

**V. Other Combination Therapies**

Also provided herein are methods for treating or delaying progression of breast cancer (e.g., locally advanced or metastatic breast cancer (e.g., locally advanced or metastatic TNBC)) in an individual comprising administering to the individual a PD-1 axis binding antagonist (e.g., an anti-PD-L1 antibody (e.g., atezolizumab) or an anti-PD-1 antibody) and a taxane (e.g., nab-paclitaxel or paclitaxel) in conjunction with another anti-cancer agent or cancer therapy. In some embodiments, the methods comprise administering to the individual a PD-1 axis binding antagonist (e.g., an anti-PD-L1 antibody (e.g., atezolizumab) or an anti-PD-1 antibody), a taxane (e.g., nab-paclitaxel or paclitaxel), and an additional therapeutic agent.

In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with a chemotherapy or chemotherapeutic agent. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with a radiation therapy or radiotherapeutic agent. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with a targeted therapy or targeted therapeutic agent. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with an immunotherapy or immunotherapeutic agent, for example a monoclonal antibody.

Without wishing to be bound to theory, it is thought that enhancing T cell stimulation, by promoting an activating co-stimulatory molecule or by inhibiting a negative co-stimulatory molecule, may promote tumor cell death thereby treating or delaying progression of cancer. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with an agonist directed against an activating co-stimulatory molecule. In some embodiments, an activating co-stimulatory molecule may include CD40, CD226, CD28, OX40, GITR, CD137, CD27, HVEM, or CD127. In some embodiments, the agonist directed against an activating co-stimulatory molecule is an agonist antibody that binds to CD40, CD226, CD28, OX40, GITR, CD137, CD27, HVEM, or CD127. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with an antagonist directed against an inhibitory co-stimulatory molecule. In some embodiments, an inhibitory co-stimulatory molecule may include CTLA-4 (also known as CD152), PD-1, TIM-3, BTLA, VISTA, LAG-3, B7-H3, B7-H4, IDO, TIGIT, MICA/B, or arginase. In some embodiments, the antagonist directed against an inhibitory co-stimulatory molecule is an antagonist antibody that binds to CTLA-4, PD-1, TIM-3, BTLA, VISTA, LAG-3, B7-H3, B7-H4, IDO, TIGIT, MICA/B, or arginase.

In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with an antagonist directed against CTLA-4 (also known as CD152), for example, a blocking antibody. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with ipilimumab (also known as MDX-010, MDX-101, or YERVOY®). In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with tremelimumab (also known as ticilimumab or CP-675,206). In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with an antagonist directed against B7-H3 (also known as CD276), for example, a blocking antibody. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with MGA271. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with an antagonist directed against a TGF beta, for example, metelimumab (also known as CAT-192), fresolimumab (also known as GC1008), or LY2157299.

In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with a treatment comprising adoptive transfer of a T cell (e.g., a cytotoxic T cell or CTL) expressing a chimeric antigen receptor (CAR). In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with a treatment comprising adoptive transfer of a T cell comprising a dominant-negative TGF beta receptor, e.g., a dominant-negative TGF beta type II receptor. In some embodiments, a PD-1 axis binding antagonist (e.g., an anti-PD-L1 antibody (e.g., atezolizumab) or an anti-PD-1 antibody) and a taxane (e.g., nab-paclitaxel or paclitaxel) may be administered in conjunction with a treatment comprising a HERCREEM protocol (see, e.g., ClinicalTrials.gov Identifier NCT00889954).

In some embodiments, a PD-1 axis binding antagonist and a taxane may be administered in conjunction with an agonist directed against CD137 (also known as TNFRSF9, 4-1BB, or ILA), for example, an activating antibody. In some embodiments, a PD-1 axis binding antagonist and a taxane may be administered in conjunction with urelumab (also known as BMS-663513). In some embodiments, a PD-1 axis binding antagonist and a taxane may be administered in conjunction with an agonist directed against CD40, for example, an activating antibody. In some embodiments, a PD-1 axis binding antagonist and a taxane may be administered in conjunction with CP-870893. In some embodiments, a PD-1 axis binding antagonist and a taxane may be administered

PD-1 axis binding antagonist and a taxane may be administered in conjunction with an agonist directed against OX40 (also known as CD134), for example, an activating antibody. In some embodiments, a PD-1 axis binding antagonist and a taxane may be administered in conjunction with an anti-OX40 antibody (e.g., AgonOX). In some embodiments, a PD-1 axis binding antagonist and a taxane may be administered in conjunction with an agonist directed against CD27, for example, an activating antibody. In some embodiments, a PD-1 axis binding antagonist and a taxane may be administered in conjunction with CDX-1127. In some embodiments, a PD-1 axis binding antagonist and a taxane may be administered in conjunction with an antagonist directed against indoleamine-2,3-dioxygenase (IDO). In some embodiments, with the IDO antagonist is 1-methyl-D-tryptophan (also known as 1-D-MT).

In some embodiments, a PD-1 axis binding antagonist and a taxane may be administered in conjunction with an antibody-drug conjugate. In some embodiments, the antibody-drug conjugate comprises mertansine or monomethyl auristatin E (MMAE). In some embodiments, a PD-1 axis binding antagonist and a taxane may be administered in conjunction with and anti-NaPi2b antibody-MMAE conjugate (also known as DNIB0600A or RG7599). In some embodiments, a PD-1 axis binding antagonist and a taxane may be administered in conjunction with trastuzumab emtansine (also known as T-DM1, ado-trastuzumab emtansine, or KADCYLA®, Genentech). In some embodiments, a PD-1 axis binding antagonist and a taxane may be administered in conjunction with DMUC5754A. In some embodiments, a PD-1 axis binding antagonist and a taxane may be administered in conjunction with an antibody-drug conjugate targeting the endothelin B receptor (EDNBR), for example, an antibody directed against EDNBR conjugated with MMAE.

In some embodiments, a PD-1 axis binding antagonist and a taxane may be administered in conjunction with an angiogenesis inhibitor. In some embodiments, a PD-1 axis binding antagonist and a taxane may be administered in conjunction with an antibody directed against a VEGF, for example, VEGF-A. In some embodiments, a PD-1 axis binding antagonist (e.g., anti-PD-L1 antibody, e.g., MPDL3280A) and a taxane (e.g., nab-paclitaxel or paclitaxel) may be administered in conjunction with bevacizumab (also known as AVASTIN®, Genentech). In some embodiments, a PD-1 axis binding antagonist and a taxane may be administered in conjunction with an antibody directed against angiopoietin 2 (also known as Ang2). In some embodiments, a PD-1 axis binding antagonist and a taxane may be administered in conjunction with MEDI3617.

In some embodiments, a PD-1 axis binding antagonist and a taxane may be administered in conjunction with an antineoplastic agent. In some embodiments, a PD-1 axis binding antagonist and a taxane may be administered in conjunction with an agent targeting CSF-1R (also known as M-CSFR or CD115). In some embodiments, a PD-1 axis binding antagonist and a taxane may be administered in conjunction with anti-CSF-1R (also known as IMC-CS4). In some embodiments, a PD-1 axis binding antagonist and a taxane may be administered in conjunction with an interferon, for example interferon alpha or interferon gamma. In some embodiments, a PD-1 axis binding antagonist and a taxane may be administered in conjunction with Roferon-A (also known as recombinant Interferon alpha-2a). In some embodiments, a PD-1 axis binding antagonist and a taxane may be administered in conjunction with GM-CSF (also known as recombinant human granulocyte macrophage colony stimulating factor, rhu GM-CSF, sargramostim, or LEUKINE®). In some embodiments, a PD-1 axis binding antagonist and a taxane may be administered in conjunction with IL-2 (also known as aldesleukin or PROLEUKIN®). In some embodiments, a PD-1 axis binding antagonist and a taxane may be administered in conjunction with IL-12. In some embodiments, a PD-1 axis binding antagonist and a taxane may be administered in conjunction with an antibody targeting CD20. In some embodiments, the antibody targeting CD20 is obinutuzumab (also known as GA101 or GAZYVA®) or rituximab. In some embodiments, a PD-1 axis binding antagonist and a taxane may be administered in conjunction with an antibody targeting GITR. In some embodiments, the antibody targeting GITR is TRX518.

In some embodiments, a PD-1 axis binding antagonist and a taxane may be administered in conjunction with a cancer vaccine. In some embodiments, the cancer vaccine is a peptide cancer vaccine, which in some embodiments is a personalized peptide vaccine. In some embodiments the peptide cancer vaccine is a multivalent long peptide, a multi-peptide, a peptide cocktail, a hybrid peptide, or a peptide-pulsed dendritic cell vaccine (see, e.g., Yamada et al., Cancer Sci, 104:14-21, 2013). In some embodiments, a PD-1 axis binding antagonist and a taxane may be administered in conjunction with an adjuvant. In some embodiments, a PD-1 axis binding antagonist and a taxane may be administered in conjunction with a treatment comprising a TLR agonist, for example, Poly-ICLC (also known as HILTONOL®), LPS, MPL, or CpG ODN. In some embodiments, a PD-1 axis binding antagonist and a taxane may be administered in conjunction with tumor necrosis factor (TNF) alpha. In some embodiments, a PD-1 axis binding antagonist and a taxane may be administered in conjunction with IL-1. In some embodiments, a PD-1 axis binding antagonist and a taxane may be administered in conjunction with HMGB1. In some embodiments, a PD-1 axis binding antagonist and a taxane may be administered in conjunction with an IL-10 antagonist. In some embodiments, a PD-1 axis binding antagonist and a taxane may be administered in conjunction with an IL-4 antagonist. In some embodiments, a PD-1 axis binding antagonist and a taxane may be administered in conjunction with an IL-13 antagonist. In some embodiments, a PD-1 axis binding antagonist and a taxane may be administered in conjunction with an HVEM antagonist. In some embodiments, a PD-1 axis binding antagonist and a taxane may be administered in conjunction with an ICOS agonist, e.g., by administration of ICOS-L, or an agonistic antibody directed against ICOS. In some embodiments, a PD-1 axis binding antagonist and a taxane may be administered in conjunction with a treatment targeting CX3CL1. In some embodiments, a PD-1 axis binding antagonist and a taxane may be administered in conjunction with a treatment targeting CXCL9. In some embodiments, a PD-1 axis binding antagonist and a taxane may be administered in conjunction with a treatment targeting CXCL10. In some embodiments, a PD-1 axis binding antagonist and a taxane may be administered in conjunction with a treatment targeting CCL5. In some embodiments, a PD-1 axis binding antagonist and a taxane may be administered in conjunction with an LFA-1 or ICAM1 agonist. In some embodiments, a PD-1 axis binding antagonist and a taxane may be administered in conjunction with a Selectin agonist.

In some embodiments, a PD-1 axis binding antagonist and a taxane may be administered in conjunction with a targeted therapy. In some embodiments, a PD-1 axis binding antagonist and a taxane may be administered in conjunction with an inhibitor of B-Raf. In some embodiments, a PD-1 axis binding antagonist and a taxane may be administered in conjunction with vemurafenib (also known as ZELBORAF®). In some embodiments, a PD-1 axis binding antagonist and a taxane may be administered in conjunction with dabrafenib (also known as TAFINLAR®). In some embodiments, a PD-1 axis binding antagonist and a taxane may be administered in conjunction with erlotinib (also known as TARCEVA®). In some embodiments, a PD-1 axis binding antagonist and a taxane may be administered in conjunction with an inhibitor of a MEK, such as MEK1 (also known as MAP2K1) or MEK2 (also known as MAP2K2). In some embodiments, a PD-1 axis binding antagonist and a taxane may be administered in conjunction with cobimetinib (also known as GDC-0973 or XL-518). In some embodiments, a PD-1 axis binding antagonist and a taxane may be administered in conjunction with trametinib (also known as MEKINIST®). In some embodiments, a PD-1 axis binding antagonist and a taxane may be administered in conjunction with an inhibitor of K-Ras. In some embodiments, a PD-1 axis binding antagonist and a taxane may be administered in conjunction with an inhibitor of c-Met. In some embodiments, a PD-1 axis binding antagonist and a taxane may be administered in conjunction with onartuzumab (also known as MetMAb). In some embodiments, a PD-1 axis binding antagonist and a taxane may be administered in conjunction with an inhibitor of Alk. In some embodiments, a PD-1 axis binding antagonist and a taxane may be administered in conjunction with AF802 (also known as CH5424802 or alectinib). In some embodiments, a PD-1 axis binding antagonist and a taxane may be administered in conjunction with an inhibitor of a phosphatidylinositol 3-kinase (PI3K). In some embodiments, a PD-1 axis binding antagonist and a taxane may be administered in conjunction with BKM120. In some embodiments, a PD-1 axis binding antagonist and a taxane may be administered in conjunction with idelalisib (also known as GS-1101 or CAL-101). In some embodiments, a PD-1 axis binding antagonist and a taxane may be administered in conjunction with perifosine (also known as KRX-0401). In some embodiments, a PD-1 axis binding antagonist and a taxane may be administered in conjunction with an inhibitor of an Akt. In some embodiments, a PD-1 axis binding antagonist may be administered in conjunction with MK2206. In some embodiments, a PD-1 axis binding antagonist and a taxane may be administered in conjunction with GSK690693. In some embodiments, a PD-1 axis binding antagonist and a taxane may be administered in conjunction with GDC-0941. In some embodiments, a PD-1 axis binding antagonist and a taxane may be administered in conjunction with an inhibitor of mTOR. In some embodiments, a PD-1 axis binding antagonist and a taxane may be administered in conjunction with sirolimus (also known as rapamycin). In some embodiments, a PD-1 axis binding antagonist and a taxane may be administered in conjunction with temsirolimus (also known as CCI-779 or TORISEL®). In some embodiments, a PD-1 axis binding antagonist and a taxane may be administered in conjunction with everolimus (also known as RAD001). In some embodiments, a PD-1 axis binding antagonist and a taxane may be administered in conjunction with ridaforolimus (also known as AP-23573, MK-8669, or deforolimus). In some embodiments, a PD-1 axis binding antagonist and a taxane may be administered in conjunction with OSI-027. In some embodiments, a PD-1 axis binding antagonist and a taxane may be administered in conjunction with AZD8055. In some embodiments, a PD-1 axis binding antagonist and a taxane may be administered in conjunction with INK128. In some embodiments, a PD-1 axis binding antagonist and a taxane may be administered in conjunction with a dual PI3K/mTOR inhibitor. In some embodiments, a PD-1 axis binding antagonist and a taxane may be administered in conjunction with XL765. In some embodiments, a PD-1 axis binding antagonist and a taxane may be administered in conjunction with GDC-0980. In some embodiments, a PD-1 axis binding antagonist and a taxane may be administered in conjunction with BEZ235 (also known as NVP-BEZ235). In some embodiments, a PD-1 axis binding antagonist and a taxane may be administered in conjunction with BGT226. In some embodiments, a PD-1 axis binding antagonist and a taxane may be administered in conjunction with GSK2126458. In some embodiments, a PD-1 axis binding antagonist and a taxane may be administered in conjunction with PF-04691502. In some embodiments, a PD-1 axis binding antagonist and a taxane may be administered in conjunction with PF-05212384 (also known as PKI-587).

In any of the preceding embodiments, the PD-1 axis binding antagonist may be a human PD-1 axis binding antagonist.

In some embodiments of any of the preceding aspects, the PD-1 axis binding antagonist is an anti-PD-L1 antibody (e.g., atezolizumab) or an anti-PD-1 antibody.

In some embodiments of any of the preceding aspects, the taxane is nab-paclitaxel.

In other embodiments of any of the preceding aspects, the taxane is paclitaxel.

**VI. PD-1 Axis Binding Antagonists**

Provided herein are methods of identifying a patient who is likely to respond to treatment with an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel). Also provided are methods of selecting a therapy for a patient suffering from a breast cancer (e.g., a locally advanced or metastatic TNBC), e.g., an anti-cancer therapy that includes a PD-1 axis binding antagonist (e.g., an anti-PD-L1 antibody (e.g., atezolizumab) or an anti-PD-1 antibody) and a taxane (e.g., nab-paclitaxel or paclitaxel). Further provided herein are methods for treating or delaying progression of breast cancer (e.g., locally advanced or metastatic breast cancer (e.g., locally advanced or metastatic TNBC)) in an individual comprising administering to the individual an effective amount of a PD-1 axis binding antagonist (e.g., an anti-PD-L1 antibody (e.g., atezolizumab) or an anti-PD-1 antibody) and a taxane (e.g., nab-paclitaxel or paclitaxel). For example, in some embodiments, the patient is PD-L1-positive. Also provided herein are methods of enhancing immune function in an individual having breast cancer (e.g., locally advanced or metastatic breast cancer (e.g., locally advanced or metastatic TNBC)) comprising administering to the individual an effective amount of a PD-1 axis binding antagonist (e.g., an anti-PD-L1 antibody (e.g., atezolizumab) or an anti-PD-1 antibody) and an taxane (e.g., nab-paclitaxel or paclitaxel). Any of the methods described herein may involve any of the PD-1 axis binding antagonists described below.

For example, a PD-1 axis binding antagonist includes a PD-L1 binding antagonist, a PD-1 binding antagonist, and a PD-L2 binding antagonist. PD-L1 (programmed death ligand 1) is also referred to in the art as "programmed cell death 1 ligand 1," "PDCD1LG1," "CD274," "B7-H," and "PDL1." An exemplary human PD-L1 is shown in UniProtKB/Swiss-Prot Accession No.Q9NZQ7.1. PD-1 (programmed death 1) is also referred to in the art as "programmed cell death 1," "PDCD1," "CD279," and "SLEB2." An exemplary human PD-1 is shown in UniProtKB/Swiss-Prot Accession No. Q15116. PD-L2 (programmed death ligand 2) is also referred to in the art as "programmed cell death 1 ligand 2," "PDCD1LG2," "CD273," "B7-DC," "Btdc," and "PDL2." An exemplary human PD-L2 is shown in UniProtKB/Swiss-Prot Accession No. Q9BQ51. In some embodiments, PD-L1, PD-1, and PD-L2 are human PD-L1, PD-1, and PD-L2.

In some embodiments, the PD-1 axis binding antagonist is an anti-PD-L1 antibody. In some embodiments, the anti-PD-L1 antibody is selected from the group consisting of atezolizumab, YW243.55.S70, MDX-1105, MEDI4736 (durvalumab), and MSB0010718C (avelumab). Antibody YW243.55.S70 is an anti-PD-L1 antibody described in WO 2010/077634. MDX-1105, also known as BMS-936559, is an anti-PD-L1 antibody described in WO2007/005874. MEDI4736 is an anti-PD-L1 monoclonal antibody described in WO2011/066389 and US2013/034559. In some embodiments, the anti-PD-L1 antibody is capable of inhibiting binding between PD-L1 and PD-1 and/or between PD-L1 and B7-1. In some embodiments, the anti-PD-L1 antibody is a monoclonal antibody. In some embodiments, the anti-PD-L1 antibody is an antibody fragment selected from the group consisting of Fab, Fab'-SH, Fv, scFv, and (Fab')$_2$ fragments. In some embodiments, the anti-PD-L1 antibody is a humanized antibody. In some embodiments, the anti-PD-L1 antibody is a human antibody.

Examples of anti-PD-L1 antibodies useful for the methods of this invention, and methods for making thereof are described in PCT patent application WO 2010/077634, WO 2007/005874, WO 2011/066389, and US 2013/034559, which are incorporated herein by reference. The anti-PD-L1 antibodies useful in this invention, including compositions containing such antibodies, may be used in combination with a taxane to treat cancer.

In some embodiments, the PD-1 binding antagonist is a molecule that inhibits the binding of PD-1 to its ligand binding partners. In a specific aspect the PD-1 ligand binding partners are PD-L1 and/or PD-L2. In another embodiment, a PD-L1 binding antagonist is a molecule that inhibits the binding of PD-L1 to its binding partners. In a specific aspect, PD-L1 binding partners are PD-1 and/or B7-1. In another embodiment, the PD-L2 binding antagonist is a molecule that inhibits the binding of PD-L2 to its binding partners. In a specific aspect, a PD-L2 binding partner is PD-1. The antagonist may be an antibody, an antigen binding fragment thereof, an immunoadhesin, a fusion protein, or oligopeptide.

In some embodiments, the PD-1 binding antagonist is an anti-PD-1 antibody (e.g., a human antibody, a humanized antibody, or a chimeric antibody). In some embodiments, the anti-PD-1 antibody is selected from the group consisting of MDX 1106 (nivolumab), MK-3475 (pembrolizumab), MEDI-0680 (AMP-514), PDR001, REGN2810, and BGB-108. In some embodiments, the PD-1 binding antagonist is an immunoadhesin (e.g., an immunoadhesin comprising an extracellular or PD-1 binding portion of PD-L1 or PD-L2 fused to a constant region (e.g., an Fc region of an immunoglobulin sequence). In some embodiments, the PD-1 binding antagonist is AMP-224. In some embodiments, the PD-L1 binding antagonist is anti-PD-L1 antibody. MDX-1106, also known as MDX-1106-04, ONO-4538, BMS-936558, or nivolumab, is an anti-PD-1 antibody described in WO2006/121168. MK-3475, also known as lambrolizumab, is an anti-PD-1 antibody described in WO2009/114335. AMP-224, also known as B7-DCIg, is a PD-L2-Fc fusion soluble receptor described in WO2010/027827 and WO2011/066342.

*Anti-PD-L 1 antibodies*

In some embodiments, the antibody in the formulation comprises at least one tryptophan (e.g., at least two, at least three, or at least four) in the heavy and/or light chain sequence. In some embodiments, amino acid tryptophan is in the HVR regions, framework regions and/or constant regions of the antibody. In some embodiments, the antibody comprises two or three tryptophan residues in the HVR regions. In some embodiments, the antibody in the formulation is an anti-PD-L1 antibody. PD-L1 (programmed death ligand 1), also known as PDL1, B7-H1, B7-4, CD274, and B7-H, is a transmembrane protein, and its interaction with PD-1 inhibits T-cell activation and cytokine production. In some embodiments, the anti-PD-L1 antibody described herein binds to human PD-L1. Examples of anti-PD-L1 antibodies that can be used in the methods described herein are described in PCT patent application WO 2010/077634 A1 and U.S. Patent No. 8,217,149, which are incorporated herein by reference in their entirety.

In some embodiments, the anti-PD-L1 antibody is capable of inhibiting binding between PD-L1 and PD-1 and/or between PD-L1 and B7-1. In some embodiments, the anti-PD-L1 antibody is a monoclonal antibody. In some embodiments, the anti-PD-L1 antibody is an antibody fragment selected from the group consisting of Fab, Fab'-SH, Fv, scFv, and (Fab')$_2$ fragments. In some embodiments, the anti-PD-L1 antibody is a humanized antibody. In some embodiments, the anti-PD-L1 antibody is a human antibody.

Anti-PD-L1 antibodies described in WO 2010/077634 A1 and US 8,217,149 may be used in the methods described herein. In some embodiments, the anti-PD-L1 antibody comprises a heavy chain variable region sequence of SEQ ID NO:3 and/or a light chain variable region sequence of SEQ ID NO:4. In a still further embodiment, provided is an isolated anti-PD-L1 antibody comprising a heavy chain variable region and/or a light chain variable region sequence, wherein:

(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence:

EVQLVESGGGLVQPGGSLRLSCAASGFTFSDSWIHWVRQAPGKGLEWVAWISPYGGSTYYADSVKGRF

TISADTSKNTAYLQMNSLRAEDTAVYYCARRHWPGGFDYWGQGTLVTVSA (SEQ ID NO:3),

and

(b) the light chain sequence has at least 85%, at least 90%, at least 91%, at least 92%, at least 93%, at least 94%, at least 95%, at least 96%, at least 97%, at least 98%, at least 99% or 100% sequence identity to the light chain sequence:

DIQMTQSPSSLSASVGDRVTITCRASQDVSTAVAWYQQKPGKAPKLLIYSASFLYSGVPSRFSGSGSGTD

FTLTISSLQPEDFATYYCQQYLYHPATFGQGTKVEIKR (SEQ ID NO:4).

In one embodiment, the anti-PD-L1 antibody comprises a heavy chain variable region comprising an HVR-H1, HVR-H2 and HVR-H3 sequence, wherein:

(a) the HVR-H1 sequence is GFTFSX$_1$SWIH (SEQ ID NO:5);
(b) the HVR-H2 sequence is AWIX$_2$PYGGSX$_3$YYADSVKG (SEQ ID NO:6);
(c) the HVR-H3 sequence is RHWPGGFDY (SEQ ID NO:7);

further wherein: X$_1$ is D or G; X$_2$ is S or L; X$_3$ is T or S. In one specific aspect, X$_1$ is D; X$_2$ is S and X$_3$ is T.

In another aspect, the polypeptide further comprises variable region heavy chain framework sequences juxtaposed between the HVRs according to the formula: (HC-FR1)-(HVR-H1)-(HC-FR2)-(HVR-H2)-(HC-FR3)-(HVR-H3)-(HC-FR4). In yet another aspect, the framework sequences are derived from human consensus framework sequences. In a further aspect, the framework sequences are VH subgroup III consensus framework. In a still further aspect, at least one of the framework sequences is the following:

HC-FR1 is EVQLVESGGGLVQPGGSLRLSCAAS (SEQ ID NO:8)
HC-FR2 is VWRQAPGKGLEVW (SEQ ID NO:9)
HC-FR3 is RFTISADTSKNTAYLQMNSLRAEDTAVYYCAR (SEQ ID NO:10)
HC-FR4 is WGQGTLVTVSA (SEQ ID NO:11).

In a still further aspect, the heavy chain polypeptide is further combined with a variable region light chain comprising an HVR-L1, HVR-L2 and HVR-L3, wherein:

(a) the HVR-L1 sequence is RASQX$_4$X$_5$X$_6$TX$_7$X$_8$A (SEQ ID NO:12);
(b) the HVR-L2 sequence is SASX$_9$LX$_{10}$S, (SEQ ID NO:13);
(c) the HVR-L3 sequence is QQX$_{11}$X$_{12}$X$_{13}$X$_{14}$PX$_{15}$T (SEQ ID NO:14);

wherein: X$_4$ is D or V; X$_5$ is V or I; X$_6$ is S or N; X$_7$ is A or F; X$_8$ is V or L; X$_9$ is F or T; X$_{10}$ is Y or A; X$_{11}$ is Y, G, F, or S; X$_{12}$ is L, Y, F or W; X$_{13}$ is Y, N, A, T, G, F or I; X$_{14}$ is H, V, P, T or I; X$_{15}$ is A, W, R, P or T. In a still further aspect, X$_4$ is D; X$_5$ is V; X$_6$ is S; X$_7$ is A; X$_8$ is V; X$_9$ is F; X$_{10}$ is Y; X$_{11}$ is Y; X$_{12}$ is L; X$_{13}$ is Y; X$_{14}$ is H; X$_{15}$ is A.

In a still further aspect, the light chain further comprises variable region light chain framework sequences juxtaposed between the HVRs according to the formula: (LC-FR1)-(HVR-L1)-(LC-FR2)-(HVR-L2)-(LC-FR3)-(HVR-L3)-(LC-FR4). In a still further aspect, the framework sequences are derived from human consensus framework sequences. In a still further aspect, the framework sequences are VL kappa I consensus framework. In a still further aspect, at least one of the framework sequence is the following:

LC-FR1 is DIQMTQSPSSLSASVGDRVTITC (SEQ ID NO:15)
LC-FR2 is WYQQKPGKAPKLLIY (SEQ ID NO:16)
LC-FR3 is GVPSRFSGSGSGTDFTLTISSLQPEDFATYYC (SEQ ID NO:17)
LC-FR4 is FGQGTKVEIKR (SEQ ID NO:18).

In another embodiment, provided is an isolated anti-PD-L1 antibody or antigen binding fragment comprising a heavy chain and a light chain variable region sequence, wherein:

(a) the heavy chain comprises an HVR-H1, HVR-H2 and HVR-H3, wherein further:

(i) the HVR-H1 sequence is GFTFSX$_1$SWIH; (SEQ ID NO:5)
(ii) the HVR-H2 sequence is AWIX$_2$PYGGSX$_3$YYADSVKG (SEQ ID NO:6)
(iii) the HVR-H3 sequence is RHWPGGFDY, and (SEQ ID NO:7)

(b) the light chain comprises an HVR-L1, HVR-L2 and HVR-L3, wherein further:

(i) the HVR-L1 sequence is RASQX$_4$X$_5$X$_6$TX$_7$X$_8$A (SEQ ID NO:12)
(ii) the HVR-L2 sequence is SASX$_9$LX$_{10}$S; and (SEQ ID NO:13)
(iii) the HVR-L3 sequence is QQX$_{11}$X$_{12}$X$_{13}$X$_{14}$PX$_{15}$T; (SEQ ID NO:14)

wherein: X$_1$ is D or G; X$_2$ is S or L; X$_3$ is T or S; X$_4$ is D or V; X$_5$ is V or I; X$_6$ is S or N; X$_7$ is A or F; X$_8$ is V or L; X$_9$ is F or T; X$_{10}$ is Y or A; X$_{11}$ is Y, G, F, or S; X$_{12}$ is L, Y, For W; X$_{13}$ is Y, N, A, T, G, F or I; X$_{14}$ is H, V, P, T or I; X$_{15}$ is A, W, R, P or T. In a specific aspect, X$_1$ is D; X$_2$ is S and X$_3$ is T. In another aspect, X$_4$ is D; X$_5$ is V; X$_6$ is S; X$_7$ is A; X$_8$ is V; X$_9$ is F; X$_{10}$ is Y; X$_{11}$ is Y; X$_{12}$ is L; X$_{13}$ is Y; X$_{14}$ is H; X$_{15}$is A. In yet another aspect, X$_1$ is D; X$_2$ is S and X$_3$ is T, X$_4$ is D; X$_5$ is V; X$_6$ is S; X$_7$ is A; X$_8$ is V; X$_9$ is F; X$_{10}$ is Y; X$_{11}$ is Y; X$_{12}$ is L; X$_{13}$ is Y; X$_{14}$ is H and X$_{15}$ is A.

In a further aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In a still further aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and 11. In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs:15, 16, 17 and 18.

In a still further specific aspect, the antibody further comprises a human or murine constant region. In a still further aspect, the human constant region is selected from the group consisting of IgG1, IgG2, IgG2, IgG3, and IgG4. In a still further specific aspect, the human constant region is IgG1. In a still further aspect, the murine constant region is selected from the group consisting of IgG1, IgG2A, IgG2B, and IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In yet another embodiment, provided is an anti-PD-L1 antibody comprising a heavy chain and a light chain variable region sequence, wherein:

(a) the heavy chain further comprises an HVR-H1, HVR-H2 and an HVR-H3 sequence having at least 85% sequence identity to GFTFSDSWIH (SEQ ID NO:19), AWISPYGGSTYYADSVKG (SEQ ID NO:20) and RHWPGGFDY (SEQ ID NO:21), respectively, or

(b) the light chain further comprises an HVR-L1, HVR-L2 and an HVR-L3 sequence having at least 85% sequence identity to RASQDVSTAVA (SEQ ID NO:22), SASFLYS (SEQ ID NO:23) and QQYLYHPAT (SEQ ID NO:24), respectively.

In a specific aspect, the sequence identity is 86%, 87%, 88%, 89%, 90%, 91%, 92%, 93%, 94%, 95%, 96%, 97%, 98%, 99% or 100%.

In another aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In yet another aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and 11. In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs:15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In another further embodiment, provided is an isolated anti-PD-L1 antibody comprising a heavy chain and a light chain variable region sequence, wherein:

(a) the heavy chain sequence has at least 85% sequence identity to the heavy chain sequence:

EVQLVESGGGLVQPGGSLRLSCAASGFTFSDSWIHWVRQAPGKGLEWVAWISPYGGSTYYADSVKGRF

TISADTSKNTAYLQMNSLRAEDTAVYYCARRHWPGGFDYWGQGTLVTVSS (SEQ ID NO:25),

and/or

(b) the light chain sequences has at least 85% sequence identity to the light chain sequence:

DIQMTQSPSSLSASVGDRVTITCRASQDVSTAVAWYQQKPGKAPKLLIYSASFLYSGVPSRFSGSGSGTD

FTLTISSLQPEDFATYYCQQYLYHPATFGQGTKVEIKR (SEQ ID NO:4).

In a specific aspect, the sequence identity is 86%, 87%, 88%, 89%, 90%, 91%, 92%, 93%, 94%, 95%, 96%, 97%, 98%, 99% or 100%. In another aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In yet another aspect, the framework sequences are derived from human consensus framework sequences. In a further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and WGQGTLVTVSS (SEQ ID NO:27).

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs:15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect, the minimal effector function results from production in prokaryotic cells. In a still further specific aspect the minimal effector function results from an "effector-less Fc mutation" or aglycosylation. In still a further embodiment, the effector-less Fc mutation is an N297A or D265A/N297A substitution in the constant region.

In a further aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In a still further aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences is the following:

| | | |
|---|---|---|
| HC-FR1 | EVQLVESGGGLVQPGGSLRLSCAASGFTFS | (SEQ ID NO:29) |
| HC-FR2 | WVRQAPGKGLEWVA | (SEQ ID NO:30) |
| HC-FR3 | RFTISADTSKNTAYLQMNSLRAEDTAVYYCAR | (SEQ ID NO:10) |
| HC-FR4 | WGQGTLVTVSS | (SEQ ID NO:27). |

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences is the following:

| | | |
|---|---|---|
| LC-FR1 | DIQMTQSPSSLSASVGDRVTITC | (SEQ ID NO:15) |
| LC-FR2 | WYQQKPGKAPKLLIY | (SEQ ID NO:16) |
| LC-FR3 | GVPSRFSGSGSGTDFTLTISSLQPEDFATYYC | (SEQ ID NO:17) |
| LC-FR4 | FGQGTKVEIK | (SEQ ID NO:28). |

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In yet another embodiment, provided is an anti-PD-L1 antibody comprising a heavy chain and a light chain variable region sequence, wherein:

(c) the heavy chain further comprises an HVR-H1, HVR-H2 and an HVR-H3 sequence having at least 85% sequence identity to GFTFSDSWIH (SEQ ID NO:19), AWISPYGGSTYYADSVKG (SEQ ID NO:20) and RHWPGGFDY (SEQ ID NO:21), respectively, and/or
(d) the light chain further comprises an HVR-L1, HVR-L2 and an HVR-L3 sequence having at least 85% sequence identity to RASQDVSTAVA (SEQ ID NO:22), SASFLYS (SEQ ID NO:23) and QQYLYHPAT (SEQ ID NO:24), respectively.

In a specific aspect, the sequence identity is 86%, 87%, 88%, 89%, 90%, 91%, 92%, 93%, 94%, 95%, 96%, 97%, 98%, 99% or 100%.

In another aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In yet another aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and WGQGTLVTVSSASTK (SEQ ID NO:31).

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs:15, 16, 17 and 18. In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In a still further embodiment, provided is an isolated anti-PD-L1 antibody comprising a heavy chain and a light chain variable region sequence, wherein:

(a) the heavy chain sequence has at least 85% sequence identity to the heavy chain sequence:

EVQLVESGGGLVQPGGSLRLSCAASGFTFSDSWIHWVRQAPGKGLEWVAWISPYGGSTYYADSVKGRF

TISADTSKNTAYLQMNSLRAEDTAVYYCARRHWPGGFDYWGQGTLVTVSSASTK (SEQ ID NO:26),

or

(b) the light chain sequences has at least 85% sequence identity to the light chain sequence:

DIQMTQSPSSLSASVGDRVTITCRASQDVSTAVAWYQQKPGKAPKLLIYSASFLYSGVPSRFSGSGSGTD

FTLTISSLQPEDFATYYCQQYLYHPATFGQGTKVEIKR (SEQ ID NO:4).

In some embodiments, provided is an isolated anti-PD-L1 antibody comprising a heavy chain and a light chain variable region sequence, wherein the light chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to the amino acid sequence of SEQ ID NO:4. In some embodiments, provided is an isolated anti-PD-L1 antibody comprising a heavy chain and a light chain variable region sequence, wherein the heavy chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to the amino acid sequence of SEQ ID NO:26. In some embodiments, provided is an isolated anti-PD-L1 antibody comprising a heavy chain and a light chain variable region sequence, wherein the light chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the amino acid sequence of SEQ ID NO:4 and the heavy chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the amino acid sequence of SEQ ID NO:26. In some embodiments, one, two, three, four or five amino acid residues at the N-terminal of the heavy and/or light chain may be deleted, substituted or modified.

In a still further embodiment, provided is an isolated anti-PD-L1 antibody comprising a heavy chain and a light chain sequence, wherein:

(a) the heavy chain sequence has at least 85% sequence identity to the heavy chain sequence:

EVQLVESGGGLVQPGGSLRLSCAASGFTFSDSWIHWVRQAPGKGLEWVAWISPYGGSTYYADSVKGRF TISADTSKNTAYLQMNSLRAEDTAVYYCARRHWPGGFDYWGQGTLVTVSSASTKGPSVFPLAPSSKSTS GGTAALGCLVKDYFPEPVTVSWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLMISRTPEVTCVVVDVSHEDPEVKFN WYVDGVEVHNAKTKPREEQYASTYRVVSVLTVLHQDWLNGKEYKCKVSNKALPAPIEKTISKAKGQPRE PQVYTLPPSREEMTKNQVSLTCLVKGFYPSDIAVEWESNGQPENNYKTTPPVLDSDGSFFLYSKLTVDKS RWQQGNVFSCSVMHEALHNHYTQKSLSLSPG (SEQ ID NO:32),

and/or

(b) the light chain sequences has at least 85% sequence identity to the light chain sequence:

DIQMTQSPSSLSASVGDRVTITCRASQDVSTAVAWYQQKPGKAPKLLIYSASFLYSGVPSRFSGSGSGTD FTLTISSLQPEDFATYYCQQYLYHPATFGQGTKVEIKRTVAAPSVFIFPPSDEQLKSGTASVVCLLNNFYP REAKVQWKVDNALQSGNSQESVTEQDSKDSTYSLSSTLTLSKADYEKHKVYACEVTHQGLSSPVTKSFN RGEC (SEQ ID NO:33).

In some embodiments, provided is an isolated anti-PD-L1 antibody comprising a heavy chain and a light chain sequence, wherein the light chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:33. In some embodiments, provided is an isolated anti-PD-L1 antibody comprising a heavy chain and a light chain sequence, wherein the heavy chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:32. In some embodiments, provided is an isolated anti-PD-L1 antibody comprising a heavy chain and a light chain sequence, wherein the light chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:33 and the heavy chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:32.

In some embodiments, the isolated anti-PD-L1 antibody is aglycosylated. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Removal of glycosylation sites form an antibody is conveniently accomplished by altering the amino acid sequence such that one of the above-described tripeptide sequences (for N-linked glycosylation sites) is removed. The alteration may be made by substitution of an asparagine, serine or threonine residue within the glycosylation site another amino acid residue (e.g., glycine, alanine or a conservative substitution).

In any of the embodiments herein, the isolated anti-PD-L1 antibody can bind to a human PD-L1, for example a human PD-L1 as shown in UniProtKB/Swiss-Prot Accession No.Q9NZQ7.1, or a variant thereof.

*Anti-PD-1 antibodies*

In some embodiments, the anti-PD-1 antibody is MDX-1106. Alternative names for "MDX-1 106" include MDX-1106-04, ONO-4538, BMS-936558, or nivolumab. In some embodiments, the anti-PD-1 antibody is nivolumab (CAS Registry Number: 946414-94-4). In a still further embodiment, provided is an isolated anti-PD-1 antibody comprising a heavy chain variable region comprising the heavy chain variable region amino acid sequence from SEQ ID NO:1 and/or a light chain variable region comprising the light chain variable region amino acid sequence from SEQ ID NO:2. In a still further embodiment, provided is an isolated anti-PD-1 antibody comprising a heavy chain and/or a light chain sequence, wherein:

(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence:

QVQLVESGGGVVQPGRSLRLDCKASGITFSNSGMHWVRQAPGKGLEWVAVIWYDGSKRYYADSVKGR

FTISRDNSKNTLFLQMNSLRAEDTAVYYCATNDDYWGQGTLVTVSSASTKGPSVFPLAPCSRSTSESTAA

LGCLVKDYFPEPVTVSWNSGALTSGVHTFPAVLQSSGLYSLSSVVTVPSSSLGTKTYTCNVDHKPSNTK

VDKRVESKYGPPCPPCPAPEFLGGPSVFLFPPKPKDTLMISRTPEVTCVVVDVSQEDPEVQFNWYVDGV

EVHNAKTKPREEQFNSTYRVVSVLTVLHQDWLNGKEYKCKVSNKGLPSSIEKTISKAKGQPREPQVYTLP

PSQEEMTKNQVSLTCLVKGFYPSDIAVEWESNGQPENNYKTTPPVLDSDGSFFLYSRLTVDKSRWQEG

NVFSCSVMHEALHNHYTQKSLSLSLGK (SEQ ID NO:1),

and

(b) the light chain sequences has at least 85%, at least 90%, at least 91%, at least 92%, at least 93%, at least 94%, at least 95%, at least 96%, at least 97%, at least 98%, at least 99% or 100% sequence identity to the light chain sequence:

EIVLTQSPATLSLSPGERATLSCRASQSVSSYLAWYQQKPGQAPRLLIYDASNRATGIPARFSGSGSGTD

FTLTISSLEPEDFAVYYCQQSSNWPRTFGQGTKVEIKRTVAAPSVFIFPPSDEQLKSGTASVVCLLNNFYP

REAKVQWKVDNALQSGNSQESVTEQDSKDSTYSLSSTLTLSKADYEKHKVYACEVTHQGLSSPVTKSFN

RGEC (SEQ ID NO:2).

*Nucleic acids, host cells, and vectors*

In a still further embodiment, provided is an isolated nucleic acid encoding any of the antibodies described herein. In some embodiments, the nucleic acid further comprises a vector suitable for expression of the nucleic acid encoding any of the previously described anti-PD-L1 antibodies. In a still further specific aspect, the vector is in a host cell suitable for expression of the nucleic acid. In a still further specific aspect, the host cell is a eukaryotic cell or a prokaryotic cell. In a still further specific aspect, the eukaryotic cell is a mammalian cell, such as Chinese hamster ovary (CHO) cell.

The antibody or antigen binding fragment thereof, may be made using methods known in the art, for example, by a process comprising culturing a host cell containing nucleic acid encoding any of the previously described anti-PD-L1 antibodies or antigen-binding fragment in a form suitable for expression, under conditions suitable to produce such antibody or fragment, and recovering the antibody or fragment, or according to any method described below in Section VII.

## VII. Antibody Preparation

The antibodies described herein are prepared using techniques available in the art for generating antibodies, exemplary methods of which are described in more detail in the following sections.

The antibody is directed against an antigen of interest (e.g., PD-L1 (such as a human PD-L1), PD1 (such as human PD-L1), PD-L2 (such as human PD-L2), etc.). Preferably, the antigen is a biologically important polypeptide and administration of the antibody to a mammal suffering from a disorder can result in a therapeutic benefit in that mammal.

In certain embodiments, an antibody provided herein has a dissociation constant (Kd) of $\leq 1\mu M$, $\leq 150$ nM, $\leq 100$ nM, $\leq 50$ nM, $\leq 10$ nM, $\leq 1$ nM, $\leq 0.1$ nM, $\leq 0.01$ nM, or 5 0.001 nM (e.g., $10^{-8}$ M or less, e.g., from $10^{-8}$ M to $10^{-13}$ M, e.g., from $10^{-9}$ M to $10^{-13}$ M).

In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g.,

Chen et al., J. Mol. Biol. 293:865-881(1999)). To establish conditions for the assay, MICROTITER® multi-well plates (Thermo Scientific) are coated overnight with 5 $\mu$g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest. The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1 % polysorbate 20 (TWEEN-20®) in PBS. When the plates have dried, 150 $\mu$l/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE®-2000 or a BIACORE®-3000 (BIAcore, Inc., Piscataway, NJ) at 25°C with immobilized antigen CM5 chips at approximately 10 response units (RU). Briefly, carboxymethylated dextran biosensor chips (CM5, BIACORE, Inc.) are activated with *N*-ethyl-*N*'-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 $\mu$g/ml (approximately 0.2 $\mu$M) before injection at a flow rate of 5 $\mu$l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 $\mu$l/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$. See, e.g., Chen et al., J. Mol. Biol. 293:865-881 (1999). If the on-rate exceeds $10^6 M^{-1} s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

*(i) Antigen Preparation*

Soluble antigens or fragments thereof, optionally conjugated to other molecules, can be used as immunogens for generating antibodies. For transmembrane molecules, such as receptors, fragments of these (e.g., the extracellular domain of a receptor) can be used as the immunogen. Alternatively, cells expressing the transmembrane molecule can be used as the immunogen. Such cells can be derived from a natural source (e.g., cancer cell lines) or may be cells which have been transformed by recombinant techniques to express the transmembrane molecule. Other antigens and forms thereof useful for preparing antibodies will be apparent to those in the art.

*(ii) Certain Antibody-Based Methods*

Polyclonal antibodies are preferably raised in animals by multiple subcutaneous (s.c.) or intraperitoneal (i.p.) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl$_2$, or R$^1$N=C=NR, where R and R$^1$ are different alkyl groups.

Animals are immunized against the antigen, immunogenic conjugates, or derivatives by combining, e.g., 100 $\mu$g or 5 $\mu$g of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies of the invention can be made using the hybridoma method first described by Kohler et al., Nature, 256:495 (1975), and further described, for example, in Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981), and Ni, Xiandai Mianyixue, 26(4):265-268 (2006) regarding human-human hybridomas. Additional methods include those described, for example, in U.S. Pat. No. 7,189,826 regarding production of monoclonal human natural IgM antibodies from hybridoma cell lines. Human hybridoma technology (Trioma technology) is described in Vollmers and Brandlein, Histology and Histopathology, 20(3):927-937 (2005) and Vollmers and Brandlein, Methods and Findings in Experimental and Clinical Pharmacology, 27(3):185-91 (2005).

For various other hybridoma techniques, see, for example, U.S. Patent Publication Nos. 2006/258841; 2006/183887 (fully human antibodies), 2006/059575; 2005/287149; 2005/100546; and 2005/026229; and U.S. Pat. Nos. 7,078,492 and 7,153,507. An exemplary protocol for producing monoclonal antibodies using the hybridoma method is described as follows. In one embodiment, a mouse or other appropriate host animal, such as a hamster, is immunized to elicit lymphocytes that produce or are capable of producing antibodies that will specifically bind to the protein used for immunization. Antibodies are raised in animals by multiple subcutaneous (SC) or intraperitoneal (IP) injections of a polypeptide of the invention or a fragment thereof, and an adjuvant, such as monophosphoryl lipid A (MPL)/trehalose dicrynomycolate (TDM) (Ribi Immunochem. Research, Inc., Hamilton, MT). A polypeptide of the invention (e.g., antigen) or a fragment thereof may be prepared using methods well known in the art, such as recombinant methods, some of which are further described herein. Serum from immunized animals is assayed for anti-antigen antibodies, and booster immunizations are optionally administered. Lymphocytes from animals producing anti-antigen antibodies are isolated. Alternatively, lymphocytes may be immunized *in vitro.*

Lymphocytes are then fused with myeloma cells using a suitable fusing agent, such as polyethylene glycol, to form a hybridoma cell. See, e.g., Goding, Monoclonal Antibodies: Principles and Practice, pp. 59-103 (Academic Press, 1986). Myeloma cells may be used that fuse efficiently, support stable high-level production of antibody by the selected antibody-producing cells, and are sensitive to a medium such as HAT medium. Exemplary myeloma cells include, but are not limited to, murine myeloma lines, such as those derived from MOPC-21 and MPC-11 mouse tumors available from the Salk Institute Cell Distribution Center, San Diego, Calif. USA, and SP-2 or X63-Ag8-653 cells available from the American Type Culture Collection, Rockville, Md. USA. Human myeloma and mouse-human heteromyeloma cell lines also have been described for the production of human monoclonal antibodies (Kozbor, J. Immunol., 133:3001 (1984; Brodeur et al., Monoclonal Antibody Production Techniques and Applications, pp. 51-63 (Marcel Dekker, Inc., New York, 1987)).

The hybridoma cells thus prepared are seeded and grown in a suitable culture medium, e.g., a medium that contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells. Preferably, serum-free hybridoma cell culture methods are used to reduce use of animal-derived serum such as fetal bovine serum, as described, for example, in Even et al., Trends in Biotechnology, 24(3), 105-108 (2006).

Oligopeptides as tools for improving productivity of hybridoma cell cultures are described in Franek, Trends in Monoclonal Antibody Research, 111-122 (2005). Specifically, standard culture media are enriched with certain amino acids (alanine, serine, asparagine, proline), or with protein hydrolyzate fractions, and apoptosis may be significantly suppressed by synthetic oligopeptides, constituted of three to six amino acid residues. The peptides are present at millimolar or higher concentrations.

Culture medium in which hybridoma cells are growing may be assayed for production of monoclonal antibodies that bind to an antibody of the invention. The binding specificity of monoclonal antibodies produced by hybridoma cells may be determined by immunoprecipitation or by an *in vitro* binding assay, such as radioimmunoassay (RIA) or enzyme-linked immunosorbent assay (ELISA). The binding affinity of the monoclonal antibody can be determined, for example, by Scatchard analysis. See, e.g., Munson et al., Anal. Biochem., 107:220 (1980).

After hybridoma cells are identified that produce antibodies of the desired specificity, affinity, and/or activity, the clones may be subcloned by limiting dilution procedures and grown by standard methods. See, e.g., Goding, *supra.* Suitable culture media for this purpose include, for example, D-MEM or RPMI-1640 medium. In addition, hybridoma cells may be grown *in vivo* as ascites tumors in an animal. Monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography. One procedure for isolation of proteins from hybridoma cells is described in US 2005/176122 and U.S. Pat. No. 6,919,436. The method includes using minimal salts, such as lyotropic salts, in the binding process and preferably also using small amounts of organic solvents in the elution process.

*(iii) Library-Derived Antibodies*

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Additional methods are reviewed, e.g., in Hoogenboom et al., in Methods in Molecular Biology 178:1-37 (O'Brien et al., ed., Human Press, Totowa, NJ, 2001) and further described, e.g., in McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

*(iv) Chimeric, Humanized and Human Antibodies*

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), for example, to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'1 Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, for example, U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

*(v) Antibody Fragments*

Antibody fragments may be generated by traditional means, such as enzymatic digestion, or by recombinant techniques. In certain circumstances there are advantages of using antibody fragments, rather than whole antibodies. The smaller size of the fragments allows for rapid clearance, and may lead to improved access to solid tumors. For a review of certain antibody fragments, see Hudson et al. (2003) Nat. Med. 9:129-134.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries discussed above. Alternatively, Fab'-SH fragments can be directly recovered from *E. coli* and chemically coupled to form F(ab')$_2$ fragments (Carter et al., Bio/Technology 10:163-167 (1992)). According to another approach, F(ab')$_2$ fragments can be isolated directly from recombinant host cell culture. Fab and F(ab')$_2$ fragment with increased *in vivo* half-life comprising salvage receptor binding epitope residues are described in U.S. Pat. No. 5,869,046. Other techniques for the production of antibody fragments will be apparent to the skilled practitioner. In certain embodiments, an antibody is a single chain Fv fragment (scFv). See, for example, WO 93/16185; U.S. Pat. Nos. 5,571,894; and 5,587,458. Fv and scFv are the only species with intact combining sites that are devoid of constant regions; thus, they may be suitable for reduced nonspecific binding during in vivo use. scFv fusion proteins may be constructed to yield fusion of an effector protein at either the amino or the carboxy terminus of an scFv. See Antibody Engineering, ed. Borrebaeck, *supra.* The antibody fragment may also be a "linear antibody," e.g., as described in U.S. Pat. No. 5,641,870, for example. Such linear antibodies may be monospecific or bispecific.

*(vi) Multispecific Antibodies*

Multispecific antibodies have binding specificities for at least two different epitopes, where the epitopes are usually from different antigens. While such molecules normally will only bind two different epitopes (i.e., bispecific antibodies, BsAbs), antibodies with additional specificities such as trispecific antibodies are encompassed by this expression when used herein. Bispecific antibodies can be prepared as full-length antibodies or antibody fragments (e.g., F(ab')$_2$ bispecific antibodies).

Methods for making bispecific antibodies are known in the art. Traditional production of full length bispecific antibodies is based on the coexpression of two immunoglobulin heavy chain-light chain pairs, where the two chains have different specificities (see, e.g., Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

One approach known in the art for making bispecific antibodies is the "knobs-into-holes" or "protuberance-into-cavity" approach (see, e.g., US Pat. No. 5,731,168). In this approach, two immunoglobulin polypeptides (e.g., heavy chain polypeptides) each comprise an interface. An interface of one immunoglobulin polypeptide interacts with a corresponding interface on the other immunoglobulin polypeptide, thereby allowing the two immunoglobulin polypeptides to associate. These interfaces may be engineered such that a "knob" or "protuberance" (these terms may be used interchangeably herein) located in the interface of one immunoglobulin polypeptide corresponds with a "hole" or "cavity" (these terms may be used interchangeably herein) located in the interface of the other immunoglobulin polypeptide. In some embodiments, the hole is of identical or similar size to the knob and suitably positioned such that when the two interfaces interact, the knob of one interface is positionable in the corresponding hole of the other interface. Without wishing to be bound to theory, this is thought to stabilize the heteromultimer and favor formation of the heteromultimer over other species, for example homomultimers. In some embodiments, this approach may be used to promote the heteromultimerization of two different immunoglobulin polypeptides, creating a bispecific antibody comprising two immunoglobulin polypeptides with binding specificities for different epitopes.

In some embodiments, a knob may be constructed by replacing a small amino acid side chain with a larger side chain. In some embodiments, a hole may be constructed by replacing a large amino acid side chain with a smaller side chain. Knobs or holes may exist in the original interface, or they may be introduced synthetically. For example, knobs or holes may be introduced synthetically by altering the nucleic acid sequence encoding the interface to replace at least one "original" amino acid residue with at least one "import" amino acid residue. Methods for altering nucleic acid sequences may include standard molecular biology techniques well known in the art. The side chain volumes of various amino acid residues are shown in the following table. In some embodiments, original residues have a small side chain volume (e.g., alanine, asparagine, aspartic acid, glycine, serine, threonine, or valine), and import residues for forming a knob are naturally occurring amino acids and may include arginine, phenylalanine, tyrosine, and tryptophan. In some embodiments, original residues have a large side chain volume (e.g., arginine, phenylalanine, tyrosine, and tryptophan), and import residues for forming a hole are naturally occurring amino acids and may include alanine, serine, threonine, and valine.

**Table 1. Properties of amino acid residues**

| Amino acid | One-letter abbreviation | Mass[a] (daltons) | Volume[b] ($Å^3$) | Accessible surface area[c] ($Å^2$) |
|---|---|---|---|---|
| Alanine (Ala) | A | 71.08 | 88.6 | 115 |
| Arginine (Arg) | R | 156.20 | 173.4 | 225 |
| Asparagine (Asn) | N | 114.11 | 117.7 | 160 |
| Aspartic Acid (Asp) | D | 115.09 | 111.1 | 150 |
| Cysteine (Cys) | C | 103.14 | 108.5 | 135 |
| Glutamine (Gln) | Q | 128.14 | 143.9 | 180 |
| Glutamic Acid (Glu) | E | 129.12 | 138.4 | 190 |
| Glycine (Gly) | G | 57.06 | 60.1 | 75 |
| Histidine (His) | H | 137.15 | 153.2 | 195 |
| Isoleucine (Ile) | I | 113.17 | 166.7 | 175 |
| Leucine (Leu) | L | 113.17 | 166.7 | 170 |
| Lysine (Lys) | K | 128.18 | 168.6 | 200 |
| Methionine (Met) | M | 131.21 | 162.9 | 185 |
| Phenylalanine (Phe) | F | 147.18 | 189.9 | 210 |
| Proline (Pro) | P | 97.12 | 122.7 | 145 |
| Serine (Ser) | S | 87.08 | 89.0 | 115 |
| Threonine (Thr) | T | 101.11 | 116.1 | 140 |
| Tryptophan (Trp) | W | 186.21 | 227.8 | 255 |
| Tyrosine (Tyr) | Y | 163.18 | 193.6 | 230 |

(continued)

| Amino acid | One-letter abbreviation | Mass[a] (daltons) | Volume[b] (Å³) | Accessible surface area[c] (Å²) |
|---|---|---|---|---|
| Valine (Val) | V | 99.14 | 140.0 | 155 |

[a]Molecular weight of amino acid minus that of water. Values from Handbook of Chemistry and Physics, 43rd ed. Cleveland, Chemical Rubber Publishing Co., 1961.
[b]Values from A.A. Zamyatnin, Prog. Biophys. Mol. Biol. 24:107-123, 1972.
[c]Values from C. Chothia, J. Mol. Biol. 105:1-14, 1975. The accessible surface area is defined in Figures 6-20 of this reference.

In some embodiments, original residues for forming a knob or hole are identified based on the three-dimensional structure of the heteromultimer. Techniques known in the art for obtaining a three-dimensional structure may include X-ray crystallography and NMR. In some embodiments, the interface is the CH3 domain of an immunoglobulin constant domain. In these embodiments, the CH3/CH3 interface of human IgG$_1$ involves sixteen residues on each domain located on four anti-parallel β-strands. Without wishing to be bound to theory, mutated residues are preferably located on the two central anti-parallel β-strands to minimize the risk that knobs can be accommodated by the surrounding solvent, rather than the compensatory holes in the partner CH3 domain. In some embodiments, the mutations forming corresponding knobs and holes in two immunoglobulin polypeptides correspond to one or more pairs provided in the following table.

**Table 2. Exemplary sets of corresponding knob-and hole-forming mutations**

| CH3 of first immunoglobulin | CH3 of second immunoglobulin |
|---|---|
| T366Y | Y407T |
| T366W | Y407A |
| F405A | T394W |
| Y407T | T366Y |
| T366Y:F405A | T394W:Y407T |
| T366W:F405W | T394S:Y407A |
| F405W:Y407A | T366W:T394S |
| F405W | T394S |

Mutations are denoted by the original residue, followed by the position using the Kabat numbering system, and then the import residue (all residues are given in single-letter amino acid code). Multiple mutations are separated by a colon.

In some embodiments, an immunoglobulin polypeptide comprises a CH3 domain comprising one or more amino acid substitutions listed in Table 2 above. In some embodiments, a bispecific antibody comprises a first immunoglobulin polypeptide comprising a CH3 domain comprising one or more amino acid substitutions listed in the left column of Table 2, and a second immunoglobulin polypeptide comprising a CH3 domain comprising one or more corresponding amino acid substitutions listed in the right column of Table 2.

Following mutation of the DNA as discussed above, polynucleotides encoding modified immunoglobulin polypeptides with one or more corresponding knob- or hole-forming mutations may be expressed and purified using standard recombinant techniques and cell systems known in the art. See, e.g., U.S. Pat. Nos. 5,731,168; 5,807,706; 5,821,333; 7,642,228; 7,695,936; 8,216,805; U.S. Pub. No. 2013/0089553; and Spiess et al., Nature Biotechnology 31: 753-758, 2013. Modified immunoglobulin polypeptides may be produced using prokaryotic host cells, such as *E. coli,* or eukaryotic host cells, such as CHO cells. Corresponding knob-and-hole-bearing immunoglobulin polypeptides may be expressed in host cells in co-culture and purified together as a heteromultimer, or they may be expressed in single cultures, separately purified, and assembled *in vitro.* In some embodiments, two strains of bacterial host cells (one expressing an immunoglobulin polypeptide with a knob, and the other expressing an immunoglobulin polypeptide with a hole) are co-cultured using standard bacterial culturing techniques known in the art. In some embodiments, the two strains may be mixed in a specific ratio, e.g., so as to achieve equal expression levels in culture. In some embodiments, the two strains may be mixed in a 50:50, 60:40, or 70:30 ratio. After polypeptide expression, the cells may be lysed together, and protein may be extracted. Standard techniques known in the art that allow for measuring the abundance of homo-multimeric vs. hetero-multimeric species may include size exclusion chromatography. In some embodiments, each modified immunoglobulin polypeptide is expressed separately using standard recombinant techniques, and they may be assembled together *in vitro.* Assembly may be achieved, for example, by purifying each modified immunoglobulin polypeptide, mixing and incubating them together in equal mass, reducing disulfides (e.g., by treating with dithiothreitol), concentrating, and reoxidizing the polypeptides. Formed bispecific antibodies may be purified using standard techniques including cation-exchange chromatography and measured using standard techniques including size exclusion chromatography. For a more detailed description of these methods, see Speiss et al., Nat. Biotechnol. 31:753-8, 2013. In some embodiments, modified immunoglobulin polypeptides may be expressed separately in CHO cells and assembled *in vitro* using the methods described above.

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is typical to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in WO96/27011, the interface between a pair of antibody molecules can be engineered to maximize the percentage of heterodimers which are recovered from recombinant cell culture. One interface comprises at least a part of the $C_H3$ domain of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')$_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med., 175: 217-225 (1992) describe the production of a fully humanized bispecific antibody F(ab')$_2$ molecule. Each Fab' fragment was separately secreted from *E. coli* and subjected to directed chemical coupling in vitro to form the bispecific antibody.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See Gruber et al, J. Immunol, 152:5368 (1994).

Another technique for making bispecific antibody fragments is the "bispecific T cell engager" or BiTE® approach (see, e.g., WO2004/106381, WO2005/061547, WO2007/042261, and WO2008/119567). This approach utilizes two antibody variable domains arranged on a single polypeptide. For example, a single polypeptide chain includes two single chain Fv (scFv) fragments, each having a variable heavy chain ($V_H$) and a variable light chain ($V_L$) domain separated by a polypeptide linker of a length sufficient to allow intramolecular association between the two domains. This single polypeptide further includes a polypeptide spacer sequence between the two scFv fragments. Each scFv recognizes a different epitope, and these epitopes may be specific for different cell types, such that cells of two different cell types are brought into close proximity or tethered when each scFv is engaged with its cognate epitope. One particular embodiment of this approach includes a scFv recognizing a cell-surface antigen expressed by an immune cell, e.g., a CD3 polypeptide on a T cell, linked to another scFv that recognizes a cell-surface antigen expressed by a target cell, such as a malignant or tumor cell.

As it is a single polypeptide, the bispecific T cell engager may be expressed using any prokaryotic or eukaryotic cell expression system known in the art, e.g., a CHO cell line. However, specific purification techniques (see, e.g., EP1691833) may be necessary to separate monomeric bispecific T cell engagers from other multimeric species, which may have biological activities other than the intended activity of the monomer. In one exemplary purification scheme, a solution containing secreted polypeptides is first subjected to a metal affinity chromatography, and polypeptides are eluted with a gradient of imidazole concentrations. This eluate is further purified using anion exchange chromatography, and polypeptides are eluted using with a gradient of sodium chloride concentrations. Finally, this eluate is subjected to size exclusion chromatography to separate monomers from multimeric species.

Antibodies with more than two valencies are contemplated. For example, trispecific antibodies can be prepared. See, e.g., Tuft et al. J. Immunol. 147: 60 (1991).

*(vii) Single-Domain Antibodies*

In some embodiments, an antibody of the invention is a single-domain antibody. A single-domain antibody is a single polypeptide chain comprising all or a portion of the heavy chain variable domain or all or a portion of the light chain variable domain of an antibody. In certain embodiments, a single-domain antibody is a human single-domain antibody (Domantis, Inc., Waltham, Mass.; *see,* e.g., U.S. Pat. No. 6,248,516 B1). In one embodiment, a single-domain antibody consists of all or a portion of the heavy chain variable domain of an antibody.

*(viii) Antibody Variants*

In some embodiments, amino acid sequence modification(s) of the antibodies described herein are contemplated. For example, it may be desirable to improve the binding affinity and/or other biological properties of the antibody. Amino acid sequence variants of the antibody may be prepared by introducing appropriate changes into the nucleotide sequence encoding the antibody, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

*(ix) Substitution, Insertion, and Deletion Variants*

In certain embodiments, antibody variants having one or more amino acid substitutions are provided. Sites of interest for substitutional mutagenesis include the HVRs and FRs. Conservative substitutions are shown in Table 3 under the heading of "conservative substitutions." More substantial changes are provided in Table 3 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**Table 3. Exemplary Substitutions**

| Original Residue | Exemplary Substitutions | Conservative Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

a. hydrophobic: Norleucine, Met, Ala, Val, Leu, Ile;
b. neutral hydrophilic: Cys, Ser, Thr, Asn, Gln;
c. acidic: Asp, Glu;
d. basic: His, Lys, Arg;
e. residues that influence chain orientation: Gly, Pro;
f. aromatic: Trp, Tyr, Phe.

Non-conservative substitutions will entail exchanging a member of one of these classes for another class.

One type of substitutional variant involves substituting one or more hypervariable region residues of a parent antibody (e.g., a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g., binding affinity).

Alterations (e.g., substitutions) may be made in HVRs, for example, to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001)). In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g., for ADEPT) or a polypeptide which increases the serum half-life of the antibody.

*(x) Glycosylation variants*

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided comprising an Fc region wherein a carbohydrate structure attached to the Fc region has reduced fucose or lacks fucose, which may improve ADCC function. Specifically, antibodies are contemplated herein that have reduced fusose relative to the amount of fucose on the same antibody produced in a wild-type CHO cell. That is, they are characterized by having a lower amount of fucose than they would otherwise have if produced by native CHO cells (e.g., a CHO cell that produce a native glycosylation pattern, such as, a CHO cell containing a native FUT8 gene). In certain embodiments, the antibody is one wherein less than about 50%, 40%, 30%, 20%, 10%, or 5% of the N-linked glycans thereon comprise fucose. For example, the amount of fucose in such an antibody may be from 1% to 80%, from 1% to 65%, from 5% to 65% or from 20% to 40%. In certain embodiments, the antibody is one wherein none of the N-linked glycans thereon comprise fucose, i.e., wherein the antibody is completely without fucose, or has no fucose or is afucosylated. The amount of fucose is determined by calculating the average amount of fucose within the sugar chain at Asn297, relative to the sum of all glycostructures attached to Asn 297 (e.g., complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol.

336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al., Arch. Biochem. Biophys. 249:533-545 (1986); US Pat Appl No US 2003/0157108 A1; and WO 2004/056312 A1, especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibody variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878; US Patent No. 6,602,684; US 2005/0123546, and Ferrara et al., Biotechnology and Bioengineering, 93(5): 851-861 (2006). Antibody variants with at least one galactose residue in the oligosaccharide attached to the Fc region are also provided. Such antibody variants may have improved CDC function. Such antibody variants are described, e.g., in WO 1997/30087; WO 1998/58964; and WO 1999/22764.

In certain embodiments, the antibody variants comprising an Fc region described herein are capable of binding to an FcγRIII. In certain embodiments, the antibody variants comprising an Fc region described herein have ADCC activity in the presence of human effector cells or have increased ADCC activity in the presence of human effector cells compared to the otherwise same antibody comprising a human wild-type IgG1Fc region.

*(xi) Fc region variants*

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g., a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII, and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g., Hellstrom et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo,* e.g., in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg et al., Blood 101:1045-1052 (2003); and Cragg et al, Blood 103:2738-2743 (2004)). FcRn binding and *in vivo* clearance/half life determinations can also be performed using methods known in the art (see, e.g., Petkova et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues). In an exemplary embodiment, the antibody comprising the following amino acid substitutions in its Fc region: S298A, E333A, and K334A.

In some embodiments, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.)). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826). See also Duncan & Winter, Nature 322:738-40 (1988); U.S. Patent No. 5,648,260; U.S. Patent No. 5,624,821; and WO 94/29351 concerning other examples of Fc region variants.

*(xii) Antibody Derivatives*

The antibodies of the invention can be further modified to contain additional nonproteinaceous moieties that are known in the art and readily available. In certain embodiments, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

*(xiii) Vectors, Host Cells, and Recombinant Methods*

Antibodies may also be produced using recombinant methods. For recombinant production of an anti-antigen antibody, nucleic acid encoding the antibody is isolated and inserted into a replicable vector for further cloning (amplification of the DNA) or for expression. DNA encoding the antibody may be readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence.

*(a) Signal Sequence Component*

An antibody of the invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (e.g., cleaved by a signal peptidase) by the host cell. For prokaryotic host cells that do not recognize and process a native antibody signal sequence, the signal sequence is substituted by a prokaryotic signal sequence selected, for example, from the group of the alkaline phosphatase, penicillinase, Ipp, or heat-stable enterotoxin II leaders. For yeast secretion the native signal sequence may be substituted by, e.g., the yeast invertase leader, a factor leader (including *Saccharomyces* and *Kluyveromyces* α-factor leaders), or acid phosphatase leader, the *C. albicans* glucoamylase leader, or the signal described in WO 90/13646. In mammalian cell expression, mammalian signal sequences as well as viral secretory leaders, for example, the herpes simplex gD signal, are available.

*(b) Origin of Replication*

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter.

*(c) Selection Gene Component*

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up antibody-encoding nucleic acid, such as DHFR, glutamine synthetase (GS), thymidine kinase, metallothionein-I and -II, preferably primate metallothionein genes, adenosine deaminase, ornithine decarboxylase, *etc.*

For example, cells transformed with the DHFR gene are identified by culturing the transformants in a culture medium containing methotrexate (Mtx), a competitive antagonist of DHFR. Under these conditions, the DHFR gene is amplified along with any other co-transformed nucleic acid. A Chinese hamster ovary (CHO) cell line deficient in endogenous DHFR activity (e.g., ATCC CRL-9096) may be used.

Alternatively, cells transformed with the GS gene are identified by culturing the transformants in a culture medium containing L-methionine sulfoximine (Msx), an inhibitor of GS. Under these conditions, the GS gene is amplified along with any other co-transformed nucleic acid. The GS selection/amplification system may be used in combination with the DHFR selection/amplification system described above.

Alternatively, host cells (particularly wild-type hosts that contain endogenous DHFR) transformed or co-transformed with DNA sequences encoding an antibody of interest, wild-type DHFR gene, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

A suitable selection gene for use in yeast is the *trp*1 gene present in the yeast plasmid YRp7 (Stinchcomb et al., Nature, 282:39 (1979)). The *trp*1 gene provides a selection marker for a mutant strain of yeast lacking the ability to grow in tryptophan, for example, ATCC No. 44076 or PEP4-1. Jones, Genetics, 85:12 (1977). The presence of the *trp*1 lesion in the yeast host cell genome then provides an effective environment for detecting transformation by growth in the absence of tryptophan. Similarly, *Leu*2-deficient yeast strains (ATCC 20,622 or 38,626) are complemented by known plasmids bearing the *Leu2* gene.

In addition, vectors derived from the 1.6 μm circular plasmid pKD1 can be used for transformation of *Kluyveromyces* yeasts. Alternatively, an expression system for large-scale production of recombinant calf chymosin was reported for *K. lactis.* See, e.g., Van den Berg, Bio/Technology, 8:135 (1990). Stable multi-copy expression vectors for secretion of mature recombinant human serum albumin by industrial strains of *Kluyveromyces* have also been disclosed. Fleer et al., Bio/Technology, 9:968-975 (1991).

*(d) Promoter Component*

Expression and cloning vectors generally contain a promoter that is recognized by the host organism and is operably linked to nucleic acid encoding an antibody. Promoters suitable for use with prokaryotic hosts include the *pho*A promoter, β-lactamase and lactose promoter systems, alkaline phosphatase promoter, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding an antibody.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoter sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Antibody transcription from vectors in mammalian host cells can be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus, Simian Virus 40 (SV40), or from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., Nature 297:598-601 (1982) on expression of human $\beta$-interferon cDNA in mouse cells under the control of a thymidine kinase promoter from herpes simplex virus. Alternatively, the Rous Sarcoma Virus long terminal repeat can be used as the promoter.

*(e) Enhancer Element Component*

Transcription of a DNA encoding an antibody of this invention by higher eukaryotes is often increased by inserting an enhancer sequence into the vector. Many enhancer sequences are now known from mammalian genes (globin, elastase, albumin, $\alpha$-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody-encoding sequence, but is preferably located at a site 5' from the promoter.

*(f) Transcription Termination Component*

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding antibody. One useful transcription termination component is the bovine growth hormone polyadenylation region. See WO94/11026 and the expression vector disclosed therein.

*(g) Selection and Transformation of Host Cells*

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 Apr. 1989), *Pseudomonas* such as *P. aeruginosa,* and *Streptomyces.* One preferred *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

Full length antibody, antibody fusion proteins, and antibody fragments can be produced in bacteria, in particular when glycosylation and Fc effector function are not needed, such as when the therapeutic antibody is conjugated to a cytotoxic agent (e.g., a toxin) that by itself shows effectiveness in tumor cell destruction. Full length antibodies have greater half-life in circulation. Production in *E. coli* is faster and more cost efficient. For expression of antibody fragments and polypeptides in bacteria, see, e.g., U.S. Pat. No. 5,648,237 (Carter et al.), U.S. Pat. No. 5,789,199 (Joly et al.), U.S. Pat. No. 5,840,523 (Simmons et al.), which describes translation initiation region (TIR) and signal sequences for optimizing expression and secretion. See also Charlton, Methods in Molecular Biology, Vol. 248 (B. K. C. Lo, ed., Humana Press, Totowa, N.J., 2003), pp. 245-254, describing expression of antibody fragments in *E. coli.* After expression, the antibody may be isolated from the *E. coli* cell paste in a soluble fraction and can be purified through, e.g., a protein A or G column depending on the isotype. Final purification can be carried out similar to the process for purifying antibody expressed e.g., in CHO cells.

In addition to prokaryotes, eukaryotic microbes such as filamentous fungi or yeast are suitable cloning or expression hosts for antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, e.g., *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K. marxianus; yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis;* and filamentous fungi such as, e.g., *Neurospora, Penicillium, Tolypocladium,* and *Aspergillus* hosts such as *A. nidulans* and *A. niger.* For a review discussing the use of yeasts and filamentous fungi for the production of therapeutic proteins, see, e.g., Gerngross, Nat. Biotech. 22:1409-1414 (2004).

Certain fungi and yeast strains may be selected in which glycosylation pathways have been "humanized," resulting in the production of an antibody with a partially or fully human glycosylation pattern. See, e.g., Li et al., Nat. Biotech. 24:210-215 (2006) (describing humanization of the glycosylation pathway in *Pichia pastoris*); and Gerngross et al., *supra.*

Suitable host cells for the expression of glycosylated antibody are also derived from multicellular organisms (invertebrates and vertebrates). Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the invention, particularly for transfection of *Spodoptera frugiperda* cells.

Plant cell cultures of cotton, corn, potato, soybean, petunia, tomato, duckweed (*Leninaceae*), alfalfa (*M. truncatula*), and tobacco can also be utilized as hosts. See, e.g., U.S. Pat. Nos. 5,959,177, 6,040,498, 6,420,548, 7,125,978, and 6,417,429 (describing PLANTIBODIES™ technology for producing antibodies in transgenic plants).

Vertebrate cells may be used as hosts, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2). Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as NS0 and Sp2/0. For a review of certain mammalian host cell lines suitable for antibody production, see, e.g., Yazaki and Wu, Methods in Molecular Biology, Vol. 248 (B. K. C. Lo, ed., Humana Press, Totowa, N.J., 2003), pp. 255-268.

Host cells are transformed with the above-described expression or cloning vectors for antibody production and cultured in conventional nutrient media modified as appropriate for inducing promoters, selecting transformants, or amplifying the genes encoding the desired sequences.

*(h) Culturing the Host Cells*

The host cells used to produce an antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

*(xiv) Purification of Antibody*

When using recombinant techniques, the antibody can be produced intracellularly, in the periplasmic space, or directly secreted into the medium. If the antibody is produced intracellularly, as a first step, the particulate debris, either host cells or lysed fragments, are removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, hydrophobic interaction chromatography, gel electrophoresis, dialysis, and affinity chromatography, with affinity chromatography being among one of the typically preferred purification steps. The suitability of protein A as an affinity ligand depends on the species and isotype of any immunoglobulin Fc domain that is present in the antibody. Protein A can be used to purify antibodies that are based on human $\gamma$1, $\gamma$2, or $\gamma$4 heavy chains (Lindmark et al., J. Immunol. Meth. 62:1-13 (1983)). Protein G is recommended for all mouse isotypes and for human $\gamma$3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a $C_H3$ domain, the Bakerbond ABX™ resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

In general, various methodologies for preparing antibodies for use in research, testing, and clinical are well-established in the art, consistent with the above-described methodologies and/or as deemed appropriate by one skilled in the art for a particular antibody of interest.

*(xv) Selecting Biologically Active Antibodies*

Antibodies produced as described above may be subjected to one or more "biological activity" assays to select an antibody with beneficial properties from a therapeutic perspective or selecting formulations and conditions that retain biological activity of the antibody. The antibody may be tested for its ability to bind the antigen against which it was raised. For example, methods known in the art (such as ELISA, Western Blot, etc.) may be used.

For example, for an anti-PD-L1 antibody, the antigen binding properties of the antibody can be evaluated in an assay that detects the ability to bind to PD-L1. In some embodiments, the binding of the antibody may be determined by saturation binding; ELISA; and/or competition assays (e.g., RIA's), for example. Also, the antibody may be subjected to other biological activity assays, e.g., in order to evaluate its effectiveness as a therapeutic. Such assays are known in the art and depend on the target antigen and intended use for the antibody. For example, the biological effects of PD-L1 blockade by the antibody can be assessed in CD8+T cells, a lymphocytic choriomeningitis virus (LCMV) mouse model and/or a syngeneic tumor model e.g., as described in US Patent 8,217,149.

To screen for antibodies which bind to a particular epitope on the antigen of interest (e.g., those which block binding of the anti-PD-L1 antibody of the example to PD-L1), a routine cross-blocking assay such as that described in Antibodies, A Laboratory Manual, Cold Spring Harbor Laboratory, Ed Harlow and David Lane (1988), can be performed. Alternatively, epitope mapping, e.g., as described in Champe et al., J. Biol. Chem. 270:1388-1394 (1995), can be performed to determine whether the antibody binds an epitope of interest.

### VIII. Pharmaceutical Compositions and Formulations

Also provided herein are pharmaceutical compositions and formulations comprising a PD-1 axis binding antagonist and/or an antibody described herein (such as an anti-PD-L1 antibody (e.g., atezolizumab) or an anti-PD-1 antibody) and, optionally, a pharmaceutically acceptable carrier. The invention also provides pharmaceutical compositions and formulations comprising taxanes, e.g., nab-paclitaxel (ABRAXANE®), paclitaxel, or docetaxel.

Pharmaceutical compositions and formulations as described herein can be prepared by mixing the active ingredients (e.g., a PD-1 axis binding antagonist (e.g., an anti-PD-L1 antibody (e.g., atezolizumab) or an anti-PD-1 antibody) and/or a taxane (e.g., nab-paclitaxel or paclitaxel) having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The compositions and formulations herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g., films or microcapsules. The formulations to be used for *in vivo* administration are generally sterile. Sterility may be readily accomplished, e.g., by filtration through sterile filtration membranes.

## IX. Articles of Manufacture or Kits

In another embodiment of the invention, an article of manufacture or a kit is provided comprising a PD-1 axis binding antagonist (e.g., an anti-PD-L1 antibody (e.g., atezolizumab) or an anti-PD-1 antibody) and/or a taxane (e.g., nab-paclitaxel or paclitaxel). In some embodiments, the article of manufacture or kit further comprises package insert comprising instructions for using the PD-1 axis binding antagonist in conjunction with a taxane to treat or delay progression of locally advanced or metastatic breast cancer (e.g., locally advanced or metastatic TNBC) in an individual or to enhance immune function of an individual having locally advanced or metastatic breast cancer (e.g., locally advanced or metastatic TNBC). Any of the PD-1 axis binding antagonists and/or taxanes described herein may be included in the article of manufacture or kits.

In some embodiments, the PD-1 axis binding antagonist (e.g., an anti-PD-L1 antibody (e.g., atezolizumab) or an anti-PD-1 antibody) and the taxane (e.g., nab-paclitaxel or paclitaxel) are in the same container or separate containers. Suitable containers include, for example, bottles, vials, bags and syringes. The container may be formed from a variety of materials such as glass, plastic (such as polyvinyl chloride or polyolefin), or metal alloy (such as stainless steel or hastelloy). In some embodiments, the container holds the formulation and the label on, or associated with, the container may indicate directions for use. The article of manufacture or kit may further include other materials desirable from a commercial and user standpoint, including other buffers, diluents, filters, needles, syringes, and package inserts with instructions for use. In some embodiments, the article of manufacture further includes one or more of another agent (e.g., a chemotherapeutic agent, and anti-neoplastic agent). Suitable containers for the one or more agent include, for example, bottles, vials, bags and syringes.

For example, the invention provides a kit for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more reagents for determining the expression level of PD-L1 in a sample (e.g., a tumor sample) obtained from the patient, wherein the patient has not been previously treated for the TNBC, and wherein a detectable expression level of PD-L1 in the sample identifies the patient as likely to respond to treatment with the anti-cancer therapy. In some embodiments, the kit further includes a PD-1 axis binding antagonist and/or a taxane.

In another example, the invention provides a kit for treating a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more of (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel); and instructions for administering the anti-cancer therapy to the patient based on a detectable expression level of PD-L1 in a sample (e.g., a tumor sample) obtained from the patient. In some embodiments, the kit includes reagents for determining the presence or expression level of one or more of PD-L1, CD8, or sTILs.

In yet another example, the invention provides a kit for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more reagents for determining the expression level of PD-L1 in tumor-infiltrating immune cells in a tumor sample obtained from the patient, wherein the patient has not been previously treated for the TNBC, and wherein a detectable expression level of PD-L1 in tumor-infiltrating immune cells that comprise about 1% or more of the tumor sample identifies the patient as likely to respond to treatment with the anti-cancer therapy. In some embodiments, the kit further includes a PD-1 axis binding antagonist and/or a taxane.

In another example, the invention provides a kit for treating a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more of (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel); and instructions for administering the anti-cancer therapy to the patient based on a detectable expression level of PD-L1 in tumor-infiltrating immune cells that comprise about 1% or more of a tumor sample obtained from the patient. In some embodiments, the kit includes reagents for determining the presence or expression level of one or more of PD-L1, CD8, or sTILs.

In a further example, the invention provides a kit for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more reagents for determining the expression level of PD-L1 in cells in a tumor sample obtained from the patient, wherein the patient has not been previously treated for the TNBC, and wherein a detectable expression level of PD-L1 about 1% or more of the tumor cells in a tumor sample identifies the patient as likely to respond to treatment with the anti-cancer therapy. In some embodiments, the kit further includes a PD-1 axis binding antagonist and/or a taxane.

In another example, the invention provides a kit for treating a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more of (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel); and instructions for administering the anti-cancer therapy to the patient based on a detectable expression level of PD-L1 in about 1% or more of the tumor cells in a tumor sample obtained from the patient. In some embodiments, the kit includes reagents for determining the presence or expression level of one or more of PD-L1, CD8, or sTILs.

In a still further example, the invention provides a kit for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more reagents for determining the expression level of CD8 in tumor-infiltrating immune cells in a tumor sample obtained from the patient, wherein the patient has not been previously treated for the TNBC, and wherein a detectable expression level of CD8 in tumor-infiltrating immune cells that comprise about 0.5% or more of the tumor sample identifies the patient as likely to respond to treatment with the anti-cancer therapy. In some embodiments, the kit further includes a PD-1 axis binding antagonist and/or a taxane.

In another example, the invention provides a kit for treating a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more of (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel); and instructions for administering the anti-cancer therapy to the patient based on a detectable expression level of CD8 in tumor-infiltrating immune cells that comprise about 0.5% or more of a tumor sample obtained from the patient. In some embodiments, the kit includes reagents for determining the presence or expression level of one or more of PD-L1, CD8, or sTILs.

In a further example, the invention provides a kit for identifying a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more reagents for determining the percentage of sTILs in a tumor sample obtained from the patient, wherein the patient has not been previously treated for the TNBC, and wherein a percentage of sTILs of about 5% or more of the tumor sample identifies the patient as likely to respond to treatment with the anti-cancer therapy. In some embodiments, the kit further includes a PD-1 axis binding antagonist and/or a taxane.

In yet another example, the invention provides a kit for treating a patient suffering from a locally advanced or metastatic breast cancer (e.g., locally advanced or metastatic TNBC) who is likely to respond to treatment with an anti-cancer therapy comprising (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel), the kit including one or more of (i) a PD-1 axis binding antagonist (e.g., a human PD-1 axis binding antagonist selected from an anti-PD-L1 antibody and an anti-PD-1 antibody) and (ii) a taxane (e.g., nab-paclitaxel or paclitaxel); and instructions for administering the anti-cancer therapy to the patient based on a percentage of sTILs of about 5% or more of a tumor sample obtained from the patient. In some embodiments, the kit includes reagents for determining the presence or expression level of one or more of PD-L1, CD8, or sTILs.

In any of the preceding kits, the anti-PD-L1 antibody may be atezolizumab.

In any of the preceding kits, the taxane may be nab-paclitaxel.

In any of the preceding kits, the taxane may be paclitaxel.

In some embodiments of any of the preceding kits, whether the patient is likely to respond to treatment with the anti-cancer therapy is determined in terms of progression-free survival.

In other embodiments of any of the preceding kits, whether the patient is likely to respond to treatment with the anti-cancer therapy is determined in terms of overall survival.

The specification is considered to be sufficient to enable one skilled in the art to practice the invention. Various modifications of the invention in addition to those shown and described herein will become apparent to those skilled in the art from the foregoing description and fall within the scope of the appended claims.

## EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

**Example 1: Combination treatment with anti-PD-L1 antibody and nab-paclitaxel (ABRAXANE®) achieved response in a phase Ib clinical trial for patients with metastatic triple-negative breast cancer (mTNBC)**

Metastatic triple-negative breast cancer (mTNBC) is associated with poor prognosis and is characterized by a high mutation rate, increased levels of tumor-infiltrating lymphocytes, and high PD-L1 expression levels. MPDL3280A is a humanized monoclonal antibody that can restore tumor-specific T-cell immunity by inhibiting the binding of PD-L1 to PD-1 and has demonstrated durable responses as a monotherapy in mTNBC. This study is the first combination trial of a checkpoint inhibitor with chemotherapy in patients with mTNBC.

**Methods**

This arm of a multi-center, multi-arm phase Ib study evaluated MPDL3280A in combination with weekly nab-paclitaxel in patients with mTNBC. Primary endpoints were safety and tolerability, with secondary endpoints of PK and clinical activity. Key eligibility criteria included measurable disease; Eastern Cooperative Oncology Group (ECOG) performance status of 0 or 1; and $\leq$ 2 prior cytotoxic regimens. Patients received atezolizumab 800 mg once every two weeks (on days 1 and 15) with nab-paclitaxel 125 mg/m$^2$ weekly (on days 1, 8, and 15) for 3 weeks in 4-week cycles, continued until loss of clinical benefit. If nab-paclitaxelwas discontinued due to toxicity, MPDL3280A could be continued as monotherapy. ORR was assessed by Response Evaluation Criteria In Solid Tumors (RECIST) v1.1. PD-L1 expression was scored at 4 diagnostic levels based on PD-L1 staining on tumor cells and tumor-infiltrating immune cells in an immunohistochemical (IHC) assay.

**Results**

Eleven patients were safety-evaluable. All patients were female with a median age of 58 years (age range: 32-75). No unexpected or dose-limiting toxicities were observed. The median duration of safety follow-up was 88 days (range: 27-182 days). The efficacy-evaluable population consisted of 5 patients who had ≥ 1 scan and ≥ 3 months follow-up. Of the five patients, four patients showed a partial response (PR) and one patient showed stable disease (SD). The observed results indicate that the combination of MPDL3280A and nab-paclitaxel is both safe and efficacious in patients with mTNBC.

**Example 2: A combination treatment regimen of anti-PD-L1 antibody and nab-paclitaxel (ABRAXANE®) as a first-line therapy for patients with mTNBC**

As an alternative, a combination treatment regimen of MPDL3280A and nab-paclitaxel can serve as a first-line therapy for patients with mTNBC.
Patients with histologically documented locally advanced or metastatic TNBC; no prior systemic therapy for advanced TNBC; ECOG performance status of 0 or 1; and measurable disease per RECIST v1.1 may be dosed with MPDL3280A (840 mg) on days 1 and 15, plus nab-paclitaxel (100 mg/m$^2$) on days 1, 8, and 15. All treatments are given on a 28-day cycle. Patients may be stratified by the presence of liver metastases, prior taxane therapy, and the PD-L1 status of tumor-infiltrating immune cells (IC0 vs IC1/2/3), which can be centrally evaluated by IHC. To capture pseudo-progression and delayed responses to MPDL3280A, patients with radiographic progression may continue to receive open-label MPDL3280A alone or with nab-paclitaxel until unacceptable toxicity or loss of clinical benefit.

**Example 3: PD-L1 expression in tumor-infiltrating immune cells (IC) is predictive of efficacy from combination treatment with the anti-PD-L1 antibody atezolizumab and nab-paclitaxel (ABRAXANE®) as a first-line therapy for patients with locally advanced or metastatic TNBC**

The Phase III IMpassion130 study (NCT02425891) evaluated atezolizumab (anti-PD-L1) + nab-paclitaxel (nab-Px) versus placebo + nabPx as first-line treatment for patients with locally advanced or metastatic TNBC. The study met its co-primary PFS endpoint in intent-to-treat (ITT) patients and in patients with PD-L1 ≥1% on tumor-infiltrating immune cells (IC+). Clinically meaningful OS benefit was seen at interim OS analysis, notably in patients with PD-L1 IC+ tumors (Table 4). This example describes efficacy data in immunologically and clinically relevant, biomarker-defined subgroups.

**Methods**

Patients had histologically documented metastatic or unresectable locally advanced TNBC (evaluated locally per American Society of Clinical Oncology/College of American Pathologists (ASCO-CAP)). Patients were randomized 1:1 to nabPx 100 mg/m$^2$ IV (on days 1, 8 and 15 of a 28-day cycle) + atezolizumab 840 mg IV (A-nabPx) q2w or placebo (P-nabPx) until progression or toxicity. The following biomarkers were centrally analyzed: PD-L1 on tumor-infiltrating immune cells (IC) and tumor cells (TC) using the VENTANA SP142 IHC assay, intratumoral CD8 by IHC, stromal tumor-infiltrating lymphocytes (sTILs), and ER/PR/HER2 status. The VENTANA SP142 IHC assay was performed according to the manufacturer's instructions. The IC and TC IHC diagnostic criteria are described in Tables 4 and 5, respectively. See also International Patent Application Publication Nos. WO 2016/183326 and WO 2016/196298, e.g., in Example 1. CD8 IHC was evaluated as percentage of CD8 immunostaining covering the center of tumor area (see, e.g., Emens et al. JAMA Oncology doi:10.1001/jamaoncol.2018.4224, 2018). sTILs were evaluated at Histogenex according to Salgado et al. Annals of Oncology, 26(2):259-271, 2015 in hematoxylin and eosin (H&E)-stained tumor slides.

**Table 4. Tumor-infiltrating immune cell (IC) IHC diagnostic criteria**

| PD-L1 Diagnostic Assessment | IC Score |
|---|---|
| Absence of any discernible PD-L1 staining<br>OR<br>Presence of discernible PD-L1 staining of any intensity in tumor-infiltrating immune cells covering <1% of tumor area occupied by tumor cells, associated intratumoral stroma, and contiguous peri-tumoral desmoplastic stroma | IC0 |

(continued)

| PD-L1 Diagnostic Assessment | IC Score |
|---|---|
| Presence of discernible PD-L1 staining of any intensity in tumor-infiltrating immune cells covering ≥1% to <5% of tumor area occupied by tumor cells, associated intratumoral stroma, and contiguous peri-tumoral desmoplastic stroma | IC1 |
| Presence of discernible PD-L1 staining of any intensity in tumor-infiltrating immune cells covering ≥5% to <10% of tumor area occupied by tumor cells, associated intratumoral stroma, and contiguous peri-tumoral desmoplastic stroma | IC2 |
| Presence of discernible PD-L1 staining of any intensity in tumor-infiltrating immune cells covering ≥10% of tumor area occupied by tumor cells, associated intratumoral stroma, and contiguous peri-tumoral desmoplastic stroma | IC3 |

**Table 5. Tumor cell (TC) IHC diagnostic criteria**

| PD-L1 Diagnostic Assessment | TC Score |
|---|---|
| Absence of any discernible PD-L1 staining OR Presence of discernible PD-L1 staining of any intensity in <1% of tumor cells | TC0 |
| Presence of discernible PD-L1 staining of any intensity in ≥1% to <5% of tumor cells | TC1 |
| Presence of discernible PD-L1 staining of any intensity in ≥5% to <50% of tumor cells | TC2 |
| Presence of discernible PD-L1 staining of any intensity in ≥50% of tumor cells | TC3 |

## Results

PD-L1 IC was highly predictive of A-nabPx efficacy (Table 6). The majority of PD-L1 TC+ tumors were also PD-L1 IC+. Intratumoral CD8, but not sTILs, was well correlated with PD-L1 IC. Consequently, CD8 was predictive of A-nabPx efficacy for PFS/OS, while sTILs only predicted PFS benefit. Local versus central TNBC assessment was concordant in most patients. Local versus central lab-defined

TNBC populations derived similar benefit from A-nabPx. The predictiveness of PD-L1 IC is shown by the statistical meaningfulness ($p < 0.05$) in the interaction analysis for both the PFS and OS evaluation of PD-L1 status (Table 7). In Table 7, a Cox Regression Model was used as follows: Time-to-event (INV-PFS or OS) = Treatment (Atezolizumab vs Placebo) + PD-L1 Status (IC 0 vs IC 1/2/3) + Interaction(Treatment x PD-L1 Status).

## Conclusions

These data demonstrate that PD-L1 IC status is the most robust predictive biomarker of those evaluated for selecting previously untreated locally advanced or metastatic TNBC patients who benefit from the combination of atezolizumab and nab-paclitaxel. PD-L1 IC status can be used to identify patients who are likely to respond to treatment with an anti-cancer therapy comprising a human PD-1 axis binding antagonist selected from an anti-PD-L1 antibody and an anti-PD-1 antibody and nab-paclitaxel. Exploratory efficacy analyses from IMpassion130 also suggest consistency between local and central ER/PR/HER2 testing.

**Table 6. Results from Impassion130 Phase III clinical trial**

| Population | A-nabPx | P-nabPx |
|---|---|---|
| Primary data, stratified | | |
| ITT, n | 451 | 451 |
| mPFS (95% CI), mo | 7.2 (5.6-7.5) | 5.5 (5.3-5.6) |
| PFS HR (95% CI) | 0.80 (0.69-0.92); P=0.0025 | |
| mOS (95% CI), mo | 21.3 (17.3-23.4) | 17.6 (15.9-20.0) |

(continued)

| Population | A-nabPx | P-nabPx |
|---|---|---|
| Primary data, stratified | | |
| OS HR (95% CI) | 0.84 (0.69-1.02); P=0.0840 | |
| PD-L1 IC+, n (%) | 185 (41%) | 184 (41%) |
| mPFS (95% CI), mo | 7.5 (6.7-9.2) | 5.0 (3.8-5.6) |
| PFS HR (95% CI) | 0.62 (0.49-0.78); P<0.0001 | |
| mOS (95% CI) mo | 25.0 (22.6-NE) | 15.5 (13.1-19.4) |
| OS HR (95% CI) | 0.62 (0.45-0.86)[a] | |
| Exploratory/biomarker data, unstratified | | |
| PD-L1 TC evaluable, n | 449 | 451 |
| PD-L1 TC+, n (%) | 38 (8%) | 40 (9%) |
| PFS HR (95% CI) | 0.51 (0.31-0.84) | |
| OS HR (95% CI) | 0.63 (0.33-1.21) | |
| CD8 evaluable, n | 371 | 349 |
| CD8 ≥0.5%, n (%) | 261 (70%) | 239 (68%) |
| PFS HR (95% CI) | 0.74 (0.61-0.91) | |
| OS HR (95% CI) | 0.66 (0.50-0.88) | |
| sTIL evaluable, n | 448 | 444 |
| sTIL ≥5%, n (%) | 282 (63%) | 272 (61%) |
| PFS HR (95% CI) | 0.76 (0.63-0.92) | |
| OS HR (95% CI) | 0.83 (0.64-1.08) | |
| cTNBC evaluable, n | 420 | 412 |
| cTNBC ITT, n (%) | 307 (73%) | 317 (77%) |
| PFS HR (95% CI) | 0.81 (0.68-0.98) | |
| OS HR (95% CI) | 0.85 (0.67-1.08) | |
| cTNBC PD-L1 IC+, n (%) | 133 (43%) | 134 (42%) |
| PFS HR (95% CI) | 0.67 (0.51-0.88) | |
| OS HR (95% CI) | 0.69 (0.47-1.00) | |
| Data cutoff: 17 April 2018 (12.9-mo median follow up).<br>cTNBC, centrally confirmed TNBC<br>TC/IC+, **PD-L1 ≥1% (VENTANA SP142 assay)**<br>[a] Not formally tested per hierarchical study design. | | |

**Table 7. PD-L1 IC Predictiveness**

| | Interaction(Treatment x PD-L1 Status) p-value |
|---|---|
| Investigator-Assessed Progression-Free Survival | 0.0055 |
| Overall Survival | 0.0178 |

**Example 4: IMpassion130: updated overall survival (OS) from a global, randomized, double-blind, placebo-controlled, Phase III study of atezolizumab + nab-paclitaxel in previously untreated locally advanced or metastatic triple-negative breast cancer (mTNBC)**

As is described in Example 3, IMpassion130 evaluated the anti-PD-L1 antibody atezolizumab (atezo) + nab-paclitaxel (nP) versus placebo + nP in first-line mTNBC. The primary PFS analysis found that atezo + nP significantly improved PFS in intent-to-treat (ITT) and PD-L1+ patients versus placebo + nP, with efficacy driven by the PD-L1+ population. At that time, the first interim OS analysis was conducted. In this Example, the second interim OS analysis is reported.

**Methods**

As is described in Example 3, eligible patients had histologically documented mTNBC, ECOG PS 0-1 and tumor tissue for PD-L1 testing. Patients were randomized 1:1 to IV atezo 840 mg or placebo on days 1 and 15 + nP 100 mg/m$^2$ on days 1, 8, and 15 of each 28 day cycle until progression (stratification factors: prior taxanes, liver metastases, PD-L1 on tumor-infiltrating immune cells [IC]). RECIST 1.1 PFS (in ITT and PD-L1+ pts) and OS (tested in ITT and, if significant, PD-L1+ patients) were co-primary endpoints.

**Results**

OS data are shown in Table 8. As of the data cutoff date (January 2, 2019), 9% of patients in the atezo + nP arm and 3% in the placebo + nP arm were still on treatment. A 7.0 month improvement in median OS was observed in PD-L1+ patients with atezo + nP (25.0 mo) versus placebo + nP (18.0 mo; HR, 0.71 [95% CI: 0.54, 0.94]). A 4.5 month safety update showed that the safety profile remained tolerable.

**Table 8. Results from Second Interim OS Analysis from IMpassion130**

| | Atezo + nP | Placebo + nP |
|---|---|---|
| **ITT population** (events/patients, n/n [%]) | 255/451 (57%) | 279/451 (62%) |
| HR (95% CI); log-rank *P* | 0.86 (0.72, 1.02); 0.078[a] | - |
| Median OS (95% CI), mo | 21.0 (19.0, 22.6) | 18.7 (16.9, 20.3) |
| 2-year OS (95% CI), % | 42 (37, 47) | 39 (34, 44) |
| Median follow-up duration, mo | 18.5 | 17.5 |
| **PD-L1+ population[b]** (events/patients, n/n [%]) | 94/185 (51%) | 110/184 (60%) |
| HR (95% CI) | 0.71 (0.54, 0.94) | - |
| Median OS (95% CI), mo | 25.0 (19.6, 30.7) | 18.0 (13.6, 20.1) |
| 2-year OS (95% CI), % | 51 (43, 59) | 37 (29, 45) |
| HRs estimated per stratified Cox model. [a] Not significant. [b] ≥ 1% PD-L1 on IC (VENTANA SP142 assay). | | |

Comparison of overall survival in PD-L1 + and PD-L1- populations is shown in Table 9.

**Table 9. Comparison of OS in PD-L1+ and PD-L1- populations**

| Population | Median OS | | HR (95% CI) |
|---|---|---|---|
| | **A + *nab*-P** | **P + *nab*-P** | |
| PD-L1 IC+ | 25.0 mo | 18.0 mo | 0.71 (0.54, 0.94) |
| PD-L1 IC- | 19.7 mo | 19.6 mo | 0.97 (0.78, 1.20) |

**Conclusions**

The second IMpassion130 interim OS analysis was consistent with the first analysis, confirming clinically meaningful OS benefit for atezolizumab + nP in previously untreated PD-L1+ mTNBC.

PD-L1 IC status predicts clinical benefit with atezolizumab + nab-paclitaxel.

**Example 5: IMpassion131, a double-blind placebo-controlled randomized phase III trial of paclitaxel ± atezolizumab as first-line therapy for inoperable locally advanced/metastatic triple-negative breast cancer (mTNBC)**

Combining atezolizumab with first-line nab-paclitaxel for mTNBC significantly improved progression-free survival (PFS) in the randomized phase III IMpassion130 trial. In patients with PD-L1-positive tumors, there was a more pronounced PFS improvement and a clinically meaningful effect on overall survival (OS; coprimary endpoints). The IMpassion131 trial (NCT03125902) evaluated first-line atezolizumab combined with paclitaxel for mTNBC.

**Methods**

In this global double-blind placebo-controlled randomized phase III trial, eligible patients had inoperable measurable locally advanced/metastatic centrally confirmed TNBC, were eligible for taxane monotherapy, and had received no prior chemotherapy or targeted systemic therapy for mTNBC. Prior (neo)adjuvant chemotherapy was permitted if completed ≥12 months before randomization. Patients were randomized 2:1 to atezolizumab 840 mg or placebo on days 1 and 15 q28d, both given with paclitaxel 90 mg/m$^2$ on days 1, 8, & 15 q28d until disease progression or unacceptable toxicity. All patients received standard corticosteroid premedication before paclitaxel. Stratification factors were tumor PD-L1 status (IC0 [immune cell expression <1%] vs IC1/2/3 [≥1% expression] tested centrally by VENTANA SP142 assay and kept blinded), prior taxane therapy, presence of liver metastases, and geographic region. The primary endpoint was investigator-assessed PFS, tested hierarchically in the PD-L1+ population (IC1/2/3) and then in the intent-to-treat (ITT) population. The analysis plan was informed by findings from IMpassion130 and is event driven for the primary PFS analysis. Secondary endpoints include OS, time to deterioration in global health status/health-related quality of life, 12-month PFS rate, objective response rate, duration of response, clinical benefit rate, and safety.

**Other Embodiments**

Some embodiments of the technology described herein can be defined according to any of the following numbered embodiments:

1. A method for identifying a patient suffering from a locally advanced or metastatic triple-negative breast cancer (TNBC) who is likely to respond to treatment with an anti-cancer therapy comprising (i) a human PD-1 axis binding antagonist selected from an anti-PD-L1 antibody and an anti-PD-1 antibody and (ii) nab-paclitaxel, the method comprising determining the expression level of PD-L1 in tumor-infiltrating immune cells in a tumor sample obtained from the patient, wherein the patient has not been previously treated for the TNBC, and wherein a detectable expression level of PD-L1 in tumor-infiltrating immune cells that comprise about 1% or more of the tumor sample identifies the patient as likely to respond to treatment with the anti-cancer therapy.

2. A method for selecting an anti-cancer therapy for a patient suffering from a locally advanced or metastatic TNBC, the method comprising:

(a) determining the expression level of PD-L1 in tumor-infiltrating immune cells in a tumor sample obtained from the patient, wherein the patient has not been previously treated for the TNBC; and
(b) selecting an anti-cancer therapy comprising (i) a human PD-1 axis binding antagonist selected from an anti-PD-L1 antibody and an anti-PD-1 antibody and (ii) nab-paclitaxel for the patient based on a detectable expression level of PD-L1 in tumor-infiltrating immune cells that comprise about 1% or more of the tumor sample.

3. The method of embodiment 1 or 2, wherein the tumor sample obtained from the patient has a detectable expression level of PD-L1 in tumor-infiltrating immune cells that comprise (i) about 5% or more of the tumor sample or (ii) or about 10% or more of the tumor sample.

4. The method of any one of embodiments 1-3, wherein the patient has received no prior chemotherapy or targeted systemic therapy for inoperable locally advanced or metastatic TNBC.

5. The method of any one of embodiments 1-4, wherein the locally advanced TNBC is unresectable.

6. The method of any one of embodiments 1-5, wherein the tumor sample is a formalin-fixed and paraffin-embedded (FFPE) tumor sample, an archival tumor sample, a fresh tumor sample, or a frozen tumor sample.

7. The method of any one of embodiments 1-6, wherein the expression level of PD-L1 is a protein expression level.

8. The method of embodiment 7, wherein the protein expression level of PD-L1 is determined using immunohistochemistry (IHC), immunofluorescence, flow cytometry, or Western blot.

9. The method of embodiment 8, wherein the protein expression level of PD-L1 is determined using IHC.

10. The method of embodiment 8 or 9, wherein the protein expression level of PD-L1 is detected using an anti-PD-L1 antibody.

11. The method of embodiment 10, wherein the anti-PD-L1 antibody is SP142.

12. The method of any one of embodiments 1-11, further comprising administering an effective amount of the anti-cancer therapy to the patient.

13. The method of any one of embodiments 1-12, wherein the human PD-1 axis binding antagonist is atezolizumab.

14. The method of any one of embodiments 1-13, wherein whether the patient is likely to respond to treatment with the anti-cancer therapy is determined in terms of progression-free survival.

15. The method of any one of embodiments 1-14, wherein whether the patient is likely to respond to treatment with the anti-cancer therapy is determined in terms of overall survival.

16. A method of treating a patient suffering from a locally advanced or metastatic TNBC, the method comprising administering to the patient an effective amount of an anti-cancer therapy comprising (i) a human PD-1 axis binding antagonist selected from an anti-PD-L1 antibody and an anti-PD-1 antibody and (ii) nab-paclitaxel, wherein the patient has not been previously treated for the TNBC, and wherein the patient has been identified as likely to respond to the anti-cancer therapy based on a detectable expression level of PD-L1 in tumor-infiltrating immune cells that comprise about 1% or more of a tumor sample obtained from the patient.

17. The method of embodiment 16, wherein the human PD-1 axis binding antagonist is atezolizumab.

18. A pharmaceutical composition comprising a human PD-1 axis binding antagonist selected from an anti-PD-L1 antibody and an anti-PD-1 antibody for use in treatment of a patient diagnosed with locally advanced or metastatic TNBC, wherein the treatment comprises administration of the human PD-1 axis binding antagonist in combination with nab-paclitaxel, and wherein the patent is identified as likely to respond to an anti-cancer therapy comprising the human PD-1 axis binding antagonist and nab-paclitaxel based on a detectable expression level of PD-L1 in tumor-infiltrating immune cells that comprise about 1% or more of a tumor sample obtained from the patient.

19. The pharmaceutical composition of embodiment 18, wherein the human PD-1 axis binding antagonist is atezolizumab.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

SEQUENCE TABLE

<110> Genentech, Inc.

<120> DIAGNOSTIC AND THERAPEUTIC METHODS FOR TRIPLE-NEGATIVE BREAST CANCER

<130> 51177-023WO4

<150> US 62/848,163
<151> 2019-05-15

<150> US 62/837,507
<151> 2019-04-23

<150> US 62/734,677
<151> 2018-09-21

<160> 33

<170> PatentIn version 3.5

<210> 1
<211> 440
<212> PRT
<213> Artificial Sequence

<220>
<223> Synthetic Construct

<400> 1

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
                20                  25                  30

Gly Met His Trp Val Arg Gln Ala Pro Gly Lys Gly Leu Glu Trp Val
            35                  40                  45

Ala Val Ile Trp Tyr Asp Gly Ser Lys Arg Tyr Tyr Ala Asp Ser Val
        50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
            115                 120                 125

Arg Ser Thr Ser Glu Ser Thr Ala Ala Leu Gly Cys Leu Val Lys Asp
        130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
        290                 295                 300

```
Asp Trp Leu Asn Gly Lys Glu Tyr Lys Cys Lys Val Ser Asn Lys Gly
305             310             315             320


Leu Pro Ser Ser Ile Glu Lys Thr Ile Ser Lys Ala Lys Gly Gln Pro
                325             330             335


Arg Glu Pro Gln Val Tyr Thr Leu Pro Pro Ser Gln Glu Glu Met Thr
                340             345             350


Lys Asn Gln Val Ser Leu Thr Cys Leu Val Lys Gly Phe Tyr Pro Ser
                355             360             365


Asp Ile Ala Val Glu Trp Glu Ser Asn Gly Gln Pro Glu Asn Asn Tyr
    370             375             380


Lys Thr Thr Pro Pro Val Leu Asp Ser Asp Gly Ser Phe Phe Leu Tyr
385             390             395             400


Ser Arg Leu Thr Val Asp Lys Ser Arg Trp Gln Glu Gly Asn Val Phe
                405             410             415


Ser Cys Ser Val Met His Glu Ala Leu His Asn His Tyr Thr Gln Lys
                420             425             430


Ser Leu Ser Leu Ser Leu Gly Lys
                435             440


<210>   2
<211>   214
<212>   PRT
<213>   Artificial Sequence

<220>
<223>   Synthetic Construct

<400>   2

Glu Ile Val Leu Thr Gln Ser Pro Ala Thr Leu Ser Leu Ser Pro Gly
1               5               10              15


Glu Arg Ala Thr Leu Ser Cys Arg Ala Ser Gln Ser Val Ser Ser Tyr
            20              25              30
```

Leu Ala Trp Tyr Gln Gln Lys Pro Gly Gln Ala Pro Arg Leu Leu Ile
        35                  40              45

Tyr Asp Ala Ser Asn Arg Ala Thr Gly Ile Pro Ala Arg Phe Ser Gly
        50                  55              60

Ser Gly Ser Gly Thr Asp Phe Thr Leu Thr Ile Ser Ser Leu Glu Pro
65                  70              75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
                100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
        130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
                180                 185                 190

Ala Cys Glu Val Thr His Gln Gly Leu Ser Ser Pro Val Thr Lys Ser
        195                 200                 205

Phe Asn Arg Gly Glu Cys
        210

<210>  3
<211>  118
<212>  PRT
<213>  Artificial Sequence

<220>

<223>    Synthetic Construct

<400>    3

Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Ser Asp Ser
            20              25              30

Trp Ile His Trp Val Arg Gln Ala Pro Gly Lys Gly Leu Glu Trp Val
        35              40              45

Ala Trp Ile Ser Pro Tyr Gly Gly Ser Thr Tyr Tyr Ala Asp Ser Val
    50              55              60

Lys Gly Arg Phe Thr Ile Ser Ala Asp Thr Ser Lys Asn Thr Ala Tyr
65              70              75              80

Leu Gln Met Asn Ser Leu Arg Ala Glu Asp Thr Ala Val Tyr Tyr Cys
            85              90              95

Ala Arg Arg His Trp Pro Gly Gly Phe Asp Tyr Trp Gly Gln Gly Thr
        100             105             110

Leu Val Thr Val Ser Ala
        115

<210>    4
<211>    108
<212>    PRT
<213>    Artificial Sequence

<220>
<223>    Synthetic Construct

<400>    4

Asp Ile Gln Met Thr Gln Ser Pro Ser Ser Leu Ser Ala Ser Val Gly
1               5               10              15

Asp Arg Val Thr Ile Thr Cys Arg Ala Ser Gln Asp Val Ser Thr Ala
            20              25              30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
        50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Leu Tyr His Pro Ala
                85                  90                  95

Thr Phe Gly Gln Gly Thr Lys Val Glu Ile Lys Arg
                100                 105

<210>   5
<211>   10
<212>   PRT
<213>   Artificial Sequence

<220>
<223>   Synthetic Construct

<220>
<221>   Xaa
<222>   (6)..(6)
<223>   Xaa is D or G

<400>   5

Gly Phe Thr Phe Ser Xaa Ser Trp Ile His
1                   5                   10

<210>   6
<211>   18
<212>   PRT
<213>   Artificial Sequence

<220>
<223>   Synthetic Construct

<220>
<221>   Xaa
<222>   (4)..(4)
<223>   Xaa is S or L

```
<220>
<221>  Xaa
<222>  (10)..(10)
<223>  Xaa is T or S

<400>  6

Ala Trp Ile Xaa Pro Tyr Gly Gly Ser Xaa Tyr Tyr Ala Asp Ser Val
1               5                   10                  15


Lys Gly



<210>  7
<211>  9
<212>  PRT
<213>  Artificial Sequence

<220>
<223>  Synthetic Construct

<400>  7

Arg His Trp Pro Gly Gly Phe Asp Tyr
1               5


<210>  8
<211>  25
<212>  PRT
<213>  Artificial Sequence

<220>
<223>  Synthetic Construct

<400>  8

Glu Val Gln Leu Val Glu Ser Gly Gly Gly Leu Val Gln Pro Gly Gly
1               5                   10                  15


Ser Leu Arg Leu Ser Cys Ala Ala Ser
                20              25


<210>  9
<211>  13
<212>  PRT
<213>  Artificial Sequence

<220>
<223>  Synthetic Construct
```

<400> 9

Trp Val Arg Gln Ala Pro Gly Lys Gly Leu Glu Trp Val
1               5               10


<210> 10
<211> 32
<212> PRT
<213> Artificial Sequence

<220>
<223> Synthetic Construct

<400> 10

Arg Phe Thr Ile Ser Ala Asp Thr Ser Lys Asn Thr Ala Tyr Leu Gln
1               5               10              15


Met Asn Ser Leu Arg Ala Glu Asp Thr Ala Val Tyr Tyr Cys Ala Arg
            20              25              30


<210> 11
<211> 11
<212> PRT
<213> Artificial Sequence

<220>
<223> Synthetic Construct

<400> 11

Trp Gly Gln Gly Thr Leu Val Thr Val Ser Ala
1               5               10


<210> 12
<211> 11
<212> PRT
<213> Artificial Sequence

<220>
<223> Synthetic Construct


<220>
<221> Xaa
<222> (5)..(5)
<223> Xaa is D or V

<220>

```
<221>  Xaa
<222>  (6)..(6)
<223>  Xaa is V or I

<220>
<221>  Xaa
<222>  (7)..(7)
<223>  Xaa is S or N

<220>
<221>  Xaa
<222>  (9)..(9)
<223>  Xaa is A or F

<220>
<221>  Xaa
<222>  (10)..(10)
<223>  Xaa is V or L

<400>  12

Arg Ala Ser Gln Xaa Xaa Xaa Thr Xaa Xaa Ala
1                5                   10


<210>  13
<211>  7
<212>  PRT
<213>  Artificial Sequence

<220>
<223>  Synthetic Construct


<220>
<221>  Xaa
<222>  (4)..(4)
<223>  Xaa is F or T

<220>
<221>  Xaa
<222>  (6)..(6)
<223>  Xaa is Y or A

<400>  13

Ser Ala Ser Xaa Leu Xaa Ser
1                5


<210>  14
<211>  9
<212>  PRT
<213>  Artificial Sequence
```

```
<220>
<223>  Synthetic Construct


<220>
<221>  Xaa
<222>  (3)..(3)
<223>  Xaa is Y, G, F, or S


<220>
<221>  Xaa
<222>  (4)..(4)
<223>  Xaa is L, Y, F, or W


<220>
<221>  Xaa
<222>  (5)..(5)
<223>  Xaa is Y, N, A, T, G, F, or I


<220>
<221>  Xaa
<222>  (6)..(6)
<223>  Xaa is H, V, P, T, or I


<220>
<221>  Xaa
<222>  (8)..(8)
<223>  Xaa is A, W, R, P, or T


<400>  14


Gln Gln Xaa Xaa Xaa Xaa Pro Xaa Thr
1                   5



<210>  15
<211>  23
<212>  PRT
<213>  Artificial Sequence


<220>
<223>  Synthetic Construct


<400>  15


Asp Ile Gln Met Thr Gln Ser Pro Ser Ser Leu Ser Ala Ser Val Gly
1               5                   10                  15



Asp Arg Val Thr Ile Thr Cys
            20
```

```
<210>  16
<211>  15
<212>  PRT
<213>  Artificial Sequence

<220>
<223>  Synthetic Construct

<400>  16

Trp Tyr Gln Gln Lys Pro Gly Lys Ala Pro Lys Leu Leu Ile Tyr
1               5                   10                  15


<210>  17
<211>  32
<212>  PRT
<213>  Artificial Sequence

<220>
<223>  Synthetic Construct

<400>  17

Gly Val Pro Ser Arg Phe Ser Gly Ser Gly Ser Gly Thr Asp Phe Thr
1               5                   10                  15


Leu Thr Ile Ser Ser Leu Gln Pro Glu Asp Phe Ala Thr Tyr Tyr Cys
            20                  25                  30


<210>  18
<211>  11
<212>  PRT
<213>  Artificial Sequence

<220>
<223>  Synthetic Construct

<400>  18

Phe Gly Gln Gly Thr Lys Val Glu Ile Lys Arg
1               5                   10


<210>  19
<211>  10
<212>  PRT
<213>  Artificial Sequence

<220>
<223>  Synthetic Construct
```

<400> 19

Gly Phe Thr Phe Ser Asp Ser Trp Ile His
1               5               10


<210> 20
<211> 18
<212> PRT
<213> Artificial Sequence

<220>
<223> Synthetic Construct

<400> 20

Ala Trp Ile Ser Pro Tyr Gly Gly Ser Thr Tyr Tyr Ala Asp Ser Val
1               5               10              15


Lys Gly


<210> 21
<211> 9
<212> PRT
<213> Artificial Sequence

<220>
<223> Synthetic Construct

<400> 21

Arg His Trp Pro Gly Gly Phe Asp Tyr
1               5


<210> 22
<211> 11
<212> PRT
<213> Artificial Sequence

<220>
<223> Synthetic Construct

<400> 22

Arg Ala Ser Gln Asp Val Ser Thr Ala Val Ala
1               5               10


<210> 23
<211> 7

```
<212>  PRT
<213>  Artificial Sequence

<220>
<223>  Synthetic Construct

<400>  23

Ser Ala Ser Phe Leu Tyr Ser
1               5


<210>  24
<211>  9
<212>  PRT
<213>  Artificial Sequence

<220>
<223>  Synthetic Construct

<400>  24

Gln Gln Tyr Leu Tyr His Pro Ala Thr
1               5


<210>  25
<211>  118
<212>  PRT
<213>  Artificial Sequence

<220>
<223>  Synthetic Construct

<400>  25

Glu Val Gln Leu Val Glu Ser Gly Gly Gly Leu Val Gln Pro Gly Gly
1               5               10              15


Ser Leu Arg Leu Ser Cys Ala Ala Ser Gly Phe Thr Phe Ser Asp Ser
            20              25              30


Trp Ile His Trp Val Arg Gln Ala Pro Gly Lys Gly Leu Glu Trp Val
            35              40              45


Ala Trp Ile Ser Pro Tyr Gly Gly Ser Thr Tyr Tyr Ala Asp Ser Val
            50              55              60


Lys Gly Arg Phe Thr Ile Ser Ala Asp Thr Ser Lys Asn Thr Ala Tyr
65              70              75              80
```

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser
            115

<210> 26
<211> 122
<212> PRT
<213> Artificial Sequence

<220>
<223> Synthetic Construct

<400> 26

Glu Val Gln Leu Val Glu Ser Gly Gly Gly Leu Val Gln Pro Gly Gly
1                   5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
            35                  40                  45

Ala Trp Ile Ser Pro Tyr Gly Gly Ser Thr Tyr Tyr Ala Asp Ser Val
        50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Ala Ser Thr Lys
            115                 120

```
<210>   27
<211>   11
<212>   PRT
<213>   Artificial Sequence

<220>
<223>   Synthetic Construct

<400>   27

Trp Gly Gln Gly Thr Leu Val Thr Val Ser Ser
1               5                   10
```

```
<210>   28
<211>   10
<212>   PRT
<213>   Artificial Sequence

<220>
<223>   Synthetic Construct

<400>   28

Phe Gly Gln Gly Thr Lys Val Glu Ile Lys
1               5                   10
```

```
<210>   29
<211>   30
<212>   PRT
<213>   Artificial Sequence

<220>
<223>   Synthetic Construct

<400>   29

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30
```

```
<210>   30
<211>   14
<212>   PRT
<213>   Artificial Sequence

<220>
```

<223> Synthetic Construct

<400> 30

Trp Val Arg Gln Ala Pro Gly Lys Gly Leu Glu Trp Val Ala
1               5                   10

<210> 31
<211> 15
<212> PRT
<213> Artificial Sequence

<220>
<223> Synthetic Construct

<400> 31

Trp Gly Gln Gly Thr Leu Val Thr Val Ser Ser Ala Ser Thr Lys
1               5                   10                  15

<210> 32
<211> 447
<212> PRT
<213> Artificial Sequence

<220>
<223> Synthetic Construct

<400> 32

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
                20                  25                  30

Trp Ile His Trp Val Arg Gln Ala Pro Gly Lys Gly Leu Glu Trp Val
            35                  40                  45

Ala Trp Ile Ser Pro Tyr Gly Gly Ser Thr Tyr Tyr Ala Asp Ser Val
        50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

```
Ala Arg Arg His Trp Pro Gly Gly Phe Asp Tyr Trp Gly Gln Gly Thr
            100             105             110

Leu Val Thr Val Ser Ser Ala Ser Thr Lys Gly Pro Ser Val Phe Pro
            115             120             125

Leu Ala Pro Ser Ser Lys Ser Thr Ser Gly Gly Thr Ala Ala Leu Gly
        130             135             140

Cys Leu Val Lys Asp Tyr Phe Pro Glu Pro Val Thr Val Ser Trp Asn
145             150             155             160

Ser Gly Ala Leu Thr Ser Gly Val His Thr Phe Pro Ala Val Leu Gln
                165             170             175

Ser Ser Gly Leu Tyr Ser Leu Ser Ser Val Val Thr Val Pro Ser Ser
            180             185             190

Ser Leu Gly Thr Gln Thr Tyr Ile Cys Asn Val Asn His Lys Pro Ser
            195             200             205

Asn Thr Lys Val Asp Lys Lys Val Glu Pro Lys Ser Cys Asp Lys Thr
        210             215             220

His Thr Cys Pro Pro Cys Pro Ala Pro Glu Leu Leu Gly Gly Pro Ser
225             230             235             240

Val Phe Leu Phe Pro Pro Lys Pro Lys Asp Thr Leu Met Ile Ser Arg
                245             250             255

Thr Pro Glu Val Thr Cys Val Val Val Asp Val Ser His Glu Asp Pro
            260             265             270

Glu Val Lys Phe Asn Trp Tyr Val Asp Gly Val Glu Val His Asn Ala
        275             280             285

Lys Thr Lys Pro Arg Glu Glu Gln Tyr Ala Ser Thr Tyr Arg Val Val
        290             295             300
```

EP 4 249 917 A2

Ser Val Leu Thr Val Leu His Gln Asp Trp Leu Asn Gly Lys Glu Tyr
305            310            315            320

Lys Cys Lys Val Ser Asn Lys Ala Leu Pro Ala Pro Ile Glu Lys Thr
               325            330            335

Ile Ser Lys Ala Lys Gly Gln Pro Arg Glu Pro Gln Val Tyr Thr Leu
               340            345            350

Pro Pro Ser Arg Glu Glu Met Thr Lys Asn Gln Val Ser Leu Thr Cys
               355            360            365

Leu Val Lys Gly Phe Tyr Pro Ser Asp Ile Ala Val Glu Trp Glu Ser
     370            375            380

Asn Gly Gln Pro Glu Asn Asn Tyr Lys Thr Thr Pro Pro Val Leu Asp
385            390            395            400

Ser Asp Gly Ser Phe Phe Leu Tyr Ser Lys Leu Thr Val Asp Lys Ser
               405            410            415

Arg Trp Gln Gln Gly Asn Val Phe Ser Cys Ser Val Met His Glu Ala
               420            425            430

Leu His Asn His Tyr Thr Gln Lys Ser Leu Ser Leu Ser Pro Gly
               435            440            445

<210>  33
<211>  214
<212>  PRT
<213>  Artificial Sequence

<220>
<223>  Synthetic Construct

<400>  33

Asp Ile Gln Met Thr Gln Ser Pro Ser Ser Leu Ser Ala Ser Val Gly
1            5            10            15

Asp Arg Val Thr Ile Thr Cys Arg Ala Ser Gln Asp Val Ser Thr Ala
               20            25            30

```
Val Ala Trp Tyr Gln Gln Lys Pro Gly Lys Ala Pro Lys Leu Leu Ile
        35                40                45

Tyr Ser Ala Ser Phe Leu Tyr Ser Gly Val Pro Ser Arg Phe Ser Gly
        50                55                60

Ser Gly Ser Gly Thr Asp Phe Thr Leu Thr Ile Ser Ser Leu Gln Pro
65                70                75                80

Glu Asp Phe Ala Thr Tyr Tyr Cys Gln Gln Tyr Leu Tyr His Pro Ala
                85                90                95

Thr Phe Gly Gln Gly Thr Lys Val Glu Ile Lys Arg Thr Val Ala Ala
            100               105               110

Pro Ser Val Phe Ile Phe Pro Pro Ser Asp Glu Gln Leu Lys Ser Gly
        115               120               125

Thr Ala Ser Val Val Cys Leu Leu Asn Asn Phe Tyr Pro Arg Glu Ala
    130               135               140

Lys Val Gln Trp Lys Val Asp Asn Ala Leu Gln Ser Gly Asn Ser Gln
145               150               155               160

Glu Ser Val Thr Glu Gln Asp Ser Lys Asp Ser Thr Tyr Ser Leu Ser
            165               170               175

Ser Thr Leu Thr Leu Ser Lys Ala Asp Tyr Glu Lys His Lys Val Tyr
            180               185               190

Ala Cys Glu Val Thr His Gln Gly Leu Ser Ser Pro Val Thr Lys Ser
        195               200               205

Phe Asn Arg Gly Glu Cys
        210
```

The following numbered paragraphs contain statements of broad combinations of the inventive technical features herein disclosed:-

1. A method for identifying a patient suffering from a locally advanced or metastatic triple-negative breast cancer (TNBC) who is likely to respond to treatment with an anti-cancer therapy comprising (i) a human PD-1 axis binding antagonist selected from an anti-PD-L1 antibody and an anti-PD-1 antibody and (ii) a taxane, the method comprising determining the expression level of PD-L1 in a tumor sample obtained from the patient, wherein the patient has not been previously treated for the TNBC, and wherein a detectable expression level of PD-L1 in the tumor sample identifies the patient as likely to respond to treatment with the anti-cancer therapy.

2. A method for identifying a patient suffering from a locally advanced or metastatic TNBC who is likely to respond to treatment with an anti-cancer therapy comprising (i) a human PD-1 axis binding antagonist selected from an anti-PD-L1 antibody and an anti-PD-1 antibody and (ii) a taxane, the method comprising determining the expression level of PD-L1 in tumor-infiltrating immune cells in a tumor sample obtained from the patient, wherein the patient has not been previously treated for the TNBC, and wherein a detectable expression level of PD-L1 in tumor-infiltrating immune cells that comprise about 1% or more of the tumor sample identifies the patient as likely to respond to treatment with the anti-cancer therapy.

3. A method for selecting an anti-cancer therapy for a patient suffering from a locally advanced or metastatic TNBC, the method comprising:

(a) determining the expression level of PD-L1 in a tumor sample obtained from the patient, wherein the patient has not been previously treated for the TNBC; and
(b) selecting an anti-cancer therapy comprising (i) a human PD-1 axis binding antagonist selected from an anti-PD-L1 antibody and an anti-PD-1 antibody and (ii) a taxane for the patient based on a detectable expression level of PD-L1 in the tumor sample.

4. A method for selecting an anti-cancer therapy for a patient suffering from a locally advanced or metastatic TNBC, the method comprising:

(a) determining the expression level of PD-L1 in tumor-infiltrating immune cells in a tumor sample obtained from the patient, wherein the patient has not been previously treated for the TNBC; and
(b) selecting an anti-cancer therapy comprising (i) a human PD-1 axis binding antagonist selected from an anti-PD-L1 antibody and an anti-PD-1 antibody and (ii) a taxane for the patient based on a detectable expression level of PD-L1 in tumor-infiltrating immune cells that comprise about 1% or more of the tumor sample.

5. The method of any one of paragraphs 1-4, wherein the tumor sample obtained from the patient has a detectable expression level of PD-L1 in tumor-infiltrating immune cells that comprise (i) about 5% or more of the tumor sample or (ii) or about 10% or more of the tumor sample.

6. The method of any one of paragraphs 1-5, wherein the patient has received no prior chemotherapy or targeted systemic therapy for inoperable locally advanced or metastatic TNBC.

7. The method of any one of paragraphs 1-6, wherein the locally advanced TNBC is unresectable.

8. The method of any one of paragraphs 1-7, wherein the tumor sample is a formalin-fixed and paraffin-embedded (FFPE) tumor sample, an archival tumor sample, a fresh tumor sample, or a frozen tumor sample.

9. The method of any one of paragraphs 1-8, wherein the expression level of PD-L1 is a protein expression level.

10. The method of paragraph 9, wherein the protein expression level of PD-L1 is determined using immunohistochemistry (IHC), immunofluorescence, flow cytometry, or Western blot.

11. The method of paragraph 10, wherein the protein expression level of PD-L1 is determined using IHC.

12. The method of paragraph 10 or 11, wherein the protein expression level of PD-L1 is detected using an anti-PD-L1 antibody.

13. The method of paragraph 12, wherein the anti-PD-L1 antibody is SP142.

14. The method of any one of paragraphs 1-13, further comprising administering an effective amount of the anti-cancer therapy to the patient.

15. The method of any one of paragraphs 1-14, wherein the human PD-1 axis binding antagonist is atezolizumab.

16. The method of any one of paragraphs 1-15, wherein whether the patient is likely to respond to treatment with the anti-cancer therapy is determined in terms of progression-free survival.

17. The method of any one of paragraphs 1-16, wherein whether the patient is likely to respond to treatment with the anti-cancer therapy is determined in terms of overall survival.

18. A method of treating a patient suffering from a locally advanced or metastatic TNBC, the method comprising administering to the patient an effective amount of an anti-cancer therapy comprising (i) a human PD-1 axis binding antagonist selected from an anti-PD-L1 antibody and an anti-PD-1 antibody and (ii) a taxane, wherein the patient has not been previously treated for the TNBC, and wherein the patient has been identified as likely to respond to the anti-cancer therapy based on a detectable expression level of PD-L1 in a tumor sample obtained from the patient.

19. A method of treating a patient suffering from a locally advanced or metastatic TNBC, the method comprising administering to the patient an effective amount of an anti-cancer therapy comprising (i) a human PD-1 axis binding antagonist selected from an anti-PD-L1 antibody and an anti-PD-1 antibody and (ii) a taxane, wherein the patient has not been previously treated for the TNBC, and wherein the patient has been identified as likely to respond to the anti-cancer therapy based on a detectable expression level of PD-L1 in tumor-infiltrating immune cells that comprise about 1% or more of a tumor sample obtained from the patient.

20. The method of paragraph 18 or 19, wherein the human PD-1 axis binding antagonist is atezolizumab.

21. The method of any one of paragraphs 1-20, wherein the taxane is nab-paclitaxel.

22. The method of any one of paragraphs 1-20, wherein the taxane is paclitaxel.

23. A pharmaceutical composition comprising a human PD-1 axis binding antagonist selected from an anti-PD-L1 antibody and an anti-PD-1 antibody for use in treatment of a patient diagnosed with locally advanced or metastatic TNBC, wherein the treatment comprises administration of the human PD-1 axis binding antagonist in combination with a taxane, and wherein the patent is identified as likely to respond to an anti-cancer therapy comprising the human PD-1 axis binding antagonist and a taxane based on a detectable expression level of PD-L1 in a tumor sample obtained from the patient.

24. A pharmaceutical composition comprising a human PD-1 axis binding antagonist selected from an anti-PD-L1 antibody and an anti-PD-1 antibody for use in treatment of a patient diagnosed with locally advanced or metastatic TNBC, wherein the treatment comprises administration of the human PD-1 axis binding antagonist in combination with a taxane, and wherein the patent is identified as likely to respond to an anti-cancer therapy comprising the human PD-1 axis binding antagonist and a taxane based on a detectable expression level of PD-L1 in tumor-infiltrating immune cells that comprise about 1% or more of a tumor sample obtained from the patient.

25. The pharmaceutical composition of paragraph 23 or 24, wherein the human PD-1 axis binding antagonist is atezolizumab.

26. The pharmaceutical composition of any one of paragraphs 23-25, wherein the taxane is nab-paclitaxel.

27. The pharmaceutical composition of any one of paragraphs 23-25, wherein the taxane is paclitaxel.

**Claims**

1. A method for identifying a patient suffering from a locally advanced or metastatic triple-negative breast cancer (TNBC) who is likely to respond to treatment with an anti-cancer therapy comprising (i) a human PD-1 axis binding antagonist selected from an anti-PD-L1 antibody and an anti-PD-1 antibody and (ii) a taxane, the method comprising:

(a) determining the expression level of PD-L1 in a tumor sample obtained from the patient, wherein the patient has not been previously treated for the TNBC, and wherein a detectable expression level of PD-L1 in the tumor sample identifies the patient as likely to respond to treatment with the anti-cancer therapy; or
(b) determining the expression level of PD-L1 in tumor-infiltrating immune cells in a tumor sample obtained from the patient, wherein the patient has not been previously treated for the TNBC, and wherein a detectable expression level of PD-L1 in tumor-infiltrating immune cells that comprise about 1% or more of the tumor sample identifies the patient as likely to respond to treatment with the anti-cancer therapy.

2. A method for selecting an anti-cancer therapy for a patient suffering from a locally advanced or metastatic TNBC, the method comprising:

(a) determining the expression level of PD-L1 in a tumor sample obtained from the patient, wherein the patient has not been previously treated for the TNBC; and selecting an anti-cancer therapy comprising (i) a human PD-1 axis binding antagonist selected from an anti-PD-L1 antibody and an anti-PD-1 antibody and (ii) a taxane for the patient based on a detectable expression level of PD-L1 in the tumor sample; or

(b) determining the expression level of PD-L1 in tumor-infiltrating immune cells in a tumor sample obtained from the patient, wherein the patient has not been previously treated for the TNBC; and selecting an anti-cancer therapy comprising (i) a human PD-1 axis binding antagonist selected from an anti-PD-L1 antibody and an anti-PD-1 antibody and (ii) a taxane for the patient based on a detectable expression level of PD-L1 in tumor-infiltrating immune cells that comprise about 1% or more of the tumor sample.

3. The method of claim 1 or 2, wherein the tumor sample obtained from the patient has a detectable expression level of PD-L1 in tumor-infiltrating immune cells that comprise (i) about 5% or more of the tumor sample or (ii) or about 10% or more of the tumor sample.

4. The method of any one of claims 1-3, wherein the patient has received no prior chemotherapy or targeted systemic therapy for inoperable locally advanced or metastatic TNBC.

5. The method of any one of claims 1-4, wherein the locally advanced TNBC is unresectable.

6. The method of any one of claims 1-5, wherein the tumor sample is a formalin-fixed and paraffin-embedded (FFPE) tumor sample, an archival tumor sample, a fresh tumor sample, or a frozen tumor sample.

7. The method of any one of claims 1-6, wherein the expression level of PD-L1 is a protein expression level, optionally wherein the protein expression level of PD-L1 is determined using immunohistochemistry (IHC), immunofluorescence, flow cytometry, or Western blot.

8. The method of claim 7, wherein the protein expression level of PD-L1 is detected using an anti-PD-L1 antibody, optionally wherein the anti-PD-L1 antibody is SP142.

9. The method of any one of claims 1-8, wherein the human PD-1 axis binding antagonist is atezolizumab.

10. The method of any one of claims 1-9, wherein whether the patient is likely to respond to treatment with the anti-cancer therapy is determined in terms of progression-free survival or overall survival.

11. A human PD-1 axis binding antagonist selected from (a) an anti-PD-L1 antibody and (b) an anti-PD-1 antibody, and/or a taxane, for use in a method of treating a patient suffering from a locally advanced or metastatic TNBC, the method comprising administering to the patient an effective amount of an anti-cancer therapy comprising (i) a human PD-1 axis binding antagonist selected from an anti-PD-L1 antibody and an anti-PD-1 antibody and (ii) a taxane, wherein the patient has not been previously treated for the TNBC, and wherein

(a) the patient has been identified as likely to respond to the anti-cancer therapy based on a detectable expression level of PD-L1 in a tumor sample obtained from the patient; or

(b) the patient has been identified as likely to respond to the anti-cancer therapy based on a detectable expression level of PD-L1 in tumor-infiltrating immune cells that comprise about 1% or more of a tumor sample obtained from the patient.

12. The human PD-1 axis binding antagonist and/or taxane for use according to claim 11, wherein the human PD-1 axis binding antagonist is atezolizumab.

13. The method of any one of claims 1-10 or the human PD-1 axis binding antagonist and/or taxane for use according to claim 11 or 12, wherein the taxane is nab-paclitaxel or paclitaxel.

**14.** A pharmaceutical composition comprising (i) a human PD-1 axis binding antagonist selected from (a) an anti-PD-L1 antibody and (b) an anti-PD-1 antibody, and/or (ii) a taxane, for use in treatment of a patient diagnosed with locally advanced or metastatic TNBC, wherein the treatment comprises administration of the human PD-1 axis binding antagonist in combination with a taxane, and wherein the patient is identified as likely to respond to an anti-cancer therapy comprising the human PD-1 axis binding antagonist and a taxane based on:

(a) a detectable expression level of PD-L1 in a tumor sample obtained from the patient, or
(b) a detectable expression level of PD-L1 in tumor-infiltrating immune cells that comprise about 1% or more of a tumor sample obtained from the patient.

**15.** The pharmaceutical composition of claim 14, wherein the human PD-1 axis binding antagonist is atezolizumab, and/or the taxane is nab-paclitaxel or paclitaxel.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4683195 A
- US 4943533 A
- WO 9640210 A
- US 5212290 A
- US 5891996 A
- WO 9850433 A
- US 6235883 B
- EP 659439 A2
- US 5616582 A
- US 5457105 A
- US 5475001 A
- US 5654307 A
- US 5679683 A
- US 6084095 A
- US 6265410 B
- US 6455534 B
- US 6521620 B
- US 6596726 B
- US 6713484 B
- US 5770599 A
- US 6140332 A
- US 5866572 A
- US 6399602 B
- US 6344459 B
- US 6602863 B
- US 6391874 B
- US 6344455 B
- US 5760041 A
- US 6002008 A
- US 5747498 A
- WO 9814451 A
- WO 9850038 A
- WO 9909016 A
- WO 9924037 A
- US 5804396 A
- WO 199909016 A
- WO 199843960 A
- WO 199738983 A, Warner Lambert
- WO 199906378 A, Warner Lambert
- WO 199906396 A, Warner Lambert
- WO 199630347 A
- WO 199633978 A
- WO 19963397 A
- WO 199633980 A
- EP 404097 A
- WO 199301161 A
- US 4816567 A
- WO 199824893 A
- WO 199634096 A

- WO 199633735 A
- WO 199110741 A
- US 5545807 A
- US 5545806 A
- US 5569825 A
- US 5625126 A
- US 5633425 A
- US 5661016 A
- US 6982321 B
- US 7087409 B
- US 6075181 A
- US 6150584 A
- US 4275149 A
- US 4737456 A
- US 4318980 A
- WO 2010077634 A
- WO 2007005874 A
- WO 2011066389 A
- US 2013034559 A
- WO 2006121168 A
- WO 2009114335 A
- WO 2010027827 A
- WO 2011066342 A
- WO 2010077634 A1
- US 8217149 B
- US 7189826 B
- US 2006258841 A
- US 2006183887 A
- US 2006059575 A
- US 2005287149 A
- US 2005100546 A
- US 2005026229 A
- US 7078492 B
- US 7153507 B
- US 2005176122 A
- US 6919436 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- US 5821337 A
- US 7527791 B
- US 5770429 A
- US 7041870 B

- US 20070061900 A
- US 5869046 A
- WO 9316185 A
- US 5571894 A
- US 5587458 A
- US 5641870 A
- WO 9308829 A
- US 5731168 A
- US 5807706 A
- US 5821333 A
- US 7642228 B
- US 7695936 B
- US 8216805 B
- US 20130089553 A
- WO 9404690 A
- WO 9627011 A
- US 4676980 A
- WO 9100360 A
- WO 92200373 A
- EP 03089 A
- WO 2004106381 A
- WO 2005061547 A
- WO 2007042261 A
- WO 2008119567 A
- EP 1691833 A
- US 6248516 B1
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1
- WO 2004056312 A1
- WO 2003085107 A
- WO 2003011878 A
- US 6602684 B
- US 20050123546 A
- WO 199730087 A
- WO 199858964 A
- WO 199922764 A
- US 5500362 A
- WO 5821337 A
- WO 2006029879 A
- WO 2005100402 A
- US 6737056 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- WO 9013646 A
- US 4965199 A
- EP 73657 A
- US 4419446 A
- US 4601978 A
- WO 9411026 A
- DD 266710
- US 5648237 A, Carter
- US 5789199 A, Joly
- US 5840523 A, Simmons
- EP 402226 A
- EP 183070 A
- EP 244234 A
- US 5959177 A
- US 6040498 A
- US 6420548 B
- US 7125978 B
- US 6417429 B
- US 4767704 A
- US 4657866 A
- US 4927762 A
- US 4560655 A
- US 5122469 A
- WO 9003430 A
- WO 8700195 A
- US RE30985 E
- US 20050260186 A
- US 20060104968 A
- US 6267958 B
- US 6171586 B
- WO 2006044908 A
- WO 2016183326 A
- WO 2016196298 A

**Non-patent literature cited in the description**

- **MULLIS et al.** Cold Spring Harbor Symp. Quant. Biol., 1987, vol. 51, 263
- PCR Technology. Stockton Press, 1989
- **CRONIN et al.** Am. J. Pathol., 2004, vol. 164 (1), 35-42
- **MA et al.** Cancer Cell, 2004, vol. 5, 607-616

- WHO Drug Information (International Nonproprietary Names for Pharmaceutical Substances). *List 74,* 2015, vol. 29 (3), 387
- *CHEMICAL ABSTRACTS,* 33069-62-4
- *Angew Chem. Inti. Ed. Engl.,* 1994, vol. 33, 183-186
- **STRAGLIOTTO et al.** *Eur. J. Cancer,* 1996, vol. 32A, 636-640
- **JOHNS et al.** *J. Biol. Chem.,* 2004, vol. 279 (29), 30375-30384
- Cell cycle regulation, oncogenes, and antineoplastic drugs. **MURAKAMI et al.** The Molecular Basis of Cancer. W.B. Saunders, 1995, 13
- **WILMAN.** Prodrugs in Cancer Chemotherapy. *Biochemical Society Transactions,* 1986, vol. 14, 375-382
- Prodrugs: A Chemical Approach to Targeted Drug Delivery,. **STELLA et al.** Directed Drug Delivery. Humana Press, 1985, 247-267
- **KLAGSBRUN ; D'AMORE.** *Annu. Rev. Physiol.,* 1991, vol. 53, 217-39
- **STREIT ; DETMAR.** *Oncogene,* 2003, vol. 22, 3172-3179
- **FERRARA ; ALITALO.** *Nature Medicine,* 1999, vol. 5 (12), 1359-1364
- **TONINI et al.** *Oncogene,* 2003, vol. 22, 6549-6556
- **SATO.** *Int. J. Clin. Oncol.,* 2003, vol. 8, 200-206
- **KABAT et al.** Sequences of Proteins of Immunological Interest. National Institute of Health, 1991
- **ABBAS et al.** Cellular and Mol. Immunology. W.B. Saunders, Co, 2000
- **PLUCKTHÜN.** The Pharmacology of Monoclonal Antibodies. Springer-Verlag, 1994, vol. 113, 269-315
- **HUDSON et al.** *Nat. Med.,* 2003, vol. 9, 129-134
- **HOLLINGER et al.** *Proc. Natl. Acad. Sci. USA,* 1993, vol. 90, 6444-6448
- **KOHLER ; MILSTEIN.** *Nature,* 1975, vol. 256, 495-97
- **HONGO et al.** *Hybridoma,* 1995, vol. 14 (3), 253-260
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol.,* 1992, vol. 222, 581-597
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnol.,* 1996, vol. 14, 845-851
- **NEUBERGER.** *Nature Biotechnol.,* 1996, vol. 14, 826
- **LONBERG et al.** *Intern. Rev. Immunol.,* 1995, vol. 13, 65-93
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. Transactions,* 1995, vol. 23, 1035-1038
- **HURLE ; GROSS.** *Curr. Op. Biotech.,* 1994, vol. 5, 428-433
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **VAN DIJK ; VAN DE WINKEL.** *Curr. Opin. Pharmacol.,* 2001, vol. 5, 368-74
- **LI et al.** *Proc. Natl. Acad. Sci. USA,* 2006, vol. 103, 3557-3562
- **XU et al.** *Immunity,* 2000, vol. 13, 37-45
- **JOHNSON ; WU.** Methods in Molecular Biology. Human Press, 2003, vol. 248, 1-25
- **HAMERS-CASTERMAN et al.** *Nature,* 1993, vol. 363, 446-448
- **SHERIFF et al.** *Nature Struct. Biol.,* 1996, vol. 3, 733-736
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917

- **KABAT et al.** Sequences of Immunological Interest. Public Health Service, National Institutes of Health, 1991
- **ZAPATA et al.** *Protein Eng,* 1995, vol. 8 (10), 1057-1062
- **SALGADO et al.** *Annals of Oncology,* 2015, vol. 26, 259-271
- Current Protocols In Molecular Biology. 1995
- **YAMADA et al.** *Cancer Sci,* 2013, vol. 104, 14-21
- *CHEMICAL ABSTRACTS,* 946414-94-4
- **CHEN et al.** *J. Mol. Biol.,* 1999, vol. 293, 865-881
- **KOHLER et al.** *Nature,* 1975, vol. 256, 495
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **EVEN et al.** *Trends in Biotechnology,* 2006, vol. 24 (3), 105-108
- **FRANEK.** *Trends in Monoclonal Antibody Research,* 2005, 111-122
- **MUNSON et al.** *Anal. Biochem.,* 1980, vol. 107, 220
- **HOOGENBOOM et al.** Methods in Molecular Biology. Human Press, 2001, vol. 178, 1-37
- **MCCAFFERTY et al.** *Nature,* vol. 348, 552-554
- **MARKS ; BRADBURY.** Methods in Molecular Biology. Human Press, 2003, vol. 248, 161-175
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **ALMAGRO ; FRANSSON.** *Front. Biosci.,* 2008, vol. 13, 1619-1633
- **QUEEN et al.** *Proc. Nat'1 Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **LONBERG.** *Curr. Opin. Immunol.,* 2008, vol. 20, 450-459
- **LONBERG.** *Nat. Biotech.,* 2005, vol. 23, 1117-1125
- **BOERNER et al.** *J. Immunol.,* 1991, vol. 147, 86
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- Antibody Engineering
- **MILLSTEIN et al.** *Nature,* 1983, vol. 305, 537-539
- **TRAUNECKER et al.** *EMBO J.,* 1991, vol. 10, 3655-3659
- Molecular weight of amino acid minus that of water. Handbook of Chemistry and Physics. Chemical Rubber Publishing Co, 1961
- **ZAMYATNIN.** *Prog. Biophys. Mol. Biol.,* 1972, vol. 24, 107-123
- **C. CHOTHIA.** *J. Mol. Biol.,* 1975, vol. 105, 1-14
- **SPIESS et al.** *Nature Biotechnology,* 2013, vol. 31, 753-758
- **SPEISS et al.** *Nat. Biotechnol.,* 2013, vol. 31, 753-8
- **SURESH et al.** *Methods in Enzymology,* 1986, vol. 121, 210
- **SHALABY et al.** *J. Exp. Med.,* 1992, vol. 175, 217-225
- **KOSTELNY et al.** *J. Immunol.,* 1992, vol. 148 (5), 1547-1553
- **GRUBER et al.** *J. Immunol,* 1994, vol. 152, 5368
- **TUFT et al.** *J. Immunol.,* 1991, vol. 147, 60
- **CHOWDHURY.** *Methods Mol. Biol.,* 2008, vol. 207, 179-196
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614

- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **FERRARA et al.** *Biotechnology and Bioengineering,* 2006, vol. 93 (5), 851-861
- **RAVETCH ; KINET.** *Annu. Rev. Immunol.,* 1991, vol. 9, 457-492
- **HELLSTROM et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG et al.** *Blood,* 2003, vol. 101, 1045-1052
- **CRAGG et al.** *Blood,* 2004, vol. 103, 2738-2743
- **PETKOVA et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **STINCHCOMB et al.** *Nature,* 1979, vol. 282, 39
- **JONES.** *Genetics,* 1977, vol. 85, 12
- **VAN DEN BERG.** *Bio/Technology,* 1990, vol. 8, 135
- **FLEER et al.** *Bio/Technology,* 1991, vol. 9, 968-975
- **REYES et al.** *Nature,* 1982, vol. 297, 598-601
- **YANIV.** *Nature,* 1982, vol. 297, 17-18
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- **HAM et al.** *Meth. Enz.,* 1979, vol. 58, 44
- **BARNES et al.** *Anal. Biochem.,* 1980, vol. 102, 255
- **LINDMARK et al.** *J. Immunol. Meth.,* 1983, vol. 62, 1-13
- **GUSS et al.** *EMBO J.,* 1986, vol. 5, 15671575
- Antibodies, A Laboratory Manual. Cold Spring Harbor Laboratory, 1988
- **CHAMPE et al.** *J. Biol. Chem.,* 1995, vol. 270, 1388-1394
- Remington's Pharmaceutical Sciences. 1980
- **EMENS et al.** *JAMA Oncology,* 2018
- **SALGADO et al.** *Annals of Oncology,* 2015, vol. 26 (2), 259-271